# EUROPEAN PATENT SPECIFICATION

(11) **EP 3 494 997 B1**
(45) Date of publication and mention of the grant of the patent: **18.09.2019**
(21) Application number: 18200462.2
(22) Date of filing: 21.07.2013
(51) Int. Cl.: A61K 48/00, C12N 15/62, C12N 15/63, C12N 15/90

(54) **INDUCIBLE DNA BINDING PROTEINS AND GENOME PERTURBATION TOOLS AND APPLICATIONS THEREOF**
INDUZIERBARE DNS-BINDENDE PROTEINE UND GENOMPERTURBATIONSTOOLS SOWIE ANWENDUNGEN DAVON
PROTÉINES DE LIAISON À L'ADN INDUCTIBLES, OUTILS DE PERTURBATION DU GÉNOME ET LEURS APPLICATIONS

(30) Priority: 25.07.2012 US 201261675778 P; 01.11.2012 US 201261721283 P; 12.12.2012 US 201261736465 P; 15.03.2013 US 201361794458 P; 17.06.2013 US 201361835973 P
(43) Date of publication of application: 12.06.2019
(62) Divisional of application: 13744916.1
(73) Proprietor: The Broad Institute, Inc., Cambridge, MA 02142 (US); Massachusetts Institute of Technology, Cambridge, MA 02139 (US); President and Fellows of Harvard College, Cambridge, MA 02138 (US)
(72) Inventor: ZHANG, Feng, Cambridge, MA 02139 (US); BRIGHAM, Mark, Somerville, MA 02145 (US); CONG, Le, Cambridge, MA 02142 (US); KONERMANN, Silvana, 8005 Zürich (CH); SANJANA, Neville Espi, Cambridge, MA 02142 (US)
(74) Representative: Cabinet Plasseraud

(56) References cited:
- WO-A1-2013/142578
- WO-A1-2013/176772
- WO-A1-2014/065596
- WO-A1-2014/089290
- WO-A1-2014/093712
- US-A1- 2010 076 057
- M. JINEK ET AL: "A Programmable Dual-RNA-Guided DNA Endonuclease in Adaptive Bacterial Immunity", SCIENCE, vol. 337, no. 6096, 17 August 2012 (2012-08-17), pages 816-821, XP055549487, US ISSN: 0036-8075, DOI: 10.1126/science.1225829
- R. SAPRANAUSKAS ET AL: "The Streptococcus thermophilus CRISPR/Cas system provides immunity in Escherichia coli", NUCLEIC ACIDS RESEARCH, vol. 39, no. 21, 3 August 2011 (2011-08-03), pages 9275-9282, XP055265024, ISSN: 0305-1048, DOI: 10.1093/nar/gkr606
- DANA CARROLL: "A CRISPR Approach to Gene Targeting", MOLECULAR THERAPY, vol. 20, no. 9, 1 September 2012 (2012-09-01), pages 1658-1660, XP055106489, ISSN: 1525-0016, DOI: 10.1038/mt.2012.171

## Description

### RELATED APPLICATIONS

This application claims priority to and claims benefit of US provisional patent application Serial Nos. 61/675,778 filed July 25, 2012, 61/721,283 filed November 1, 2012, 61/736,465 filed December 12, 2012, 61/794,458 filed March 15, 2013 and 61/835,973 filed June 17, 2013 titled INDUCIBLE DNA BINDING PROTEINS AND GENOME PERTURBATION TOOLS AND APPLICATIONS THEREOF.

Reference is also made to US Provisional Application No. 61/565,171 filed November 30, 2011 and US Application Nos. 13/554,922 filed July 30, 2012 and 13/604,945 filed September 6, 2012, titled NUCLEOTIDE-SPECIFIC RECOGNITION SEQUENCES FOR DESIGNER TAL EFFECTORS.

Reference is also made to US Provisional Application Nos. 61/736,527 filed December 12, 2012; 61/748,427 filed January 2, 2013; 61/757,972 filed January 29, 2013, 61/768,959, filed February 25, 2013 and 61/791,409 filed March 15, 2013, titled SYSTEMS METHODS AND COMPOSITIONS FOR SEQUENCE MANIPULATION.

Reference is also made to US Provisional Application Nos. 61/758,468 filed January 30, 2013 and 61/769,046 filed March 15, 2013, titled ENGINEERING AND OPTIMIZATION OF SYSTEMS, METHODS AND COMPOSITIONS FOR SEQUENCE MANIPULATION.

Reference is also made to US Provisional Application Nos. 61/835,931; 61/835,936; 61/836,080; 61/836,101; 61/836,123 and 61/836,127 filed June 17, 2013.

Reference is also made to US Provisional Application No. 61/842,322, filed July 2, 2013, titled CRISPR-CAS SYSTEMS AND METHODS FOR ALTERING EXPRESSION OF GENE PRODUCTS and US Provisional Application No. 61/847,537, filed July 17, 2013, titled DELIVERY, ENGINEERING AND OPTIMIZATION OF SYSTEMS, METHODS AND COMPOSITIONS FOR SEQUENCE MANIPULATION AND APPLICATIONS.

The foregoing applications, and all documents cited therein or during their prosecution ("appln cited documents") and all documents cited or referenced in the appln cited documents, and all documents cited or referenced herein ("herein cited documents"), and all documents cited or referenced in herein cited documents, together with any manufacturer's instructions, descriptions, product specifications, and product sheets for any products mentioned hereinmay be employed in the practice of the invention.

### FIELD OF THE INVENTION

The present invention generally relates to CRISPR-Cas complexes, systems and related uses.

### FEDERAL FUNDING LEGEND

This invention was made with government support under R01NS073124 and Pioneer Award 1DP1MH100706 awarded by the National Institutes of Health. The Government has certain rights in the invention.

### BACKGROUND OF THE INVENTION

Precise and efficient genome targeting technologies are needed to enable systematic reverse engineering of causal genetic variations by allowing selective perturbation of individual genetic elements. Although genome-editing technologies such as designer zinc fingers (ZFs) (M. H. Porteus, D. Baltimore, Chimeric nucleases stimulate gene targeting in human cells. Science 300, 763 (May 2, 2003); J. C. Miller et al., An improved zinc-finger nuclease architecture for highly specific genome editing. Nat Biotechnol 25, 778 (Jul, 2007); J. D. Sander et al., Selection-free zinc-finger-nuclease engineering by context-dependent assembly (CoDA). Nat Methods 8, 67 (Jan, 2011) and A. J. Wood et al., Targeted genome editing across species using ZFNs and TALENs. Science 333, 307 (Jul 15, 2011)), transcription activator-like effectors (TALEs) >A. J. Wood et al., Targeted genome editing across species using ZFNs and TALENs. Science 333, 307 (Jul 15, 2011); M. Christian et al., Targeting DNA double-strand breaks with TAL effector nucleases. Genetics 186, 757 (Oct, 2010); F. Zhang et al., Efficient construction of sequence-specific TAL effectors for modulating mammalian transcription. Nat Biotechnol 29, 149 (Feb, 2011); J. C. Miller et al., A TALE nuclease architecture for efficient genome editing. Nat Biotechnol 29, 143 (Feb, 2011); D. Reyon et al., FLASH assembly of TALENs for high-throughput genome editing. Nat Biotechnol 30, 460 (May, 2012); J. Boch et al., Breaking the code of DNA binding specificity of TAL-type III effectors. Science 326, 1509 (Dec 11, 2009) and M. J. Moscou, A. J. Bogdanove, A simple cipher governs DNA recognition by TAL effectors. Science 326, 1501 (Dec 11, 2009)), and homing meganucleases (B. L. Stoddard, Homing endonuclease structure and function. Quarterly reviews of biophysics 38, 49 (Feb, 2005)) have begun to enable targeted genome modifications, there remains a need for new technologies that are scalable, affordable, and easy to engineer. Here, Applicants report the development of a new class of precision genome engineering tools based on the RNA-guided Cas9 nuclease (M. Jinek et al., A programmable dual-RNA-guided DNA endonuclease in adaptive bacterial immunity. Science 337, 816 (Aug 17, 2012); G. Gasiunas, R. Barrangou, P. Horvath, V. Siksnys, Cas9-crRNA ribonucleoprotein complex mediates specific DNA cleavage for adaptive immunity in bacteria. Proc Natl Acad Sci USA 109, E2579 (Sep 25, 2012) and J. E. Garneau et al., The CRISPR/Cas bacterial immune system cleaves bacteriophage and plasmid DNA. Nature 468, 67 (Nov 4, 2010)) from the type II prokaryotic CRISPR adaptive immune system (H. Deveau, J. E. Garneau, S. Moineau, CRISPR/Cas system and its role in phage-bacteria interactions. Annual review of microbiology 64, 475 (2010); P. Horvath, R. Barrangou, CRISPR/Cas, the immune system of bacteria and archaea. Science 327, 167 (Jan 8, 2010); K. S. Makarova et al., Evolution and classification of the CRISPR-Cas systems. Nat Rev Microbiol 9, 467 (Jun, 2011) and D. Bhaya, M. Davison, R. Barrangou, CRISPR-Cas systems in bacteria and archaea: versatile small RNAs for adaptive defense and regulation. Annu Rev Genet 45, 273 (2011)).

Citation or identification of any document in this application is not an admission that such document is available as prior art to the present invention.

### SUMMARY OF THE INVENTION AND OF THE PRESENT DISCLOSURE

The present invention is defined by the appended claims. Aspects of the present invention and/or present disclosure are described below. Aspects of the present disclosure may also pertain to the invention as defined the appended claims.

In particular, the present invention provides a Clustered Regularly Interspersed Short Palindromic Repeats (CRISPR)-Cas complex, comprising:
- a Cas9, wherein the Cas9 has two or more nuclear localization signals;
- a guide sequence linked to a tracr mate sequence, and
- a tracr sequence hybridizable with all or a portion of the tracr mate sequence;
   wherein the guide sequence is designed to have complementarity with a target sequence in a eukaryotic cell and is capable of directing sequence-specific binding of the CRISPR complex to the target sequence in the eukaryotic cell.

The invention also provides a Clustered Regularly Interspersed Short Palindromic Repeats (CRISPR)-Cas vector system, comprising:
a first regulatory element operably linked to one or more sequences encoding (1) a guide sequence linked to a tracr mate sequence, and (2) a tracr sequence hybridizable with all or a portion of the tracr mate sequence;
a second regulatory element operably linked to a sequence encoding a Cas9, wherein the Cas9 has two or more nuclear localization signals;
wherein components (a) and (b) are located on same or different vectors of the system and
wherein the guide sequence is designed to have complementarity with a target sequence in a eukaryotic cell.

The invention also provides the CRISPR-Cas complex according to the invention or the CRISPR-Cas vector system of the invention for use in therapy.

Further disclosed is use of the CRISPR complex according to the invention or the CRISPR-Cas vector system according to the invention for genome engineering, wherein said use is not a method of treatment of the animal or human body by therapy, and wherein said use is not a process for modifying the germ-line genetic identity of human beings.

Further aspects of disclosure provide for systems or methods as described herein wherein the CRISPR system may comprise a vector system comprising: a) a first regulatory element operably linked to a CRISPR-Cas system guide RNA that targets a locus of interest, b) a second regulatory inducible element operably linked to a Cas protein, wherein components (a) and (b) may be located on same or different vectors of the system, wherein the guide RNA targets DNA of the locus of interest, wherein the Cas protein and the guide RNA do not naturally occur together. The Cas protein is a Cas9 enzyme.
The invention also provides for the vector being a AAV or a lentivirus.

A further aspect of disclosure is an inducible multiplex genome engineering using CRISPR (clustered regularly interspaced short palindromic repeats)/Cas systems.

The present invention also relates to nucleic acid encoding the polypeptides of the present invention. The nucleic acid may comprise a promoter, advantageously human Synapsin I promoter (hSyn). In a particularly advantageous embodiment, the nucleic acid may be packaged into an adeno associated viral vector (AAV).

The invention further also relates to methods of treatment or therapy that encompass the methods and compositions described herein.

These and other embodiments are disclosed and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings.

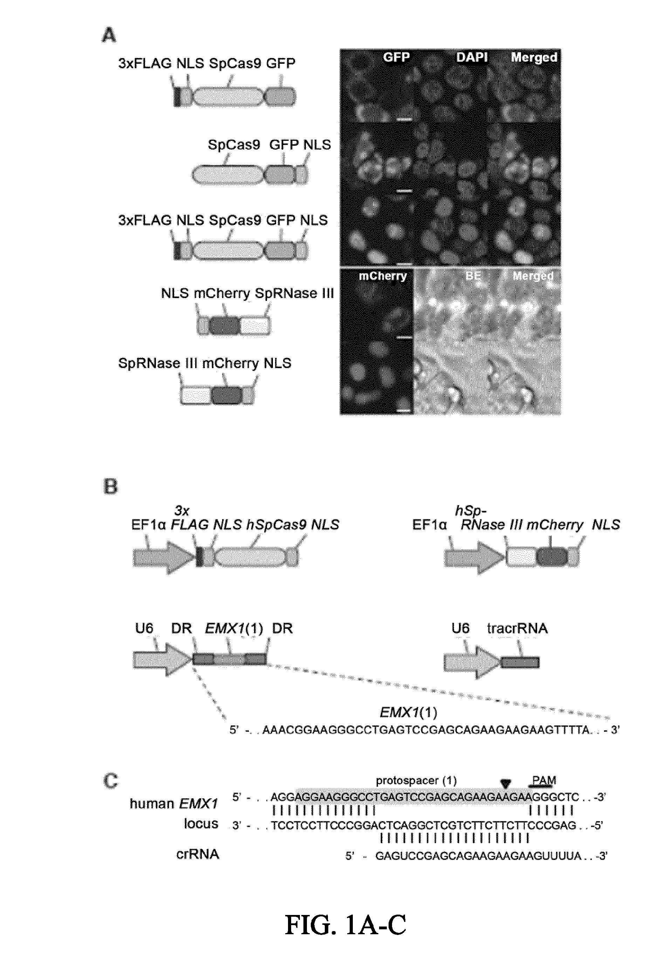

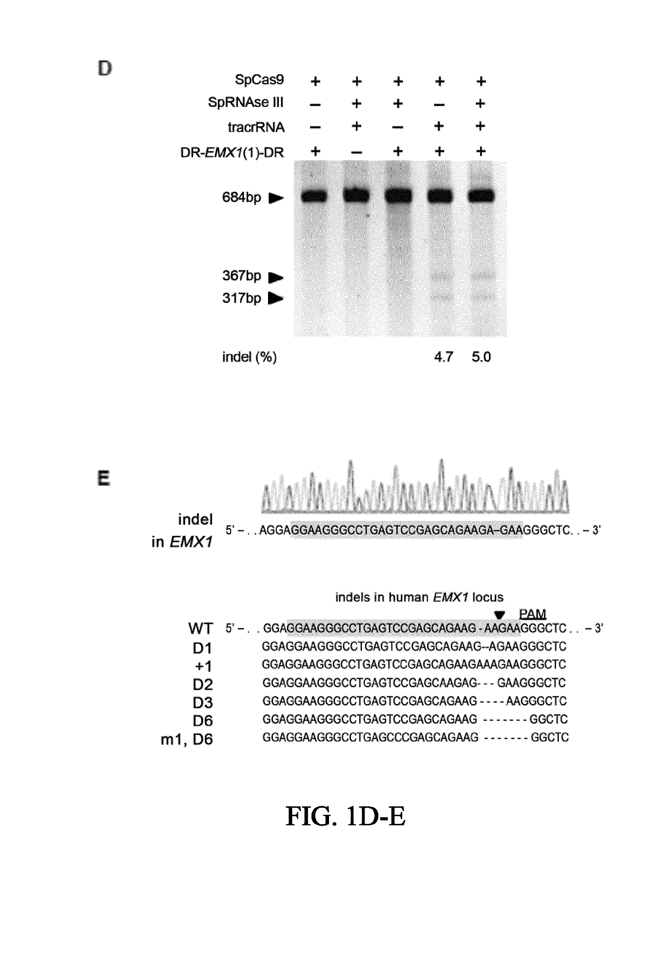

**FIGS. 1A-E** depict a Type II CRISPR locus from *Streptococcus pyogenes* SF370 can be reconstituted in mammalian cells to facilitate targeted DSBs of DNA. **(A)** Engineering of SpCas9 and SpRNase III with NLSs enables import into the mammalian nucleus. **(B)** Mammalian expression of SpCas9 and SpRNase III are driven by the EFla promoter, whereas tracrRNA and pre-crRNA array (DR-Spacer-DR) are driven by the U6 promoter. A protospacer (blue highlight) from the human *EMX1* locus with PAM is used as template for the spacer in the pre-crRNA array. **(C)** Schematic representation of base pairing between target locus and *EMX1-*targeting crRNA. Red arrow indicates putative cleavage site. **(D)** SURVEYOR assay for SpCas9-mediated indels. **(E)** An example chromatogram showing a micro-deletion, as well as representative sequences of mutated alleles identified from 187 clonal amplicons. Red dashes, deleted bases; red bases, insertions or mutations. Scale bar = 10µm.

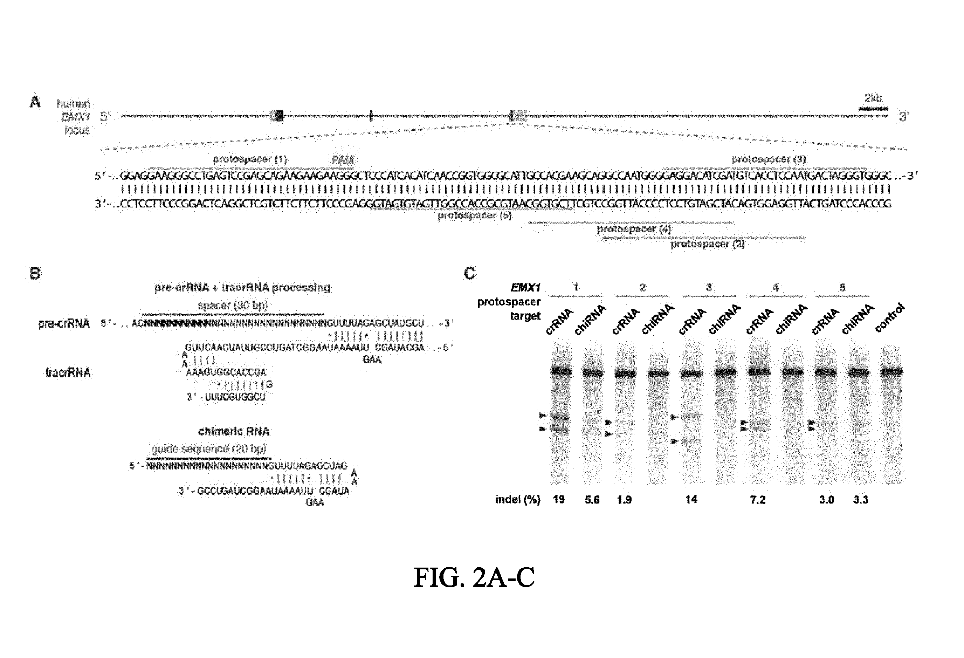

**FIGS. 2A-C** depict a SpCas9 can be reprogrammed to target multiple genomic loci in mammalian cells. **(A)** Schematic of the human *EMX1* locus showing the location of five protospacers, indicated by blue lines with corresponding PAM in magenta. **(B)** Schematic of the pre-crRNA:tracrRNA complex (top) showing hybridization between the direct repeat (gray) region of the pre-crRNA and tracrRNA. Schematic of a chimeric RNA design (M. Jinek et al., A programmable dual-RNA-guided DNA endonuclease in adaptive bacterial immunity. Science 337, 816 (Aug 17, 2012)) (bottom). tracrRNA sequence is shown in red and the 20bp spacer sequence in blue. **(C)** SURVEYOR assay comparing the efficacy of Cas9-mediated cleavage at five protospacers in the human *EMX1* locus. Each protospacer is targeted using either processed pre-crRNA:tracrRNA complex (crRNA) or chimeric RNA (chiRNA).

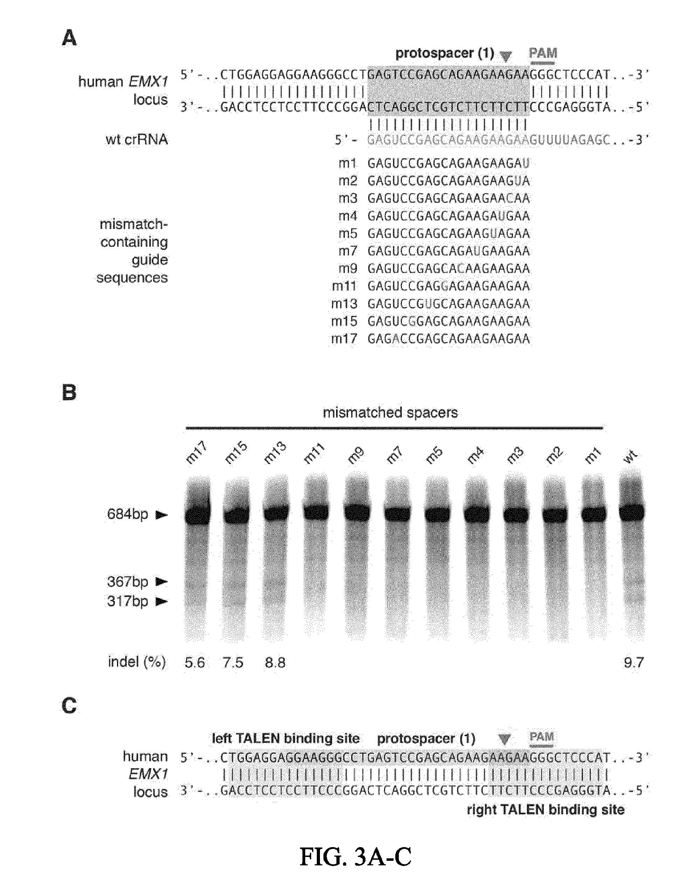

**FIGS. 3A-D** depict an evaluation of the SpCas9 specificity and comparison of efficiency with TALENs. **(A)** *EMX1-*targeting chimeric crRNAs with single point mutations were generated to evaluate the effects of spacer-protospacer mismatches. **(B)** SURVEYOR assay comparing the cleavage efficiency of different mutant chimeric RNAs. **(C)** Schematic showing the design of TALENs targeting *EMX1.* **(D)** SURVEYOR gel comparing the efficiency of TALEN and SpCas9 (*N* = 3).

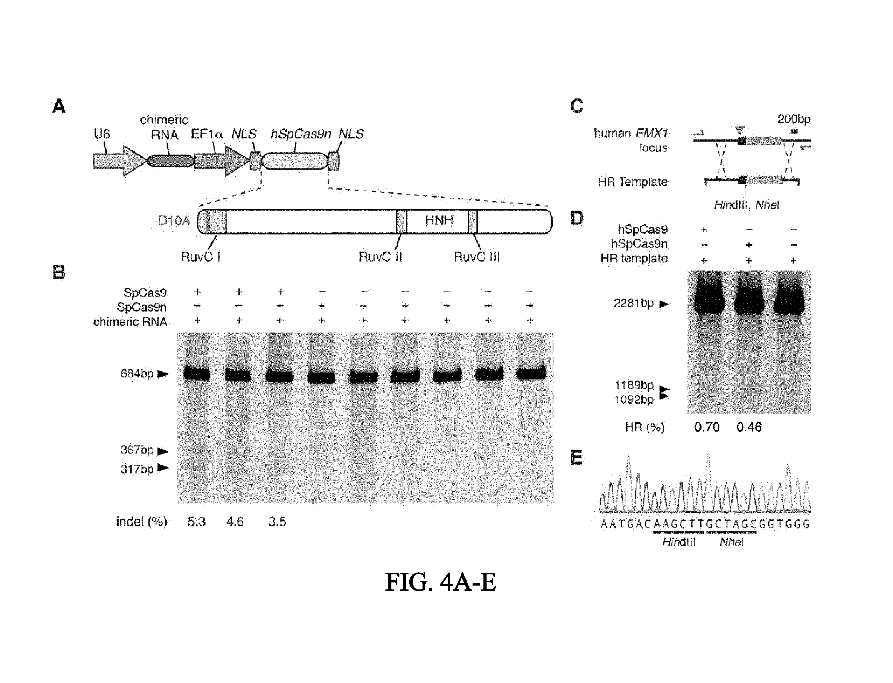

**FIGS. 4A-G** depict applications of Cas9 for homologous recombination and multiplex genome engineering. **(A)** Mutation of the RuvC I domain converts Cas9 into a nicking enzyme (SpCas9n) **(B)** Co-expression of *EMX1-*targeting chimeric RNA with SpCas9 leads to indels, whereas SpCas9n does not (*N* = 3). **(C)** Schematic representation of the recombination strategy. A repair template is designed to insert restriction sites into *EMX1* locus. Primers used to amplify the modified region are shown as red arrows. **(D)** Restriction fragments length polymorphism gel analysis. Arrows indicate fragments generated by *Hin*dIII digestion. **(E)** Example chromatogram showing successful recombination. **(F)** SpCas9 can facilitate multiplex genome modification using a crRNA array containing two spacers targeting *EMX1* and *PVALB.* Schematic showing the design of the crRNA array (top). Both spacers mediate efficient protospacer cleavage (bottom). **(G)** SpCas9 can be used to achieve precise genomic deletion. Two spacers targeting *EMX1* (top) mediated a 118bp genomic deletion (bottom).

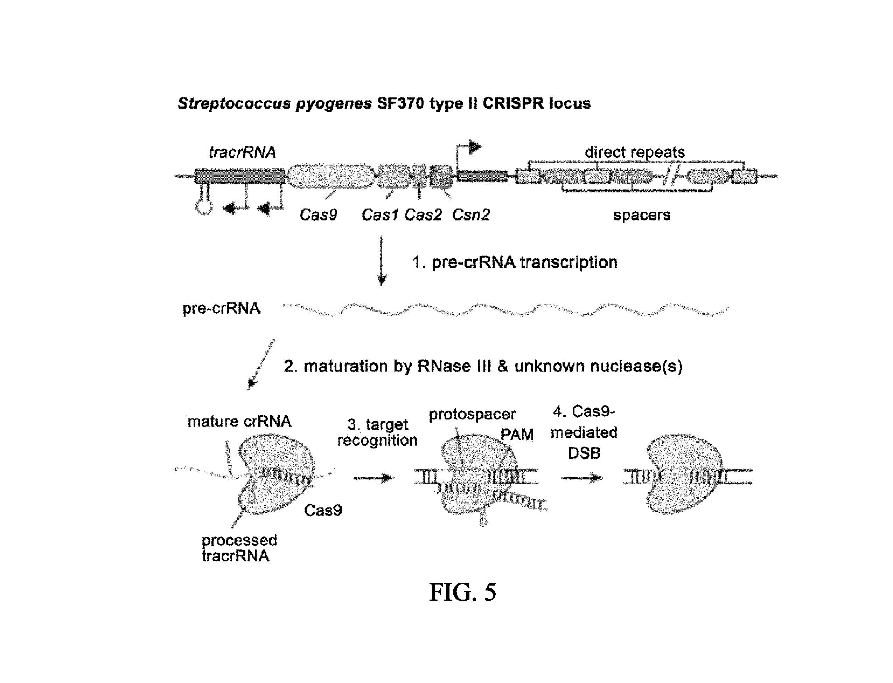

**FIG. 5** depicts a schematic of the type II CRISPR-mediated DNA double-strand break. The type II CRISPR locus from *Streptococcus pyogenes* SF370 contains a cluster of four genes, *Cas9, Cas1, Cas2,* and *Csn1,* as well as two non-coding RNA elements, tracrRNA and a characteristic array of repetitive sequences (direct repeats) interspaced by short stretches of non-repetitive sequences (spacers, 30bp each) (*15-18, 30, 31*). Each spacer is typically derived from foreign genetic material (protospacer), and directs the specificity of CRISPR-mediated nucleic acid cleavage. In the target nucleic acid, each protospacer is associated with a protospacer adjacent motif (PAM) whose recognition is specific to individual CRISPR systems (22, 23). The Type II CRISPR system carries out targeted DNA double-strand break (DSB) in sequential steps (M. Jinek et al., Science 337, 816 (Aug 17, 2012); Gasiunas, R. et al. Proc Natl Acad Sci USA 109, E2579 (Sep 25, 2012); J. E. Garneau et al., Nature 468, 67 (Nov 4, 2010); R. Sapranauskas et al., Nucleic Acids Res 39, 9275 (Nov, 2011); A. H. Magadan et al. PLoS One 7, e40913 (2012)). First, the pre-crRNA array and tracrRNA are transcribed from the CRISPR locus. Second, tracrRNA hybridizes to the direct repeats of pre-crRNA and associates with Cas9 as a duplex, which mediates the processing of the pre-crRNA into mature crRNAs containing individual,truncated spacer sequences. Third, the mature crRNA:tracrRNA duplex directs Cas9 to the DNA target consisting of the protospacer and the requisite PAM via heteroduplex formation between the spacer region of the crRNA and the protospacer DNA. Finally, Cas9 mediates cleavage of target DNA upstream of PAM to create a DSB within the protospacer.

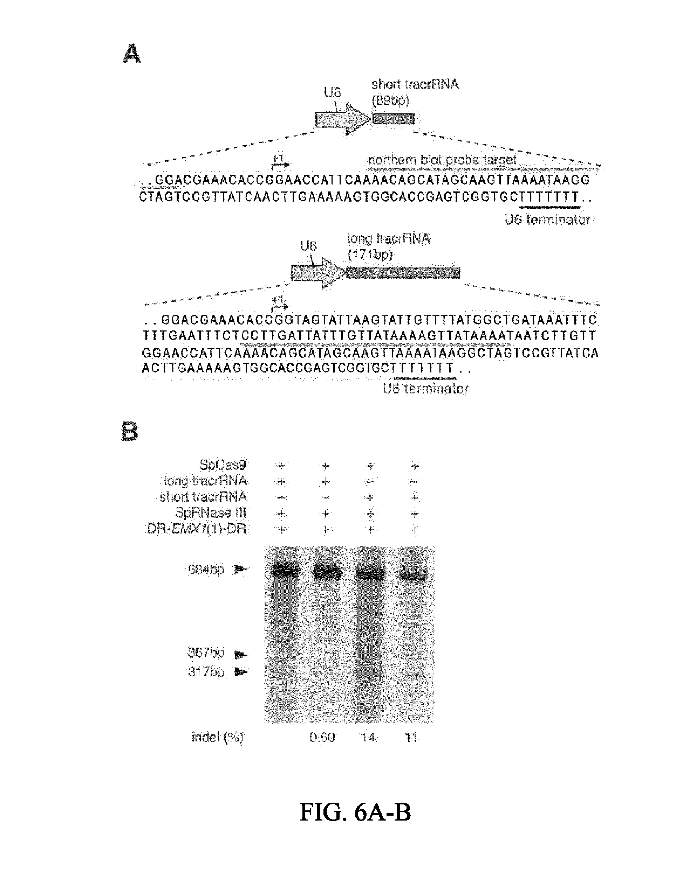

**FIGS. 6A-C** depict a comparison of different tracrRNA transcripts for Cas9-mediated gene targeting. **(A)** Schematic showing the design and sequences of two tracrRNA transcripts tested (short and long). Each transcript is driven by a U6 promoter. Transcription start site is marked as +1 and transcription terminator is as indicated. Blue line indicates the region whose reverse-complement sequence is used to generate northern blot probes for tracrRNA detection. **(B)** SURVEYOR assay comparing the efficiency of hSpCas9-mediated cleavage of the *EMX1* locus. Two biological replicas are shown for each tracrRNA transcript. **(C)** Northern blot analysis of total RNA extracted from 293FT cells transfected with U6 expression constructs carrying long or short tracrRNA, as well as SpCas9 and DR*-EMX1*(1)-DR. Left and right panels are from 293FT cells transfected without or with SpRNase III respectively. U6 indicate loading control blotted with a probe targeting human U6 snRNA. Transfection of the short tracrRNA expression construct led to abundant levels of the processed form of tracrRNA (∼75bp) (E. Deltcheva et al., Nature 471, 602 (Mar 31, 2011)). Very low amounts of long tracrRNA are detected on the Northern blot. As a result of these experiments, Applicants chose to use short tracrRNA for application in mammalian cells.

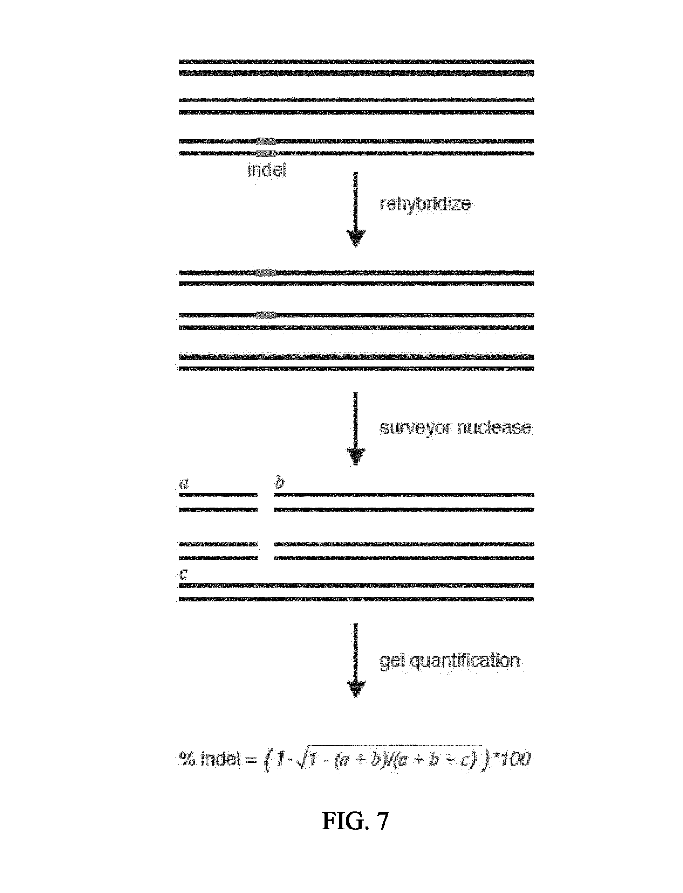

**FIG. 7** depicts a SURVEYOR assay for detection of double strand break-induced micro insertions and deletions (D. Y. Guschin et al. Methods Mol Biol 649, 247 (2010)). Schematic of the SURVEYOR assay used to determine Cas9-mediated cleavage efficiency. First, genomic PCR (gPCR) is used to amplify the Cas9 target region from a heterogeneous population of modified and unmodified cells, and the gPCR products are reannealed slowly to generate heteroduplexes. The reannealed heteroduplexes are cleaved by SURVEYOR nuclease, whereas homoduplexes are left intact. Cas9-mediated cleavage efficiency (% indel) is calculated based on the fraction of cleaved DNA.

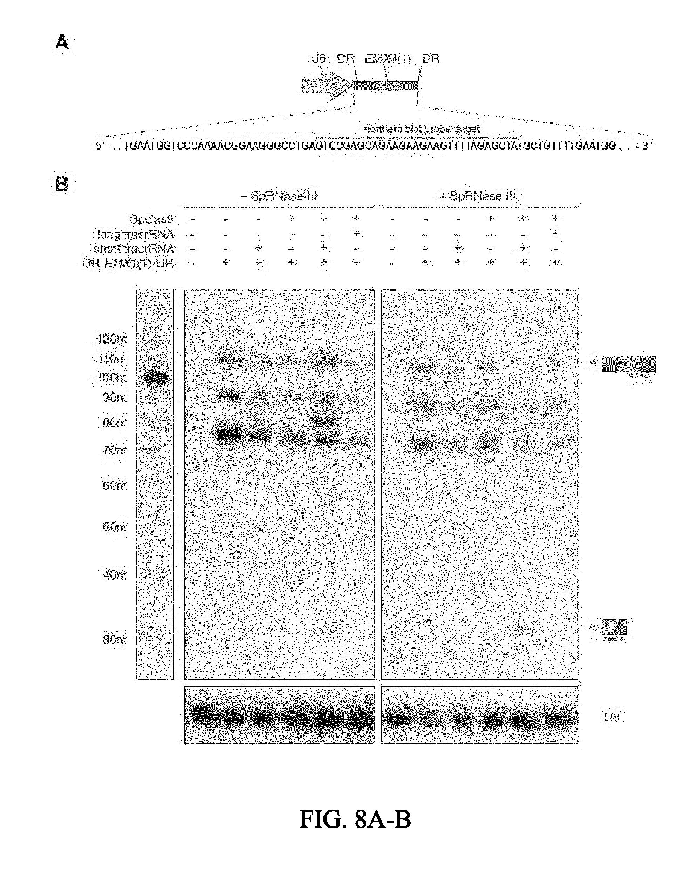

**FIG. 8A-B** depict a Northern blot analysis of crRNA processing in mammalian cells. **(A)** Schematic showing the expression vector for a single spacer flanked by two direct repeats (DR*-EMX1(1)-DR).* The 30bp spacer targeting the human *EMX1* locus protospacer 1 (Table 1) is shown in blue and direct repeats are in shown in gray. Orange line indicates the region whose reversecomplement sequence is used to generate northern blot probes for *EMX1*(1) crRNA detection. **(B)** Northern blot analysis of total RNA extracted from 293FT cells transfected with U6 expression constructs carrying DR*-EMX1*(1)*-DR.* Left and right panels are from 293FT cells transfected without or with SpRNase III respectively. DR*-EMX*1(1)*-*DR was processed into mature crRNAs only in the presence of SpCas9 and short tracrRNA, and was not dependent on the presence of SpRNase III. The mature crRNA detected from transfected 293FT total RNA is ∼33bp and is shorter than the 39-42bp mature crRNA from *S. pyogenes* (E. Deltcheva et al., Nature 471, 602 (Mar 31, 2011)), suggesting that the processed mature crRNA in human 293FT cells is likely different from the bacterial mature crRNA in *S. pyogenes.*

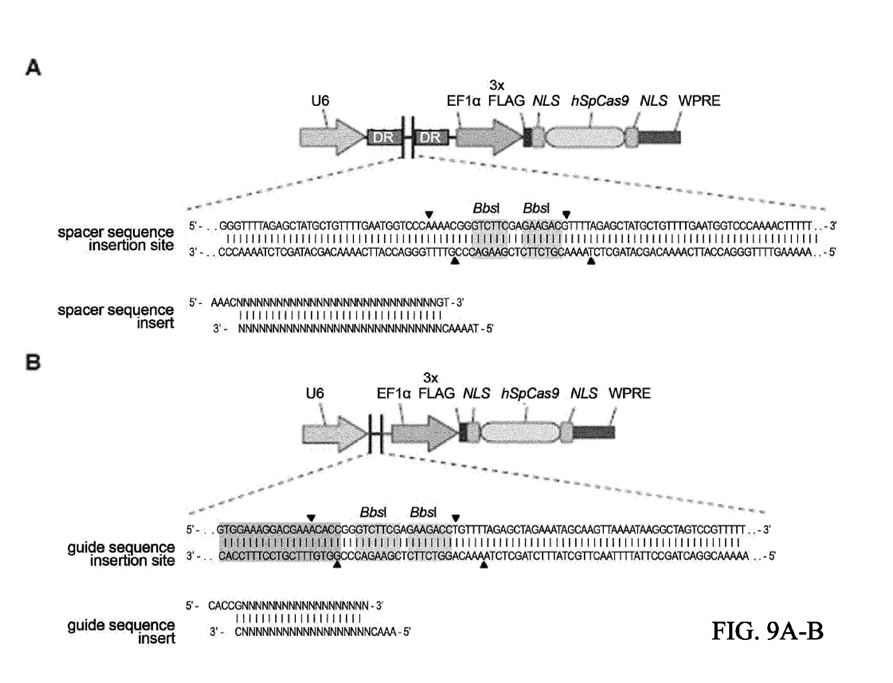

**FIG. 9A-B** depict a bicistronic expression vectors for pre-crRNA array or chimeric crRNA with Cas9. **(A)** Schematic showing the design of an expression vector for the pre-crRNA array. Spacers can be inserted between two *Bbs*I sites using annealed oligonucleotides. Sequence design for the oligonucleotides are shown below with the appropriate ligation adapters indicated. **(B)** Schematic of the expression vector for chimeric crRNA. The guide sequence can be inserted between two *Bbs*I sites using annealed oligonucleotides. The vector already contains the partial direct repeat (gray) and partial tracrRNA (red) sequences. WPRE, Woodchuck hepatitis virus posttranscriptional regulatory element.

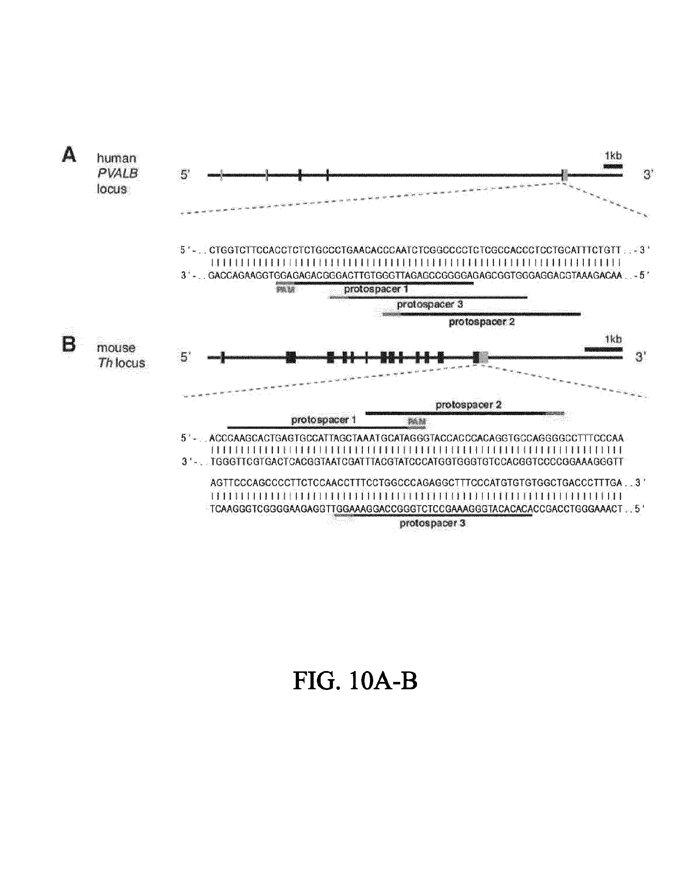

**FIGS. 10A-B** depict a selection of protospacers in the human *PVALB* and mouse *Th* loci. Schematic of the human *PVALB* **(A)** and mouse *Th* **(B)** loci and the location of the three protospacers within the last exon of the *PVALB* and *Th* genes, respectively. The 30bp protospacers are indicated by black lines and the adjacent PAM sequences are indicated by the magenta bar. Protospacers on the sense and anti-sense strands are indicated above and below the DNA sequences respectively.

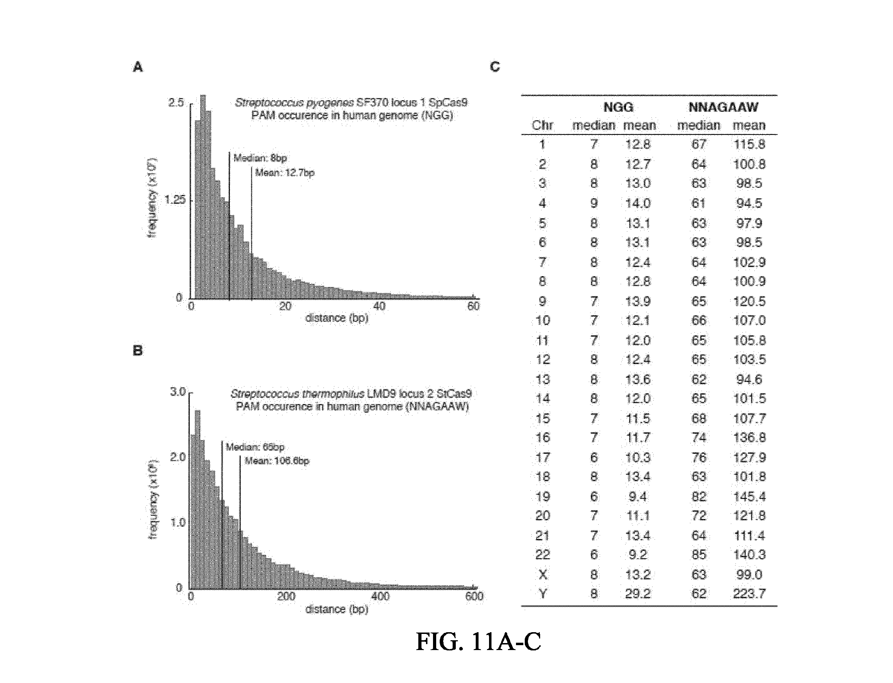

**FIGS. 11A-C** depict occurrences of PAM sequences in the human genome. Histograms of distances between adjacent *Streptococcus pyogenes* SF370 locus 1 PAM (NGG) **(A)** and *Streptococcus thermophiles* LMD9 locus 1 PAM (NNAGAAW) **(B)** in the human genome. **(C)** Distances for each PAM by chromosome. Chr, chromosome. Putative targets were identified using both the plus and minus strands of human chromosomal sequences. Given that there may be chromatin, DNA methylation-, RNA structure, and other factors that may limit the cleavage activity at some protospacer targets, it is important to note that the actual targeting ability might be less than the result of this computational analysis.

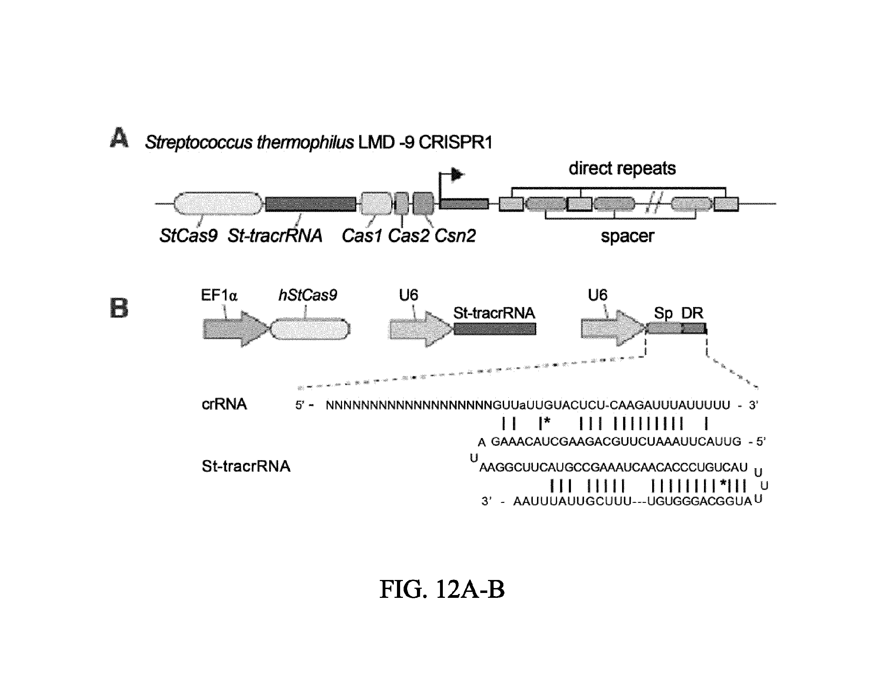

**FIGS. 12A-D** depict type II CRISPR from *Streptococcus thermophilus* LMD-9 can also function in eukaryotic cells. **(A)** Schematic of CRISPR locus 2 from *Streptococcus thermophilus* LMD-9. **(B)** Design of the expression system for the *S. thermphilus* CRISPR system. Human codon-optimized *hStCas9* is expressed using a constitutive EFla promoter. Mature versions of tracrRNA and crRNA are expressed using the U6 promoter to ensure precise transcription initiation. Sequences for the mature crRNA and tracrRNA are shown. A single based indicated by the lower case "a" in the crRNA sequence was used to remove the polyU sequence, which serves as a RNA Pol III transcriptional terminator. **(C)** Schematic showing protospacer and corresponding PAM sequences targets in the human *EMX1* locus. Two protospacer sequences are highlighted and their corresponding PAM sequences satisfying the NNAGAAW motif are indicated by magenta lines. Both protospacers are targeting the anti-sense strand. **(D)** SURVEYOR assay showing StCas9-mediated cleavage in the target locus. RNA guide spacers 1 and 2 induced 14% and 6.4% respectively. Statistical analysis of cleavage activity across biological replica at these two protospacer sites can be found in Table 1.

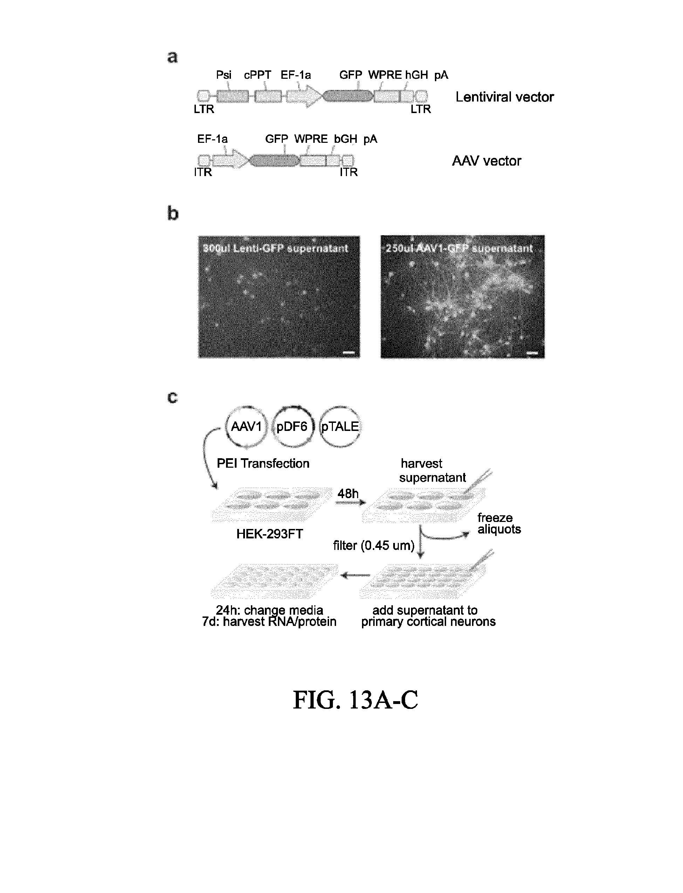

**FIGS. 13A-C** depict **AAV supernatant production. (a)** Lentiviral and AAV vectors carrying GFP were used to test transduction efficiency. **(b)** Primary embryonic cortical neurons were transduced with 300 and 250 µL supernatant derived from the same number of AAV or lentivirus-transfected 293FT cells. Representative images of GFP expression were collected at 7 d.p.i. Scale bars = 50 µm. **(c)** The depicted process was developed for the production of AAV supernatant and subsequent transduction of primary neurons. 293FT cells were transfected with an AAV vector carrying the gene of interest, the AAV1 serotype packaging vector (pAAV1), and helper plasmid (pDF6) using PEI. 48 h later, the supernatant was harvested and filtered through a 0.45 µm PVDF membrane. Primary neurons were then transduced with supernatant and remaining aliquots were stored at -80°C. Stable levels of AAV construct expression were reached after 5-6 days. AAV supernatant production following this process can be used for production of up to 96 different viral constructs in 96-well format.

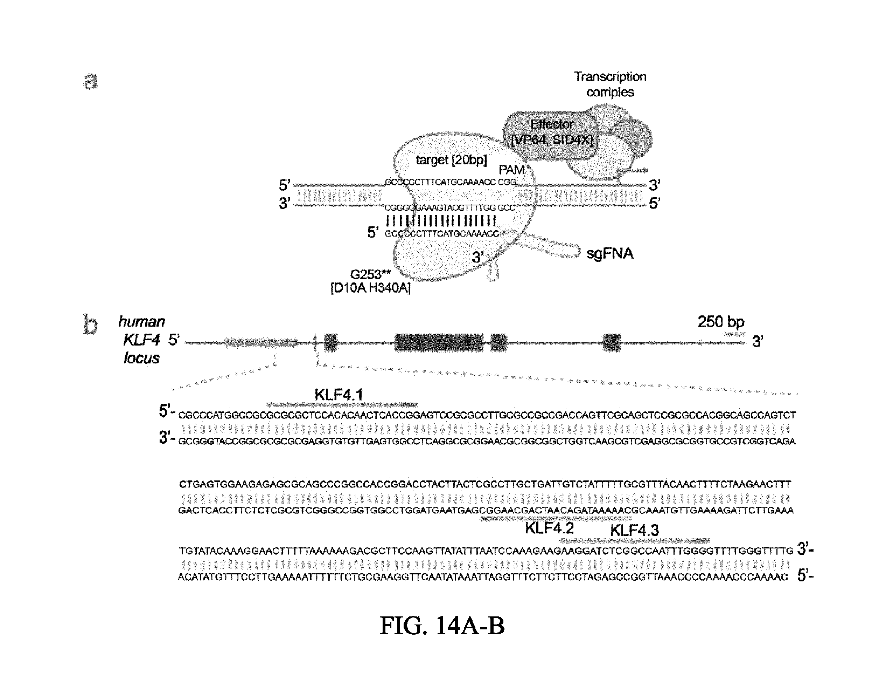

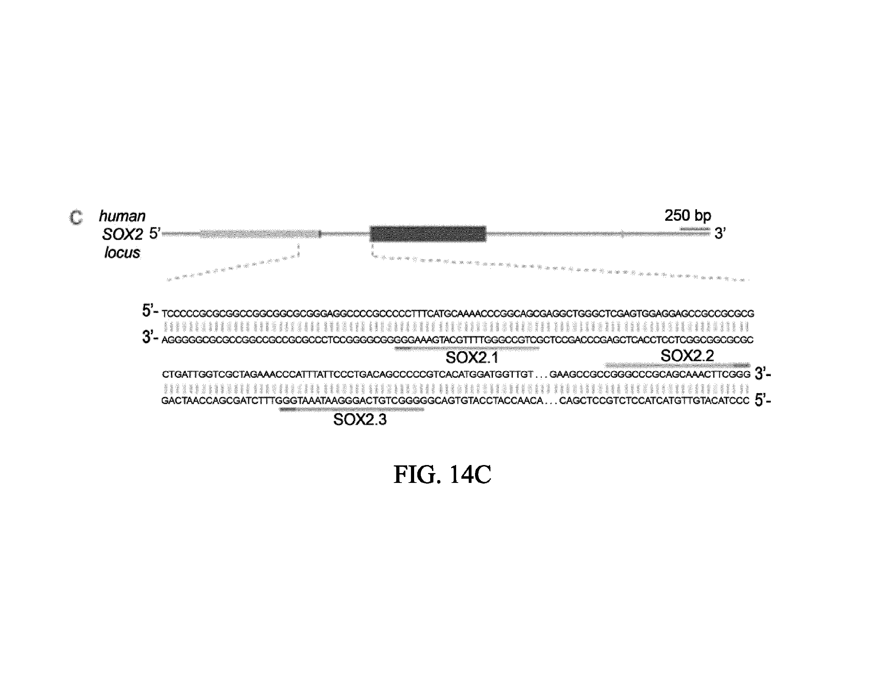

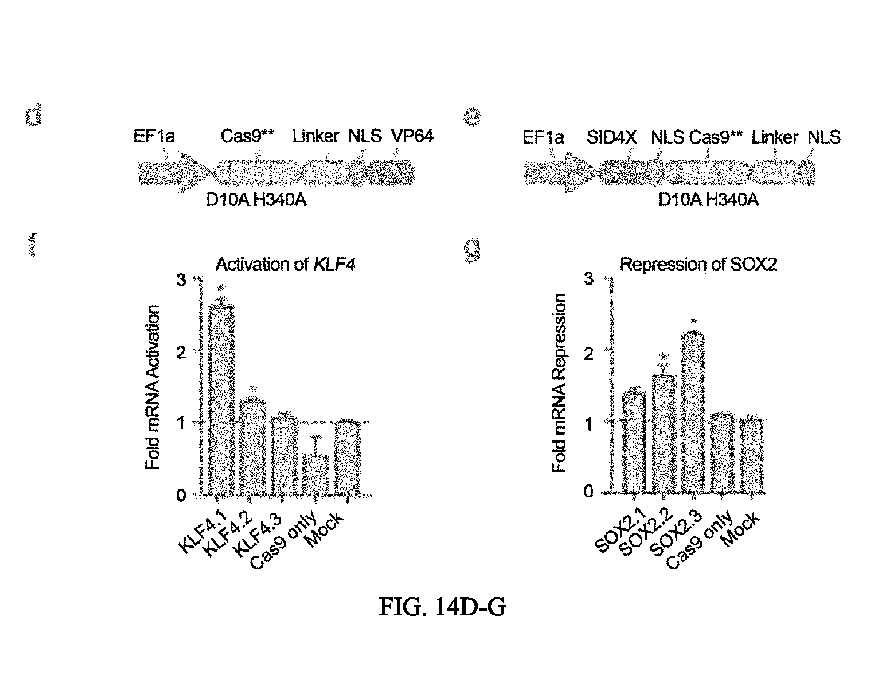

**FIGS. 14A-G** depict RNA-guided DNA binding protein Cas9 can be used to target transcription effector domains to specific genomic loci. **(a)** The RNA-guided nuclease Cas9 from the type II *Streptococcus pyogenes* CRISPR/Cas system can be converted into a nucleolytically-inactive RNA-guided DNA binding protein (Cas9**) by introducing two alanine substitutions (D10A and H840A). Schematic showing that a synthetic guide RNA (sgRNA) can direct Cas9**-effector fusion to a specific locus in the human genome. The sgRNA contains a 20bp guide sequence at the 5' end which specifies the target sequence. On the target genomic DNA, the 20bp target site needs to be followed by a 5'-NGG PAM motif. **(b, c)** Schematics showing the sgRNA target sites in the human *KLF4* and *SOX2* loci respectively. Each target site is indicated by the blue bar and the corresponding PAM sequence is indicated by the magenta bar. **(d, e)** Schematics of the Cas9**-VP64 transcription activator and SID4X-Cas9** transcription repressor constructs. **(f, g)** Cas9**-VP64 and SID4X-Cas9** mediated activation of *KLF4* and repression of *SOX2* respectively. All mRNA levels were measured relative to GFP mock transfected control cells (mean ± s.e.m.; *n* = 3).

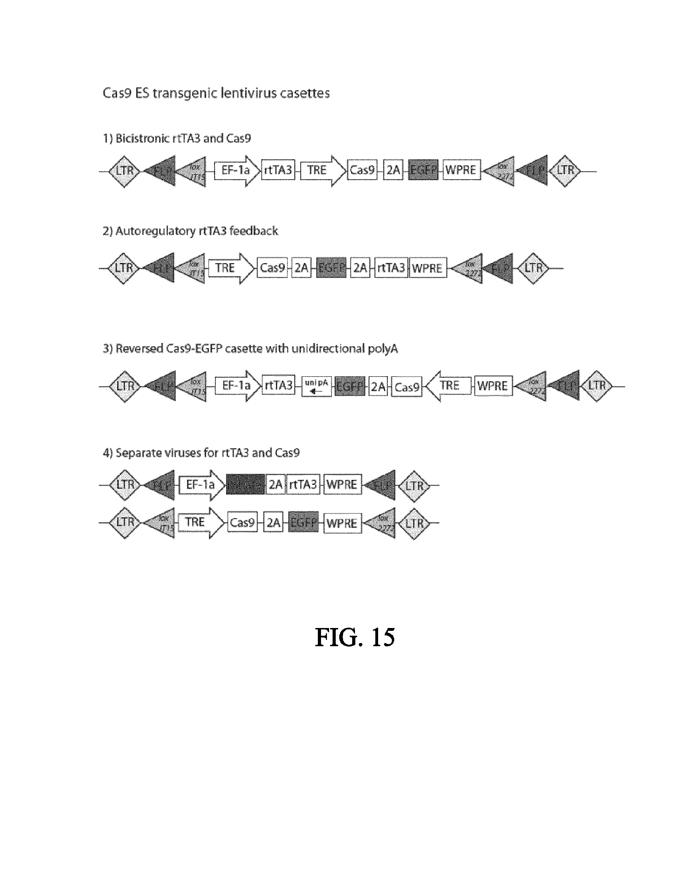

**FIG. 15** depicts Tet Cas9 vector designs for inducible Cas9.

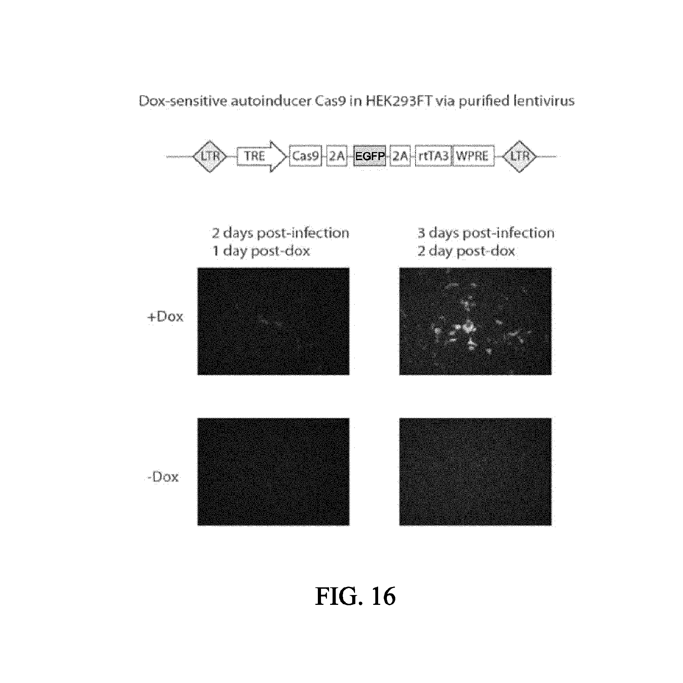

**FIG. 16** depicts a vector and EGFP expression in 293FT cells after Doxycycline induction of Cas9 and EGFP.

### DETAILED DESCRIPTION OF THE INVENTION

The term "nucleic acid" or "nucleic acid sequence" refers to a deoxyribonucleic or ribonucleic oligonucleotide in either single- or double-stranded form. The term encompasses nucleic acids, *i.e.,* oligonucleotides, containing known analogues of natural nucleotides. The term also encompasses nucleic-acid-like structures with synthetic backbones, see, e.g., Eckstein, 1991; Baserga et al., 1992; Milligan, 1993; WO 97/03211; WO 96/39154; Mata, 1997; Strauss-Soukup, 1997; and Samstag, 1996.

As used herein, "recombinant" refers to a polynucleotide synthesized or otherwise manipulated *in vitro* (e.g., "recombinant polynucleotide"), to methods of using recombinant polynucleotides to produce gene products in cells or other biological systems, or to a polypeptide ("recombinant protein") encoded by a recombinant polynucleotide. "Recombinant means" encompasses the ligation of nucleic acids having various coding regions or domains or promoter sequences from different sources into an expression cassette or vector for expression of, e.g., inducible or constitutive expression of polypeptide coding sequences in the vectors of invention.

The term "heterologous" when used with reference to a nucleic acid, indicates that the nucleic acid is in a cell or a virus where it is not normally found in nature; or, comprises two or more subsequences that are not found in the same relationship to each other as normally found in nature, or is recombinantly engineered so that its level of expression, or physical relationship to other nucleic acids or other molecules in a cell, or structure, is not normally found in nature. A similar term used in this context is "exogenous". For instance, a heterologous nucleic acid is typically recombinantly produced, having two or more sequences from unrelated genes arranged in a manner not found in nature; e.g., a human gene operably linked to a promoter sequence inserted into an adenovirus-based vector of the invention. As an example, a heterologous nucleic acid of interest may encode an immunogenic gene product, wherein the adenovirus is administered therapeutically or prophylactically as a carrier or drug-vaccine composition. Heterologous sequences may comprise various combinations of promoters and sequences, examples of which are described in detail herein.

A "therapeutic ligand" may be a substance which may bind to a receptor of a target cell with therapeutic effects.

A "therapeutic effect" may be a consequence of a medical treatment of any kind, the results of which are judged by one of skill in the field to be desirable and beneficial. The "therapeutic effect" may be a behavioral or physiologic change which occurs as a response to the medical treatment. The result may be expected, unexpected, or even an unintended consequence of the medical treatment. A "therapeutic effect" may include, for example, a reduction of symptoms in a subject suffering from infection by a pathogen.

A "target cell" may be a cell in which an alteration in its activity may induce a desired result or response. As used herein, a cell may be an in vitro cell. The cell may be an isolated cell which may not be capable of developing into a complete organism.

A "ligand" may be any substance that binds to and forms a complex with a biomolecule to serve a biological purpose. As used herein, "ligand" may also refer to an "antigen" or "immunogen". As used herein "antigen" and "immunogen" are used interchangeably.

"Expression" of a gene or nucleic acid encompasses not only cellular gene expression, but also the transcription and translation of nucleic acid(s) in cloning systems and in any other context.

As used herein, a "vector" is a tool that allows or facilitates the transfer of an entity from one environment to another. By way of example, some vectors used in recombinant DNA techniques allow entities, such as a segment of DNA (such as a heterologous DNA segment, such as a heterologous cDNA segment), to be transferred into a target cell. The present invention comprehends recombinant vectors that may include viral vectors, bacterial vectors, protozoan vectors, DNA vectors, or recombinants thereof.

With respect to exogenous DNA for expression in a vector (e.g., encoding an epitope of interest and/or an antigen and/or a therapeutic) and documents providing such exogenous DNA, as well as with respect to the expression of transcription and/or translation factors for enhancing expression of nucleic acid molecules, and as to terms such as "epitope of interest", "therapeutic", "immune response", "immunological response", "protective immune response", "immunological composition", "immunogenic composition", and "vaccine composition", *inter alia,* reference is made to U.S. Patent No. 5,990,091 issued November 23, 1999, and WO 98/00166 and WO 99/60164, and the documents cited therein and the documents of record in the prosecution of that patent and those PCT applications. Thus, U.S. Patent No. 5,990,091 and WO 98/00166 and WO 99/60164 and documents cited therein and documents of record in the prosecution of that patent and those PCT applications, and other documents cited herein, may be consulted in the practice of this invention; and, all exogenous nucleic acid molecules, promoters, and vectors cited therein may be used in the practice of this invention. In this regard, mention is also made of U.S. Patents Nos. 6,706,693; 6,716,823; 6,348,450; U.S. Patent Application Serial Nos. 10/424,409; 10/052,323; 10/116,963; 10/346,021; and WO 99/08713, published February 25, 1999, from PCT/US98/16739.

Aspects of the invention comprehend the CRISPR-Cas systems of the invention being delivered into an organism or a cell or to a locus of interest via a delivery system. One means of delivery is via a vector, wherein the vector is a viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided. In some embodiments, one or more of the viral or plasmid vectors may be delivered via nanoparticles, exosomes, microvesciles, or a gene-gun.

As used herein, the terms "drug composition" and "drug", "vaccinal composition", "vaccine", "vaccine composition", "therapeutic composition" and "therapeutic-immunologic composition" cover any composition that induces protection against an antigen or pathogen. In some embodiments, the protection may be due to an inhibition or prevention of infection by a pathogen. In other embodiments, the protection may be induced by an immune response against the antigen(s) of interest, or which efficaciously protects against the antigen; for instance, after administration or injection into the subject, elicits a protective immune response against the targeted antigen or immunogen or provides efficacious protection against the antigen or immunogen expressed from the inventive adenovirus vectors of the invention. The term "pharmaceutical composition" means any composition that is delivered to a subject. In some embodiments, the composition may be delivered to inhibit or prevent infection by a pathogen.

A "therapeutically effective amount" is an amount or concentration of the recombinant vector encoding the gene of interest, that, when administered to a subject, produces a therapeutic response or an immune response to the gene product of interest.

The term "viral vector" as used herein includes but is not limited to retroviruses, adenoviruses, adeno-associated viruses, alphaviruses, and herpes simplex virus.

For the purpose of the invention, the cells may be a eukaryotic cell, advantageously an animal cell, more advantageously a mammalian cell.

The present invention also contemplates a multiplex genome engineering using CRISPR/Cas sytems. Functional elucidation of causal genetic variants and elements requires precise genome editing technologies. The type II prokaryotic CRISPR (clustered regularly interspaced short palindromic repeats) adaptive immune system has been shown to facilitate RNA-guided site-specific DNA cleavage. Applicants engineered two different type II CRISPR systems and demonstrate that Cas9 nucleases can be directed by short RNAs to induce precise cleavage at endogenous genomic loci in human and mouse cells. Cas9 can also be converted into a nicking enzyme to facilitate homology-directed repair with minimal mutagenic activity. Finally, multiple guide sequences can be encoded into a single CRISPR array to enable simultaneous editing of several sites within the mammalian genome, demonstrating easy programmability and wide applicability of the CRISPR technology.

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In some embodiments, one or more elements of a CRISPR system is derived from a type I, type II, or type III CRISPR system. In some embodiments, one or more elements of a CRISPR system is derived from a particular organism comprising an endogenous CRISPR system, such as *Streptococcus pyogenes.* In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides. In some embodiments, a target sequence is located in the nucleus or cytoplasm of a cell.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, all or a portion of the tracr sequence may also form part of a CRISPR complex, such as by hybridization to all or a portion of a tracr mate sequence that is operably linked to the guide sequence. In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments, the CRISPR enzyme, guide sequence, tracr mate sequence, and tracr sequence are operably linked to and expressed from the same promoter.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments, a vector comprises a regulatory element operably linked to an enzyme-coding sequence encoding a CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In some embodiments, the unmodified CRISPR enzyme has DNA cleavage activity, such as Cas9. In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from *S. pyogenes* converts Cas9 from a nuclease that cleaves both strands to a nickase (cleaves a single strand). Other examples of mutations that render Cas9 a nickase include, without limitation, H840A, N854A, and N863A. As a further example, two or more catalytic domains of Cas9 (RuvC I, RuvC II, and RuvC III) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human primate. In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ (visited Jul. 9, 2002), and these tables can be adapted in a number of ways. See Nakamura, Y., et al. "Codon usage tabulated from the international DNA sequence databases: status for the year 2000"Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments, a vector encodes a CRISPR enzyme comprising one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the Nor C-terminus. Non-limiting examples of NLSs include an NLS sequence derived from: the NLS of the SV40 virus large T-antigen, having the amino acid sequence PKKKRKV; the NLS from nucleoplasmin (e.g. the nucleoplasmin bipartite NLS with the sequence KRPAATKKAGQAKKKK); the c-myc NLS having the amino acid sequence PAAKRVKLD or RQRRNELKRSP; the hRNPA1 M9 NLS having the sequence NQSSNFGPMKGGNFGGRSSGPYGGGGQYFAKPRNQGGY; the sequence RMRIZFKNKGKDTAELRRRRVEVSVELRKAKKDEQILKRRNV of the IBB domain from importin-alpha; the sequences VSRKRPRP and PPKKARED of the myoma T protein; the sequence POPKKKPL of human p53; the sequence SALIKKKKKMAP of mouse c-abl IV; the sequences DRLRR and PKQKKRK of the influenza virus NS1; the sequence RKLKKKIKKL of the Hepatitis virus delta antigen; the sequence REKKKFLKRR of the mouse Mx1 protein; the sequence KRKGDEVDGVDEVAKKKSKK of the human poly(ADP-ribose) polymerase; and the sequence RKCLQAGMNLEARKTKK of the steroid hormone receptors (human) glucocorticoid.

In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

In another embodiment of the present invention, the invention relates to an inducible CRISPR which may comprise an inducible Cas9.

The CRISPR system may be encoded within a vector system which may comprise one or more vectors which may comprise I. a first regulatory element operably linked to a CRISPR/Cas system chimeric RNA (chiRNA) polynucleotide sequence, wherein the polynucleotide sequence may comprise (a) a guide sequence capable of hybridizing to a target sequence in a eukaryotic cell, (b) a tracr mate sequence, and (c) a tracr sequence, and II. a second regulatory element operably linked to an enzyme-coding sequence encoding a CRISPR enzyme which may comprise at least one or more nuclear localization sequences, wherein (a), (b) and (c) are arranged in a 5' to 3'orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex may comprise the CRISPR enzyme complexed with (1) the guide sequence that is hybridized to the target sequence, and (2) the tracr mate sequence that is hybridized to the tracr sequence, wherein the enzyme coding sequence encoding the CRISPR enzyme further encodes a heterologous functional domain.

In an advantageous embodiment, the inducible Cas9 may be prepared in a lentivirus. For example, FIG. 61 depicts Tet Cas9 vector designs and FIG. 62 depicts a vector and EGFP expression in 293FT cells. In particular, an inducible tetracycline system is contemplated for an inducible CRISPR..The vector may be designed as described in Markusic et al., Nucleic Acids Research, 2005, Vol. 33, No. 6 e63. The tetracycline-dependent transcriptional regulatory system is based on the Escherichia coli Tn10 Tetracycline resistance operator consisting of the tetracycline repressor protein (TetR) and a specific DNA-binding site, the tetracycline operator sequence (TetO). In the absence of tetracycline, TetR dimerizes and binds to the TetO. Tetracycline or doxycycline (a tetracycline derivative) can bind and induce a conformational change in the TetR leading to its disassociation from the TetO. In an advantageous embodiment, the vector may be a single Tet-On lentiviral vector with autoregulated rtTA expression for regulated expression of the CRISPR complex. Tetracycline or doxycycline may be contemplated for activating the inducible CRISPR complex.

In another embodiment, a cumate gene-switch system is contemplated for an inducible CRISPR. A similar system as described in Mullick et al., BMC Biotechnology 2006, 6:43 doi:10.1186/1472-6750-6-43. The inducible cumate system involves regulatory mechanisms of bacterial operons (cmt and cym) to regulate gene expression in mammalian cells using three different strategies. In the repressor configuration, regulation is mediated by the binding of the repressor (CymR) to the operator site (CuO), placed downstream of a strong constitutive promoter. Addition of cumate, a small molecule, relieves the repression. In the transactivator configuration, a chimaeric transactivator (cTA) protein, formed by the fusion of CymR with the activation domain of VP16, is able to activate transcription when bound to multiple copies of CuO, placed upstream of the CMV minimal promoter. Cumate addition abrogates DNA binding and therefore transactivation by cTA. The invention also contemplates a reverse cumate activator (rcTA), which activates transcription in the presence rather than the absence of cumate. CymR may be used as a repressor that reversibly blocks expression from a strong promoter, such as CMV. Certain aspects of the Cumate repressor/operator system are further described in US patent No. 7745592.

Other inducible systems are contemplated such as, but not limited to, regulation by heavy-metals [Mayo KE et al., Cell 1982, 29:99-108; Searle PF et al., Mol Cell Biol 1985, 5:1480-1489 and Brinster RL et al., Nature (London) 1982, 296:39-42], steroid hormones [Hynes NE et al., Proc Natl Acad Sci USA 1981, 78:2038-2042; Klock G et al., Nature (London) 1987, 329:734-736 and Lee F et al., Nature (London) 1981, 294:228-232.], heat shock [Nouer L: Heat Shock Response. Boca Raton, FL: CRC; 1991] and other reagents have been developed [Mullick A, Massie B: Transcription, translation and the control of gene expression. In Encyclopedia of Cell Technology Edited by: Speir RE. Wiley; 2000:1140-1164 and Fussenegger M, . Biotechnol Prog 2001, 17:1-51]. However, there are limitations with these inducible mammalian promoters such as "leakiness" of the "off' state and pleiotropic effects of inducers (heat shock, heavy metals, glucocorticoids etc.). The use of insect hormones (ecdysone) has been proposed in an attempt to reduce the interference with cellular processes in mammalian cells [No D et al., Proc Natl Acad Sci USA 1996, 93:3346-3351]. Another elegant system uses rapamycin as the inducer [Rivera VM et al., Nat Med 1996, 2:1028-1032] but the role of rapamycin as an immunosuppressant was a major limitation to its use in vivo and therefore it was necessary to find a biologically inert compound [Saez E et al., Proc Natl Acad Sci USA 2000, 97:14512-14517] for the control of gene expression.

The present invention also relates to nucleic acid encoding the polypeptides of the present invention. The nucleic acid may comprise a promoter, advantageously human Synapsin I promoter (hSyn). In a particularly advantageous embodiment, the nucleic acid may be packaged into an adeno associated viral vector (AAV).

Also contemplated by the present invention are recombinant vectors and recombinant adenoviruses that may comprise subviral particles from more than one adenovirus serotype. For example, it is known that adenovirus vectors may display an altered tropism for specific tissues or cell types (Havenga, M.J.E. et al., 2002), and therefore, mixing and matching of different adenoviral capsids, i.e., fiber, or penton proteins from various adenoviral serotypes may be advantageous. Modification of the adenoviral capsids, including fiber and penton may result in an adenoviral vector with a tropism that is different from the unmodified adenovirus. Adenovirus vectors that are modified and optimized in their ability to infect target cells may allow for a significant reduction in the therapeutic or prophylactic dose, resulting in reduced local and disseminated toxicity.

Viral vector gene delivery systems are commonly used in gene transfer and gene therapy applications. Different viral vector systems have their own unique advantages and disadvantages. Viral vectors that may be used to express the pathogen-derived ligand of the present invention include but are not limited to adenoviral vectors, adeno-associated viral vectors, alphavirus vectors, herpes simplex viral vectors, and retroviral vectors, described in more detail below.

Additional general features of adenoviruses are such that the biology of the adenovirus is characterized in detail; the adenovirus is not associated with severe human pathology; the adenovirus is extremely efficient in introducing its DNA into the host cell; the adenovirus may infect a wide variety of cells and has a broad host range; the adenovirus may be produced in large quantities with relative ease; and the adenovirus may be rendered replication defective and/or non-replicating by deletions in the early region 1 ("E1") of the viral genome.

Adenovirus is a non-enveloped DNA virus. The genome of adenovirus is a linear double-stranded DNA molecule of approximately 36,000 base pairs ("bp") with a 55-kDa terminal protein covalently bound to the 5'-terminus of each strand. The adenovirus DNA contains identical inverted terminal repeats ("ITRs") of about 100 bp, with the exact length depending on the serotype. The viral origins of replication are located within the ITRs exactly at the genome ends. DNA synthesis occurs in two stages. First, replication proceeds by strand displacement, generating a daughter duplex molecule and a parental displaced strand. The displaced strand is single stranded and may form a "panhandle" intermediate, which allows replication initiation and generation of a daughter duplex molecule. Alternatively, replication may proceed from both ends of the genome simultaneously, obviating the requirement to form the panhandle structure.

During the productive infection cycle, the viral genes are expressed in two phases: the early phase, which is the period up to viral DNA replication, and the late phase, which coincides with the initiation of viral DNA replication. During the early phase, only the early gene products, encoded by regions E1, E2, E3 and E4, are expressed, which carry out a number of functions that prepare the cell for synthesis of viral structural proteins (Berk, A.J., 1986). During the late phase, the late viral gene products are expressed in addition to the early gene products and host cell DNA and protein synthesis are shut off. Consequently, the cell becomes dedicated to the production of viral DNA and of viral structural proteins (Tooze, J., 1981).

The E1 region of adenovirus is the first region of adenovirus expressed after infection of the target cell. This region consists of two transcriptional units, the E1A and E1B genes, both of which are required for oncogenic transformation of primary (embryonal) rodent cultures. The main functions of the E1A gene products are to induce quiescent cells to enter the cell cycle and resume cellular DNA synthesis, and to transcriptionally activate the E1B gene and the other early regions (E2, E3 and E4) of the viral genome. Transfection of primary cells with the E1A gene alone may induce unlimited proliferation (immortalization), but does not result in complete transformation. However, expression of E1A, in most cases, results in induction of programmed cell death (apoptosis), and only occasionally is immortalization obtained (Jochemsen et al., 1987). Co-expression of the E1B gene is required to prevent induction of apoptosis and for complete morphological transformation to occur. In established immortal cell lines, high-level expression of E1A may cause complete transformation in the absence of E1B (Roberts, B.E. et al., 1985).

The E1B encoded proteins assist E1A in redirecting the cellular functions to allow viral replication. The E1B 55 kD and E4 33 kD proteins, which form a complex that is essentially localized in the nucleus, function in inhibiting the synthesis of host proteins and in facilitating the expression of viral genes. Their main influence is to establish selective transport of viral mRNAs from the nucleus to the cytoplasm, concomitantly with the onset of the late phase of infection. The E1B 21 kD protein is important for correct temporal control of the productive infection cycle, thereby preventing premature death of the host cell before the virus life cycle has been completed. Mutant viruses incapable of expressing the E1B 21 kD gene product exhibit a shortened infection cycle that is accompanied by excessive degradation of host cell chromosomal DNA (deg-phenotype) and in an enhanced cytopathic effect (cyt-phenotype; Telling et al., 1994). The deg and cyt phenotypes are suppressed when in addition the E1A gene is mutated, indicating that these phenotypes are a function of E1A (White, E. et al., 1988). Furthermore, the E1B 21 kDa protein slows down the rate by which E1A switches on the other viral genes. It is not yet known by which mechanisms EIB 21 kD quenches these E1A dependent functions.

In contrast to, for example, retroviruses, adenoviruses do not efficiently integrate into the host cell's genome, are able to infect non-dividing cells, and are able to efficiently transfer recombinant genes *in vivo* (Brody et al., 1994). These features make adenoviruses attractive candidates for *in vivo* gene transfer of, for example, an antigen or immunogen of interest into cells, tissues or subjects in need thereof.

Adenovirus vectors containing multiple deletions are preferred to both increase the carrying capacity of the vector and reduce the likelihood of recombination to generate replication competent adenovirus (RCA). Where the adenovirus contains multiple deletions, it is not necessary that each of the deletions, if present alone, would result in a replication defective and/or non-replicating adenovirus. As long as one of the deletions renders the adenovirus replication defective or non-replicating, the additional deletions may be included for other purposes, e.g., to increase the carrying capacity of the adenovirus genome for heterologous nucleotide sequences. Preferably, more than one of the deletions prevents the expression of a functional protein and renders the adenovirus replication defective and/or non-replicating and/or attenuated. More preferably, all of the deletions are deletions that would render the adenovirus replication-defective and/or non-replicating and/or attenuated. However, the invention also encompasses adenovirus and adenovirus vectors that are replication competent and/or wild-type, *i.e.* comprises all of the adenoviral genes necessary for infection and replication in a subject.

Embodiments of the invention employing adenovirus recombinants may include E1-defective or deleted, or E3-defective or deleted, or E4-defective or deleted or adenovirus vectors comprising deletions of E1 and E3, or E1 and E4, or E3 and E4, or E1, E3, and E4 deleted, or the "gutless" adenovirus vector in which all viral genes are deleted. The adenovirus vectors may comprise mutations in E1, E3, or E4 genes, or deletions in these or all adenoviral genes. The E1 mutation raises the safety margin of the vector because E1-defective adenovirus mutants are said to be replication-defective and/or non-replicating in non-permissive cells, and are, at the very least, highly attenuated. The E3 mutation enhances the immunogenicity of the antigen by disrupting the mechanism whereby adenovirus down-regulates MHC class I molecules. The E4 mutation reduces the immunogenicity of the adenovirus vector by suppressing the late gene expression, thus may allow repeated re-vaccination utilizing the same vector. The present invention comprehends adenovirus vectors of any serotype or serogroup that are deleted or mutated in E1, or E3, or E4, or E1 and E3, or E1 and E4. Deletion or mutation of these adenoviral genes result in impaired or substantially complete loss of activity of these proteins.

The "gutless" adenovirus vector is another type of vector in the adenovirus vector family. Its replication requires a helper virus and a special human 293 cell line expressing both E1 a and Cre, a condition that does not exist in a natural environment; the vector is deprived of all viral genes, thus the vector as a vaccine carrier is non-immunogenic and may be inoculated multiple times for re-vaccination. The "gutless" adenovirus vector also contains 36 kb space for accommodating antigen or immunogen(s) of interest, thus allowing co-delivery of a large number of antigen or immunogens into cells.

Adeno-associated virus (AAV) is a single-stranded DNA parvovirus which is endogenous to the human population. Although capable of productive infection in cells from a variety of species, AAV is a dependovirus, requiring helper functions from either adenovirus or herpes virus for its own replication. In the absence of helper functions from either of these helper viruses, AAV will infect cells, uncoat in the nucleus, and integrate its genome into the host chromosome, but will not replicate or produce new viral particles.

The genome of AAV has been cloned into bacterial plasmids and is well characterized. The viral genome consists of 4682 bases which include two terminal repeats of 145 bases each. These terminal repeats serve as origins of DNA replication for the virus. Some investigators have also proposed that they have enhancer functions. The rest of the genome is divided into two functional domains. The left portion of the genome codes for the rep functions which regulate viral DNA replication and vital gene expression. The right side of the vital genome contains the cap genes that encode the structural capsid proteins VP1, VP2 and VP3. The proteins encoded by both the rep and cap genes function in trans during productive AAV replication.

AAV is considered an ideal candidate for use as a transducing vector, and it has been used in this manner. Such AAV transducing vectors comprise sufficient cis-acting functions to replicate in the presence of adenovirus or herpes virus helper functions provided in trans. Recombinant AAV (rAAV) have been constructed in a number of laboratories and have been used to carry exogenous genes into cells of a variety of lineages. In these vectors, the AAV cap and/or rep genes are deleted from the viral genome and replaced with a DNA segment of choice. Current vectors may accommodate up to 4300 bases of inserted DNA.

To produce rAAV, plasmids containing the desired vital construct are transfected into adenovirus-infected cells. In addition, a second helper plasmid is cotransfected into these cells to provide the AAV rep and cap genes which are obligatory for replication and packaging of the recombinant viral construct. Under these conditions, the rep and cap proteins of AAV act in trans to stimulate replication and packaging of the rAAV construct. Three days after transfection, rAAV is harvested from the cells along with adenovirus. The contaminating adenovirus is then inactivated by heat treatment.

Herpes Simplex Virus 1 (HSV-1) is an enveloped, double-stranded DNA virus with a genome of 153 kb encoding more than 80 genes. Its wide host range is due to the binding of viral envelope glycoproteins to the extracellular heparin sulphate molecules found in cell membranes (WuDunn & Spear, 1989). Internalization of the virus then requires envelope glycoprotein gD and fibroblast growth factor receptor (Kaner, 1990). HSV is able to infect cells lytically or may establish latency. HSV vectors have been used to infect a wide variety of cell types (Lowenstein, 1994; Huard, 1995; Miyanohara, 1992; Liu, 1996; Goya, 1998).

There are two types of HSV vectors, called the recombinant HSV vectors and the amplicon vectors. Recombinant HSV vectors are generated by the insertion of transcription units directly into the HSV genome, through homologous recombination events. The amplicon vectors are based on plasmids bearing the transcription unit of choice, an origin of replication, and a packaging signal.

HSV vectors have the obvious advantages of a large capacity for insertion of foreign genes, the capacity to establish latency in neurons, a wide host range, and the ability to confer transgene expression to the CNS for up to 18 months (Carpenter & Stevens, 1996).

Retroviruses are enveloped single-stranded RNA viruses, which have been widely used in gene transfer protocols. Retroviruses have a diploid genome of about 7-10 kb, composed of four gene regions termed *gag, pro, pol* and *env.* These gene regions encode for structural capsid proteins, viral protease, integrase and viral reverse transcriptase, and envelope glycoproteins, respectively. The genome also has a packaging signal and *cis*-acting sequences, termed long-terminal repeats (LTRs), at each end, which have a role in transcriptional control and integration.

The most commonly used retroviral vectors are based on the Moloney murine leukaemia virus (Mo-MLV) and have varying cellular tropisms, depending on the receptor binding surface domain of the envelope glycoprotein.

Recombinant retroviral vectors are deleted from all retroviral genes, which are replaced with marker or therapeutic genes, or both. To propagate recombinant retroviruses, it is necessary to provide the viral genes, *gag, pol* and *env* in *trans.*

Lentiviruses are complex retroviruses that have the ability to infect and express their genes in both mitotic and post-mitotic cells. The most commonly known lentivirus is the human immunodeficiency virus (HIV), which uses the envelope glycoproteins of other viruses to target a broad range of cell types.

Alphaviruses, including the prototype Sindbis virus (SIN), Semliki Forest virus (SFV), and Venezuelan equine encephalitis virus (VEE), constitute a group of enveloped viruses containing plus-stranded RNA genomes within icosahedral capsids.

The viral vectors of the present invention are useful for the delivery of nucleic acids expressing antigens or immunogens to cells both *in vitro* and *in vivo.* In particular, the inventive vectors may be advantageously employed to deliver or transfer nucleic acids to cells, more preferably mammalian cells. Nucleic acids of interest include nucleic acids encoding peptides and proteins, preferably therapeutic (e.g., for medical or veterinary uses) or immunogenic (e.g., for vaccines) peptides or proteins.

Preferably, the codons encoding the antigen or immunogen of interest are "optimized" codons, i.e., the codons are those that appear frequently in, e.g.., highly expressed genes in the subject's species, instead of those codons that are frequently used by, for example, an influenza virus. Such codon usage provides for efficient expression of the antigen or immunogen in animal cells. In other embodiments, for example, when the antigen or immunogen of interest is expressed in bacteria, yeast or another expression system, the codon usage pattern is altered to represent the codon bias for highly expressed genes in the organism in which the antigen or immunogen is being expressed. Codon usage patterns are known in the literature for highly expressed genes of many species (e.g., Nakamura et al., 1996; Wang et al., 1998; McEwan et al. 1998).

As a further alternative, the viral vectors may be used to infect a cell in culture to express a desired gene product, e.g., to produce a protein or peptide of interest. Preferably, the protein or peptide is secreted into the medium and may be purified therefrom using routine techniques known in the art. Signal peptide sequences that direct extracellular secretion of proteins are known in the art and nucleotide sequences encoding the same may be operably linked to the nucleotide sequence encoding the peptide or protein of interest by routine techniques known in the art. Alternatively, the cells may be lysed and the expressed recombinant protein may be purified from the cell lysate. Preferably, the cell is an animal cell, more preferably a mammalian cell. Also preferred are cells that are competent for transduction by particular viral vectors of interest. Such cells include PER.C6 cells, 911 cells, and HEK293 cells.

A culture medium for culturing host cells includes a medium commonly used for tissue culture, such as M199-earle base, Eagle MEM (E-MEM), Dulbecco MEM (DMEM), SC-UCM102, UP-SFM (GIBCO BRL), EX-CELL302 (Nichirei), EX-CELL293-S (Nichirei), TFBM-01 (Nichirei), ASF104, among others. Suitable culture media for specific cell types may be found at the American Type Culture Collection (ATCC) or the European Collection of Cell Cultures (ECACC). Culture media may be supplemented with amino acids such as L-glutamine, salts, anti-fungal or anti-bacterial agents such as Fungizone®, penicillin-streptomycin, animal serum, and the like. The cell culture medium may optionally be serum-free.

Therapeutic or diagnostic compositions of the invention are administered to an individual in amounts sufficient to treat or diagnose disorders. The effective amount may vary according to a variety of factors such as the individual's condition, weight, sex and age. Other factors include the mode of administration.

The pharmaceutical compositions may be provided to the individual by a variety of routes such as subcutaneous, topical, oral and intramuscular.

Compounds identified according to the methods disclosed herein may be used alone at appropriate dosages. Alternatively, co-administration or sequential administration of other agents may be desirable.

The present invention also has the objective of providing suitable topical, oral, systemic and parenteral pharmaceutical formulations for use in the novel methods of treatment of the present invention. The compositions containing compounds identified according to this invention as the active ingredient may be administered in a wide variety of therapeutic dosage forms in conventional vehicles for administration. For example, the compounds may be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts.

Advantageously, compounds of the present invention may be administered in a single daily dose, or the total daily dosage may be administered in divided doses of two, three or four times daily. Furthermore, compounds for the present invention may be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

For combination treatment with more than one active agent, where the active agents are in separate dosage formulations, the active agents may be administered concurrently, or they each may be administered at separately staggered times.

The dosage regimen utilizing the compounds of the present invention is selected in accordance with a variety of factors including type, species, age, weight, sex and medical condition of the patient; the severity of the condition to be treated; the route of administration; the renal, hepatic and cardiovascular function of the one patient; and the particular compound thereof employed. A physician of ordinary skill may readily determine and prescribe the effective amount of the drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.

Although the present invention and its advantages have been described in detail, it should be understood that various changes, substitutions and alterations may be made herein without departing from the scope of the invention as defined in the appended claims.

The present invention will be further illustrated in the following Examples which are given for illustration purposes only and are not intended to limit the invention in any way.

### Examples

### Example 1

### Multiplex Genome Engineering Using CRISPR/Cas Systems

Functional elucidation of causal genetic variants and elements requires precise genome editing technologies. The type II prokaryotic CRISPR (clustered regularly interspaced short palindromic repeats) adaptive immune system has been shown to facilitate RNA-guided site-specific DNA cleavage. Applicants engineered two different type II CRISPR systems and demonstrate that Cas9 nucleases can be directed by short RNAs to induce precise cleavage at endogenous genomic loci in human and mouse cells. Cas9 can also be converted into a nicking enzyme to facilitate homology-directed repair with minimal mutagenic activity. Finally, multiple guide sequences can be encoded into a single CRISPR array to enable simultaneous editing of several sites within the mammalian genome, demonstrating easy programmability and wide applicability of the CRISPR technology.

Prokaryotic CRISPR adaptive immune systems can be reconstituted and engineered to mediate multiplex genome editing in mammalian cells.

Precise and efficient genome targeting technologies are needed to enable systematic reverse engineering of causal genetic variations by allowing selective perturbation of individual genetic elements. Although genome-editing technologies such as designer zinc fingers (ZFs) (M. H. Porteus, D. Baltimore, Chimeric nucleases stimulate gene targeting in human cells. Science 300, 763 (May 2, 2003); J. C. Miller et al., An improved zinc-finger nuclease architecture for highly specific genome editing. Nat Biotechnol 25, 778 (Jul, 2007); J. D. Sander et al., Selection-free zinc-finger-nuclease engineering by context-dependent assembly (CoDA). Nat Methods 8, 67 (Jan, 2011) and A. J. Wood et al., Targeted genome editing across species using ZFNs and TALENs. Science 333, 307 (Jul 15, 2011)), transcription activator-like effectors (TALEs) (A. J. Wood et al., Targeted genome editing across species using ZFNs and TALENs. Science 333, 307 (Jul 15, 2011); M. Christian et al., Targeting DNA double-strand breaks with TAL effector nucleases. Genetics 186, 757 (Oct, 2010); F. Zhang et al., Efficient construction of sequence-specific TAL effectors for modulating mammalian transcription. Nat Biotechnol 29, 149 (Feb, 2011); J. C. Miller et al., A TALE nuclease architecture for efficient genome editing. Nat Biotechnol 29, 143 (Feb, 2011); D. Reyon et al., FLASH assembly of TALENs for high-throughput genome editing. Nat Biotechnol 30, 460 (May, 2012); J. Boch et al., Breaking the code of DNA binding specificity of TAL-type III effectors. Science 326, 1509 (Dec 11, 2009) and M. J. Moscou, A. J. Bogdanove, A simple cipher governs DNA recognition by TAL effectors. Science 326, 1501 (Dec 11, 2009)), and homing meganucleases (B. L. Stoddard, Homing endonuclease structure and function. Quarterly reviews of biophysics 38, 49 (Feb, 2005)) have begun to enable targeted genome modifications, there remains a need for new technologies that are scalable, affordable, and easy to engineer. Here, Applicants report the development of a new class of precision genome engineering tools based on the RNA-guided Cas9 nuclease (M. Jinek et al., A programmable dual-RNA-guided DNA endonuclease in adaptive bacterial immunity. Science 337, 816 (Aug 17, 2012); G. Gasiunas, R. Barrangou, P. Horvath, V. Siksnys, Cas9-crRNA ribonucleoprotein complex mediates specific DNA cleavage for adaptive immunity in bacteria. Proc Natl Acad Sci USA 109, E2579 (Sep 25, 2012) and J. E. Garneau et al., The CRISPR/Cas bacterial immune system cleaves bacteriophage and plasmid DNA. Nature 468, 67 (Nov 4, 2010)) from the type II prokaryotic CRISPR adaptive immune system (H. Deveau, J. E. Garneau, S. Moineau, CRISPR/Cas system and its role in phage-bacteria interactions. Annual review of microbiology 64, 475 (2010); P. Horvath, R. Barrangou, CRISPR/Cas, the immune system of bacteria and archaea. Science 327, 167 (Jan 8, 2010); K. S. Makarova et al., Evolution and classification of the CRISPR-Cas systems. Nat Rev Microbiol 9, 467 (Jun, 2011) and D. Bhaya, M. Davison, R. Barrangou, CRISPR-Cas systems in bacteria and archaea: versatile small RNAs for adaptive defense and regulation. Annu Rev Genet 45, 273 (2011)).

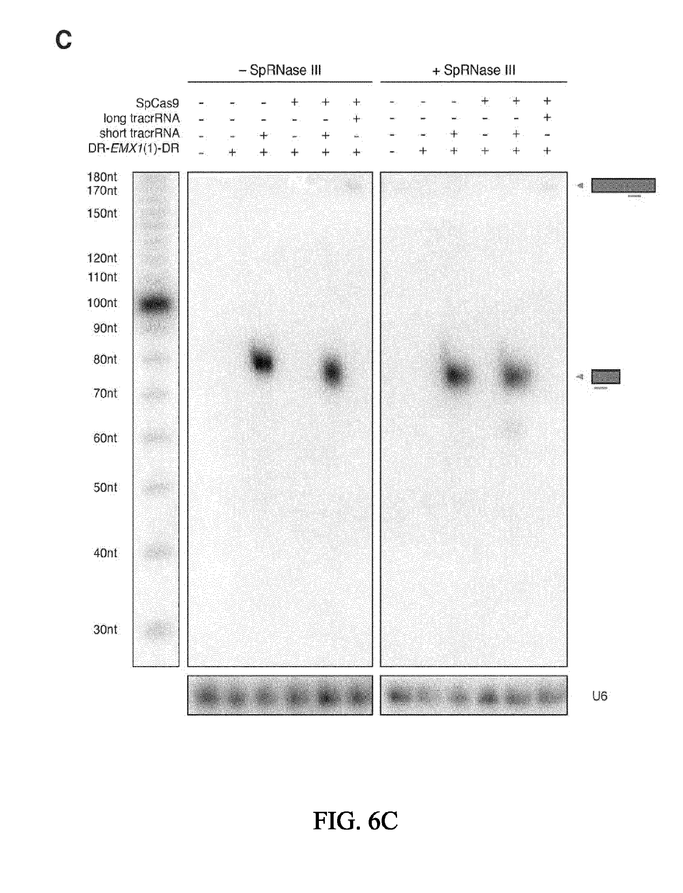

The *Streptococcus pyogenes* SF370 type II CRISPR locus consists of four genes, including the Cas9 nuclease, as well as two non-coding RNAs: tracrRNA and a pre-crRNA array containing nuclease guide sequences (spacers) interspaced by identical direct repeats (DRs) (FIG. 5) (E. Deltcheva et al., CRISPR RNA maturation by trans-encoded small RNA and host factor RNase III. Nature 471, 602 (Mar 31, 2011)). Applicants sought to harness this prokaryotic RNA-programmable nuclease system to introduce targeted double stranded breaks (DSBs) in mammalian chromosomes through heterologous expression of the key components. It has been previously shown that expression of tracrRNA, pre-crRNA, host factor RNase III, and Cas9 nuclease are necessary and sufficient for cleavage of DNA *in vitro* (M. Jinek et al., A programmable dual-RNA-guided DNA endonuclease in adaptive bacterial immunity. Science 337, 816 (Aug 17, 2012) and G. Gasiunas, R. Barrangou, P. Horvath, V. Siksnys, Cas9-crRNA ribonucleoprotein complex mediates specific DNA cleavage for adaptive immunity in bacteria. Proc Natl Acad Sci USA 109, E2579 (Sep 25, 2012)) and in prokaryotic cells (R. Sapranauskas et al., The Streptococcus thermophilus CRISPR/Cas system provides immunity in Escherichia coli. Nucleic Acids Res 39, 9275 (Nov, 2011) and A. H. Magadan, M. E. Dupuis, M. Villion, S. Moineau, Cleavage of phage DNA by the Streptococcus thermophilus CRISPR3-Cas system. PLoS One 7, e40913 (2012)). Applicants codon optimized the *S. pyogenes Cas9 (SpCas9)* and *RNase III (SpRNase III)* and attached nuclear localization signals (NLS) to ensure nuclear compartmentalization in mammalian cells. Expression of these constructs in human 293FT cells revealed that two NLSs are required for targeting SpCas9 to the nucleus (FIG. 1A). To reconstitute the non-coding RNA components of CRISPR, Applicants expressed an 89-nucleotide (nt) tracrRNA (FIG. 6) under the RNA polymerase III U6 promoter (FIG. 1B). Similarly, Applicants used the U6 promoter to drive the expression of a pre-crRNA array comprising a single guide spacer flanked by DRs (FIG. 1B). Applicants designed an initial spacer to target a 30-basepair (bp) site (protospacer) in the human *EMX1* locus that precedes an NGG, the requisite protospacer adjacent motif (PAM) (FIG. 1C and FIG. 5) (H. Deveau et al., Phage response to CRISPR-encoded resistance in Streptococcus thermophilus. J Bacteriol 190, 1390 (Feb, 2008) and F. J. Mojica, C. Diez-Villasenor, J. Garcia-Martinez, C. Almendros, Short motif sequences determine the targets of the prokaryotic CRISPR defence system. Microbiology 155, 733 (Mar, 2009)).

To test whether heterologous expression of the CRISPR system (SpCas9, SpRNase III, tracrRNA, and pre-crRNA) can achieve targeted cleavage of mammalian chromosomes, Applicants transfected 293FT cells with different combinations of CRISPR components. Since DSBs in mammalian DNA are partially repaired by the indel-forming non-homologous end joining (NHEJ) pathway, Applicants used the SURVEYOR assay (FIG. 7) to detect endogenous target cleavage (FIG. 1D and FIG. 6B). Co-transfection of all four required CRISPR components resulted in efficient cleavage of the protospacer (FIG. 1D and FIG. 6B), which is subsequently verified by Sanger sequencing (FIG. IE). Interestingly, SpRNase III was not necessary for cleavage of the protospacer (FIG. 1D), and the 89-nt tracrRNA is processed in its absence (FIG. 6C). Similarly, maturation of pre-crRNA does not require RNase III (FIG. 1D and FIG. 8), suggesting that there may be endogenous mammalian RNases that assist in pre-crRNA maturation (M. Jinek, J. A. Doudna, A three-dimensional view of the molecular machinery of RNA interference. Nature 457, 405 (Jan 22, 2009); C. D. Malone, G. J. Hannon, Small RNAs as guardians of the genome. Cell 136, 656 (Feb 20, 2009) and G. Meister, T. Tuschl, Mechanisms of gene silencing by double-stranded RNA. Nature 431, 343 (Sep 16, 2004)). Removing any of the remaining RNA or Cas9 components abolished the genome cleavage activity of the CRISPR system (FIG. 1D). These results define a minimal three-component system for efficient CRISPR-mediated genome modification in mammalian cells.

Next, Applicants explored the generalizability of CRISPR-mediated cleavage in eukaryotic cells by targeting additional protospacers within the *EMX1* locus (FIG. 2A). To improve co-delivery, Applicants designed an expression vector to drive both pre-crRNA and SpCas9 (FIG. 9). In parallel, Applicants adapted a chimeric crRNA-tracrRNA hybrid (FIG. 2B, top) design recently validated *in vitro* (M. Jinek et al., A programmable dual-RNA-guided DNA endonuclease in adaptive bacterial immunity. Science 337, 816 (Aug 17, 2012)), where a mature crRNA is fused to a partial tracrRNA via a synthetic stem-loop to mimic the natural crRNA:tracrRNA duplex (FIG. 2B, bottom). Applicants observed cleavage of all protospacer targets when SpCas9 is co-expressed with pre-crRNA (DR-spacer-DR) and tracrRNA. However, not all chimeric RNA designs could facilitate cleavage of their genomic targets (FIG. 2C, Table 1). Applicants then tested targeting of additional genomic loci in both human and mouse cells by designing pre-crRNAs and chimeric RNAs targeting the human *PVALB* and the mouse *Th* loci (FIG. 10). Applicants achieved efficient modification at all three mouse *Th* and one *PVALB* targets using the crRNA:tracrRNA design, thus demonstrating the broad applicability of the CRISPR system in modifying different loci across multiple organisms (Table 1). For the same protospacer targets, cleavage efficiencies of chimeric RNAs were either lower than those of crRNA:tracrRNA duplexes or undetectable. This may be due to differences in the expression and stability of RNAs, degradation by endogenous RNAi machinery, or secondary structures leading to inefficient Cas9 loading or target recognition.

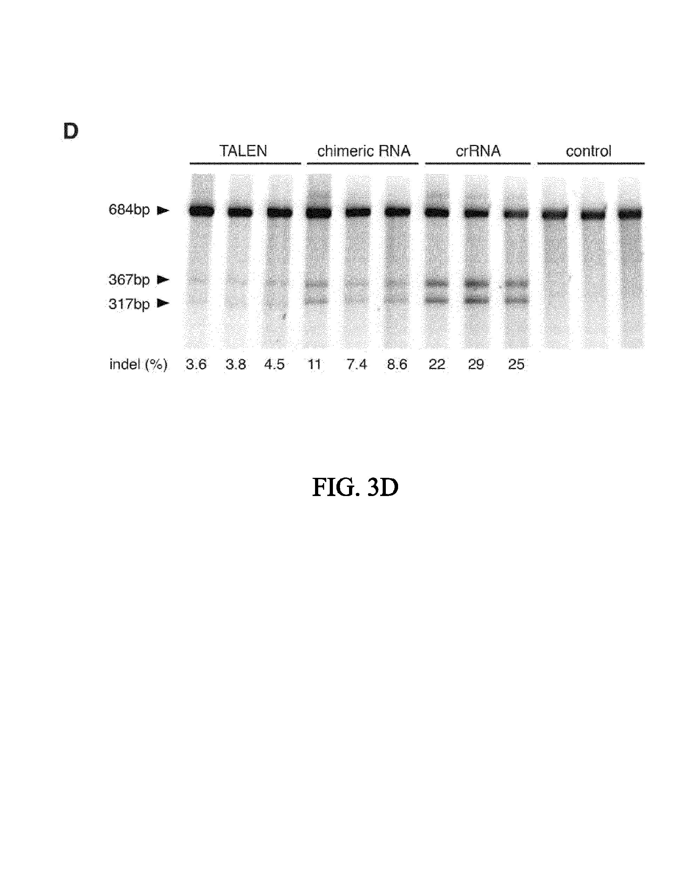

Effective genome editing requires that nucleases target specific genomic loci with both high precision and efficiency. To investigate the specificity of CRISPR-mediated cleavage, Applicants analyzed single-nucleotide mismatches between the spacer and its mammalian protospacer target (FIG. 3A). Applicants observed that single-base mismatch up to 12-bp 5' of the PAM completely abolished genomic cleavage by SpCas9, whereas spacers with mutations farther upstream retained activity against the protospacer target (FIG. 3B). This is consistent with previous bacterial and *in vitro* studies of Cas9 specificity (M. Jinek et al., A programmable dual-RNA-guided DNA endonuclease in adaptive bacterial immunity. Science 337, 816 (Aug 17, 2012) and R. Sapranauskas et al., The Streptococcus thermophilus CRISPR/Cas system provides immunity in Escherichia coli. Nucleic Acids Res 39, 9275 (Nov, 2011)). Furthermore, CRISPR is able to mediate genomic cleavage as efficiently as a pair of TALE nucleases (TALEN) targeting the same *EMX1* protospacer (FIG. 3, C and D).

Targeted modification of genomes ideally avoids mutations arising from the error-prone NHEJ mechanism. The wild-type SpCas9 is able to mediate site-specific DSBs, which can be repaired through either NHEJ or homology-directed repair (HDR). Applicants engineered an aspartate-to-alanine substitution (D10A) in the RuvC I domain of SpCas9 to convert the nuclease into a DNA nickase (SpCas9n, FIG. 4A) (M. Jinek et al., A programmable dual-RNA-guided DNA endonuclease in adaptive bacterial immunity. Science 337, 816 (Aug 17, 2012); G. Gasiunas, R. Barrangou, P. Horvath, V. Siksnys, Cas9-crRNA ribonucleoprotein complex mediates specific DNA cleavage for adaptive immunity in bacteria. Proc Natl Acad Sci U S A 109, E2579 (Sep 25, 2012) and R. Sapranauskas et al., The Streptococcus thermophilus CRISPR/Cas system provides immunity in Escherichia coli. Nucleic Acids Res 39, 9275 (Nov, 2011)), because nicked genomic DNA is typically repaired either seamlessly or through high-fidelity HDR. SURVEYOR (FIG. 4B) and sequencing of 327 amplicons did not detect any indels induced by SpCas9n. However, it is worth noting that nicked DNA can in rare cases be processed via a DSB intermediate and result in a NHEJ event (M. T. Certo et al., Tracking genome engineering outcome at individual DNA breakpoints. Nat Methods 8, 671 (Aug, 2011)). Applicants then tested Cas9-mediated HDR at the same *EMX1* locus with a homology repair template to introduce a pair of restriction sites near the protospacer (FIG. 4C). SpCas9 and SpCas9n catalyzed integration of the repair template into *EMX1* locus at similar levels (FIG. 4D), which Applicants further verified via Sanger sequencing (FIG. 4E). These results demonstrate the utility of CRISPR for facilitating targeted genomic insertions. Given the 14-bp (12-bp from the seed sequence and 2-bp from PAM) target specificity (FIG. 3B) of the wild type SpCas9, the use of a nickase may reduce off-target mutations.

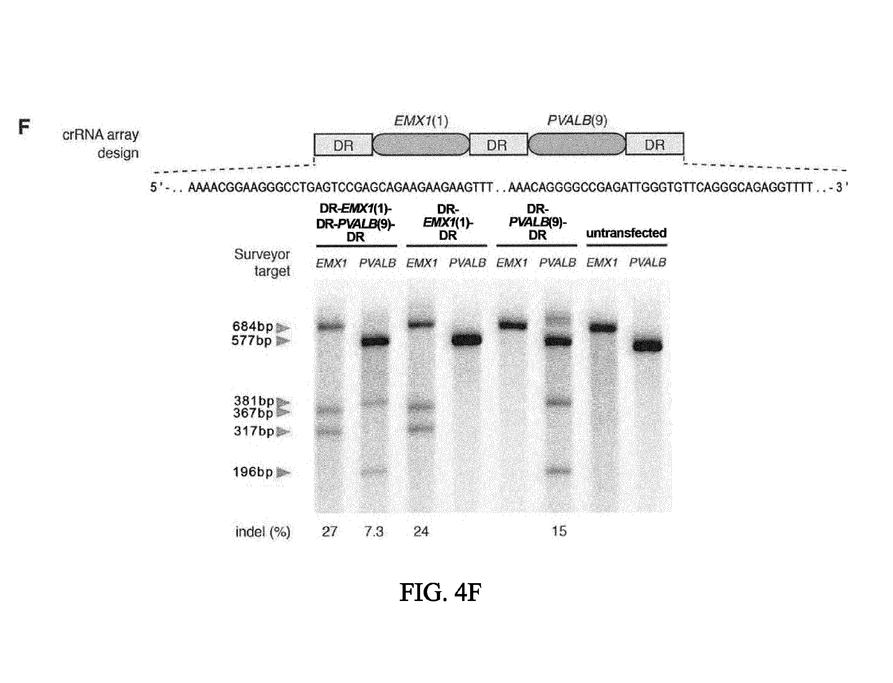

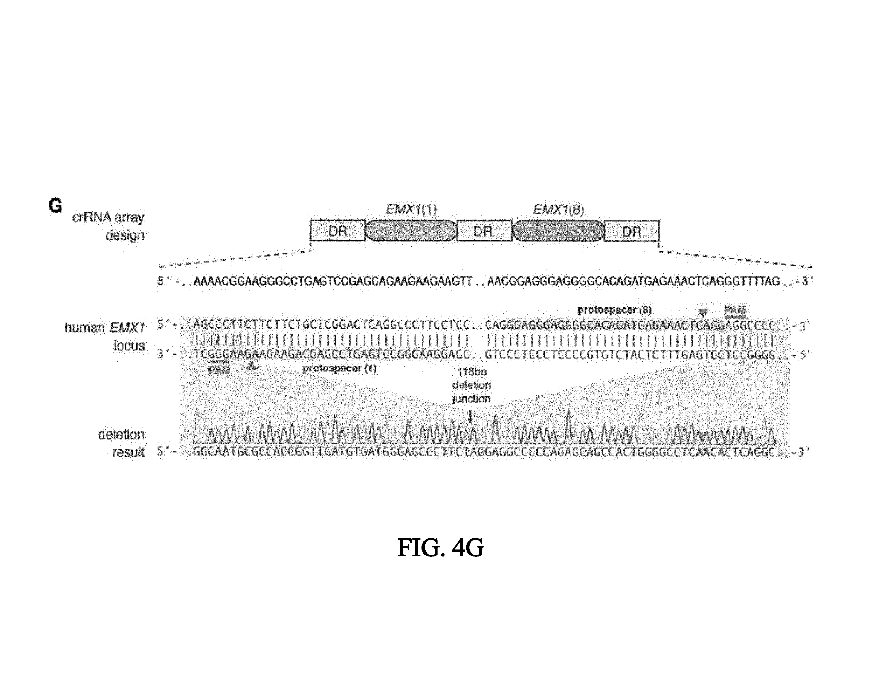

Finally, the natural architecture of CRISPR loci with arrayed spacers (FIG. 5) suggests the possibility of multiplexed genome engineering. Using a single CRISPR array encoding a pair of *EMX1-* and *PVALB*-targeting spacers, Applicants detected efficient cleavage at both loci (FIG. 4F). Applicants further tested targeted deletion of larger genomic regions through concurrent DSBs using spacers against two targets within *EMX1* spaced by 119-bp, and observed a 1.6% deletion efficacy (3 out of 182 amplicons; FIG. 4G), thus demonstrating the CRISPR system can mediate multiplexed editing within a single genome.

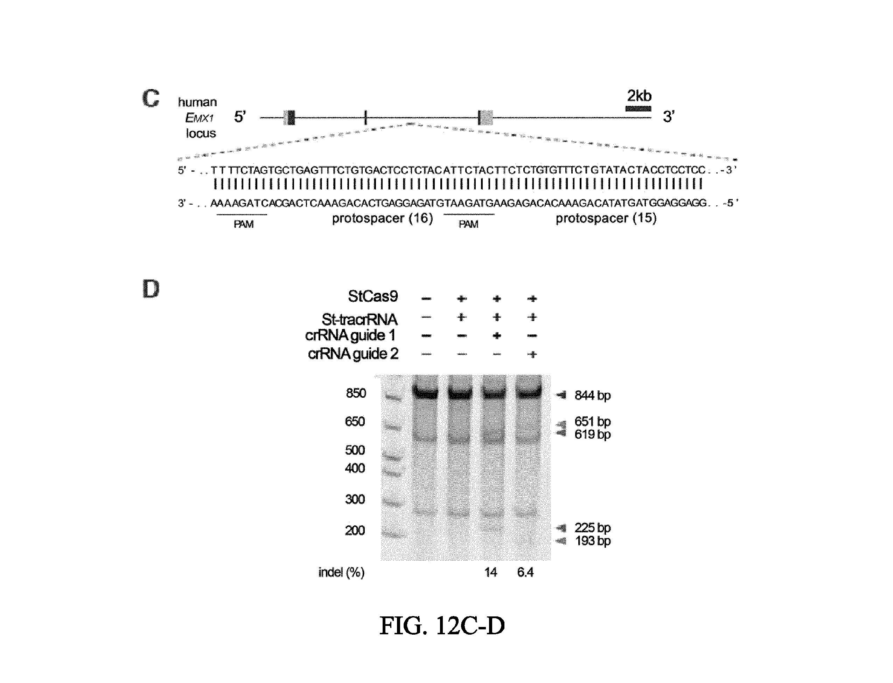

The ability to use RNA to program sequence-specific DNA cleavage defines a new class of genome engineering tools. Here, Applicants have shown that the *S. pyogenes* CRISPR system can be heterologously reconstituted in mammalian cells to facilitate efficient genome editing; an accompanying study has independently confirmed high efficiency CRISPR-mediated genome targeting in several human cell lines (Mali et al.). However, several aspects of the CRISPR system can be further improved to increase its efficiency and versatility. The requirement for an NGG PAM restricts the *S. pyogenes* CRISPR target space to every 8-bp on average in the human genome (FIG. 11), not accounting for potential constraints posed by crRNA secondary structure or genomic accessibility due to chromatin and DNA methylation states. Some of these restrictions may be overcome by exploiting the family of Cas9 enzymes and its differing PAM requirements (H. Deveau et al., Phage response to CRISPR-encoded resistance in Streptococcus thermophilus. J Bacteriol 190, 1390 (Feb, 2008) and F. J. Mojica, C. Diez-Villasenor, J. Garcia-Martinez, C. Almendros, Short motif sequences determine the targets of the prokaryotic CRISPR defence system. Microbiology 155, 733 (Mar, 2009)) across the microbial diversity (K. S. Makarova et al., Evolution and classification of the CRISPR-Cas systems. Nat Rev Microbiol 9, 467 (Jun, 2011)). Indeed, other CRISPR loci are likely to be transplantable into mammalian cells; for example, the *Streptococcus thermophilus* LMD-9 CRISPR1 can also mediate mammalian genome cleavage (FIG. 12). Finally, the ability to carry out multiplex genome editing in mammalian cells enables powerful applications across basic science, biotechnology, and medicine (P. A. Carr, G. M. Church, Genome engineering. Nat Biotechnol 27, 1151 (Dec, 2009)).

### Example 2

### Multiplex Genome Engineering Using CRISPR/Cas Systems: Supplementary Material

*Cell culture and transfection.* Human embryonic kidney (HEK) cell line 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and 100µg/mL streptomycin at 37°C with 5% CO2 incubation. Mouse neuro2A (N2A) cell line (ATCC) was maintained with DMEM supplemented with 5% fetal bovine serum (HyClone), 2mM GlutaMAX (Life Technologies), 100U/mL penicillin, and 100µg/mL streptomycin at 37°C with 5% CO₂.

293FT or N2A cells were seeded into 24-well plates (Corning) one day prior to transfection at a density of 200,000 cells per well. Cells were transfected using Lipofectamine 2000 (Life Technologies) following the manufacturer's recommended protocol. For each well of a 24-well plate a total of 800ng plasmids was used.

*Suveryor assay and sequencing analysis for genome modification.* 293FT or N2A cells were transfected with plasmid DNA as described above. Cells were incubated at 37°C for 72 hours post transfection before genomic DNA extraction. Genomic DNA was extracted using the QuickExtract DNA extraction kit (Epicentre) following the manufacturer's protocol. Briefly, cells were resuspended in QuickExtract solution and incubated at 65°C for 15 minutes and 98°C for 10 minutes.

Genomic region surrounding the CRISPR target site for each gene was PCR amplified, and products were purified using QiaQuick Spin Column (Qiagen) following manufacturer's protocol. A total of 400ng of the purified PCR products were mixed with 2µl 10X Taq polymerase PCR buffer (Enzymatics) and ultrapure water to a final volume of 20µl, and subjected to a re-annealing process to enable heteroduplex formation: 95°C for 10min, 95C to 85°C ramping at - 2°C/s, 85°C to 25°C at - 0.25°C/s, and 25°C hold for 1 minute. After reannealing, products were treated with SURVEYOR nuclease and SURVEYOR enhancer S (Transgenomics) following the manufacturer's recommended protocol, and analyzed on 4-20 Novex TBE poly-acrylamide gels (Life Technologies). Gels were stained with SYBR Gold DNA stain (Life Technologies) for 30 minutes and imaged with a Gel Doc gel imaging system (Biorad). Quantification was based on relative band intensities.

*Restriction fragment length polymorphism assay for detection of homologous recombination.* HEK 293FT and N2A cells were transfected with plasmid DNA, and incubated at 37°C for 72 hours before genomic DNA extraction as described above. The target genomic region was PCR amplified using primers outside the homology arms of the homologous recombination (HR) template. PCR products were separated on a 1% agarose gel and extracted with MinElute GelExtraction Kit (Qiagen). Purified products were digested with HindIII (Fermentas) and analyzed on a 6% Novex TBE poly-acrylamide gel (Life Technologies).

*RNA extraction and purification.* HEK 293FT cells were maintained and transfected as stated previously. Cells were harvested by trypsinization followed by washing in phosphate buffered saline (PBS). Total cell RNA was extracted with TRI reagent (Sigma) following manufacturer's protocol. Extracted total RNA was quantified using Naonodrop (Thermo Scientific) and normalized to same concentration.

*Northern blot analysis of crRNA and tracrRNA expression in mammalian cells.* RNAs were mixed with equal volumes of 2X loading buffer (Ambion), heated to 95°C for 5 min, chilled on ice for 1 min and then loaded onto 8% denaturing polyacrylamide gels (SequaGel, National Diagnostics) after pre-running the gel for at least 30 minutes. The samples were electrophoresed for 1.5 hours at 40W limit. Afterwards, the RNA was transferred to Hybond N+ membrane (GE Healthcare) at 300 mA in a semi-dry transfer apparatus (Bio-rad) at room temperature for 1.5 hours. The RNA was crosslinked to the membrane using autocrosslink button on Stratagene UV Crosslinker the Stratalinker (Stratagene). The membrane was pre-hybridized in ULTRAhyb-Oligo Hybridization Buffer (Ambion) for 30 min with rotation at 42°C and then probes were added and hybridized overnight. Probes were ordered from IDT and labeled with [gamma-32P] ATP (Perkin Elmer) with T4 polynucleotide kinase (New England Biolabs). The membrane was washed once with pre-warmed (42°C) 2xSSC, 0.5% SDS for 1 min followed by two 30 minute washes at 42°C. The membrane was exposed to phosphor screen for one hour or overnight at room temperature and then scanned with phosphorimager (Typhoon).

Table 1. Protospacer sequences and modification efficiencies of mammalian genomic targets. Protospacer targets designed based on Streptococcus pyogenes type II CRISPR and Streptococcus thermophilus CRISPR1 loci with their requisite PAMs against three different genes in human and mouse genomes. Cells were transfected with Cas9 and either precrRNA/ tracrRNA or chimeric RNA. Cells were analyzed 72 hours after transfection. Percent indels are calculated based on SURVEYOR assay results from indicated cell lines, N = 3 for all protospacer targets, errors are S.E.M. N.D., not detectable using the SURVEYOR assay; N.T., not tested in this study.

| **Cas9** | **target species** | **gene** | **protospacer ID** | **protospacer sequence (5' to 3')** | **PAM** | **strand** | **cell line tested** | **% indel (pre-crRNA + tracrRNA)** | **% indel (chimeric RNA)** |
|---|---|---|---|---|---|---|---|---|---|
| *S*. *pyogenes SF370 type II CRISPR* | *Homo sapiens* | *EMX1* | 1 | GGAAGGGCCTGAGATCCGAGCAGAAGAAGAA | GGG | + | 293FT | 20 ± 1.8 | 6.7 ± 0.62 |
| | | *EMX1* | 2 | CATTGGAGGTGACATCGATGTCCTCCCCAT | TGG | - | 293FT | 2.1 ± 0.31 | N.D. |
| | | *EMX1* | 3 | GGACATCGATGTCACCTCCAATGACTAGGG | TGG | + | 293FT | 14 ± 1.1 | N.D. |
| | | *EMX1* | 4 | CATCGATGTCCTCCCCATTGGCCTGCTTCG | TGG | - | 293FT | 11 ± 1.7 | N.D. |
| | | *EMX1* | 5 | TTCGTGGCAATGCGCCACCGGTTGATGTGA | TGG | - | 293FT | 4.3 ± 0.46 | 2.1 ± 0.51 |
| | | *EMX1* | 6 | TCGTGGCAATGCGCCACCGGTTGATGTGAT | GGG | - | 293FT | 4.0 ± 0.66 | 0.41 ± 0.25 |
| | | *EMX1* | 7 | TCCAGCTTCTGCCGTTTGTACTTTGTCCTC | CGG | - | 293FT | 1.5 ± 0.12 | N.D. |
| | | *EMX1* | 8 | GGAGGGAGGGGCACAGATGAGAAACTCAGG | AGG | - | 293FT | 7.8 ± 0.83 | 2.3 ± 1.2 |
| | *Homo sapiens* | *PVALB* | 9 | AGGGGCCGAGATTGGGTGTTCAGGGCAGAG | AGG | + | 293FT | 21 ± 2.6 | 6.5 ± 0.32 |
| | | *PVALB* | 10 | ATGCAGGAGGGTGGCGAGAGGGGCCGAGAT | TGG | + | 293FT | N.D. | N.D. |
| | | *PVALB* | 11 | GGTGGCGAGAGGGGCCGAGATTGGGTGTTC | AGG | + | 293FT | N.D. | N.D. |
| | *Mus musculus* | *Th* | 12 | CAAGCACTGAGTGCCATTAGCTAAATGCAT | AGG | - | Neuro2A | 27 ± 4.3 | 4.1 = 2.2 |
| | | *Th* | 13 | AATGCATAGGGTACCACCCACAGGTGCCAG | GGG | - | Neuro2A | 4.8 ± 1.2 | N.D. |
| | | *Th* | 14 | ACACACATGGGAAAGCCTCTGGGCCAGGAA | AGG | + | Neuro2A | 11.3 ± 1.3 | N.D. |
| *S. thermophilus LMD-9 CRISPR1* | *Homo sapiens* | *EMX1* | 15 | GGAGGAGGTAGTATACAGAACACAGAGAA | TAGAAT | - | 293FT | 14 ± 0.88 | N.T. |
| | | *EMX1* | 16 | AGAATGTAGAGGAGTCACAGAACTCAGCA | CTAGAAA | - | 293FT | 7.8 ± 0.77 | N.T. |

**Table 2. Sequences for primers and probes used for SURVEYOR assay, RFLP assay, genomic sequencing, and Northern blot.**

| **Primer name** | **Assay** | **Genomic Target** | **Primer sequence** |
|---|---|---|---|
| Sp-EMX1-F | SURVEYOR assay, sequencing | *EMX1* | AAAACCACCCTTCTCTCTGGC |
| Sp-EMX1-R | SURVEYOR assay, sequencing | *EMX1* | GGAGATTGGAGACACGGAGAG |
| Sp-PVALB-F | SURVEYOR assay, sequencing | *PVALB* | CTGGAAAGCCAATGCCTGAC |
| Sp-PVALB-R | SURVEYOR assay, sequencing | *PVALB* | GGCAGCAAACTCCTTGTCCT |
| Sp-Th-F | SURVEYOR assay, sequencing | *Th* | GTGCTTTGCAGAGGCCTACC |
| Sp-Th-R | SURVEYOR assay, sequencing | *Th* | CCTGGAGCGCATGCAGTAGT |
| St-EMX1-F | SURVEYOR assay, sequencing | *EMX1* | ACCTTCTGTGTTTCCACCATTC |
| St-EMX1-R | SURVEYOR assay, sequencing | *EMX1* | TTGGGGAGTGCACAGACTTC |
| Sp-EMX1-RFLP-F | RFLP, sequencing | *EMX1* | GGCTCCCTGGGTTCAAAGTA |
| Sp-EMX1-RFLP-R | RFLP, sequencing | *EMX1* | AGAGGGGTCTGGATGTCGTAA |
| Pb_EMX1_sp1 | Northern Blot Probe | Not applicable | TAGCTCTAAAACTTCTTCTTCTGCTCGGAC |
| Pb_tracrRNA | Northern Blot Probe | Not applicable | CTAGCCTTATTTTAACTTGCTATGCTGTTT |

### Supplementary Sequences

> U6-short tracrRNA (Streptococcus pyogenes SF370) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGAACCATTCAAAACAGCATAGCAAGTTAA
AATAAGGCTAGTCCGTTATCAACTTGAAAAAGTGGCACCGAGTCGGTGCTTTTTTT
> U6-long tracrRNA (Streptococcus pyogenes SF370) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGTAGTATTAAGTATTGTTTTATGGCTGATA
AATTTCTTTGAATTTCTCCTTGATTATTTGTTATAAAAGTTATAAAATAATCTTGTTG
GAACCATTCAAAACAGCATAGCAAGTTAAAATAAGGCTAGTCCGTTATCAACTTGA
AAAAGTGGCACCGAGTCGGTGCTTTTTTT
> U6-DR-BbsI backbone-DR (Streptococcus pyogenes SF370) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGGTTTTAGAGCTATGCTGTTTTGAATGGTC
CCAAAACGGGTCTTCGAGAAGACGTTTTAGAGCTATGCTGTTTTGAATGGTCCCAAA
AC
> U6-chimeric RNA-BbsI backbone (Streptococcus pyogenes SF370) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGGTCTTCGAGAAGACCTGTTTTAGAGCTA
GAAATAGCAAGTTAAAATAAGGCTAGTCCG
> 3xFLAG-NLS-SpCas9-NLS 
ATGGACTATAAGGACCACGACGGAGACTACAAGGATCATGATATTGATTA
CAAAGACGATGACGATAAGATGGCCCCAAAGAAGAAGCGGAAGGTCGGTATCCAC
GGAGTCCCAGCAGCCGACAAGAAGTACAGCATCGGCCTGGACATCGGCACCAACTC
TGTGGGCTGGGCCGTGATCACCGACGAGTACAAGGTGCCCAGCAAGAAATTCAAGG
TGCTGGGCAACACCGACCGGCACAGCATCAAGAAGAACCTGATCGGAGCCCTGCTG
TTCGACAGCGGCGAAACAGCCGAGGCCACCCGGCTGAAGAGAACCGCCAGAAGAA
GATACACCAGACGGAAGAACCGGATCTGCTATCTGCAAGAGATCTTCAGCAACGAG
ATGGCCAAGGTGGACGACAGCTTCTTCCACAGACTGGAAGAGTCCTTCCTGGTGGA
AGAGGATAAGAAGCACGAGCGGCACCCCATCTTCGGCAACATCGTGGACGAGGTGG
CCTACCACGAGAAGTACCCCACCATCTACCACCTGAGAAAGAAACTGGTGGACAGC
ACCGACAAGGCCGACCTGCGGCTGATCTATCTGGCCCTGGCCCACATGATCAAGTTC
CGGGGCCACTTCCTGATCGAGGGCGACCTGAACCCCGACAACAGCGACGTGGACAA
GCTGTTCATCCAGCTGGTGCAGACCTACAACCAGCTGTTCGAGGAAAACCCCATCAA
CGCCAGCGGCGTGGACGCCAAGGCCATCCTGTCTGCCAGACTGAGCAAGAGCAGAC
GGCTGGAAAATCTGATCGCCCAGCTGCCCGGCGAGAAGAAGAATGGCCTGTTCGGC
AACCTGATTGCCCTGAGCCTGGGCCTGACCCCCAACTTCAAGAGCAACTTCGACCTG
GCCGAGGATGCCAAACTGCAGCTGAGCAAGGACACCTACGACGACGACCTGGACAA
CCTGCTGGCCCAGATCGGCGACCAGTACGCCGACCTGTTTCTGGCCGCCAAGAACCT
GTCCGACGCCATCCTGCTGAGCGACATCCTGAGAGTGAACACCGAGATCACCAAGG
CCCCCCTGAGCGCCTCTATGATCAAGAGATACGACGAGCACCACCAGGACCTGACC
CTGCTGAAAGCTCTCGTGCGGCAGCAGCTGCCTGAGAAGTACAAAGAGATTTTCTTC
GACCAGAGCAAGAACGGCTACGCCGGCTACATTGACGGCGGAGCCAGCCAGGAAG
AGTTCTACAAGTTCATCAAGCCCATCCTGGAAAAGATGGACGGCACCGAGGAACTG
CTCGTGAAGCTGAACAGAGAGGACCTGCTGCGGAAGCAGCGGACCTTCGACAACGG
CAGCATCCCCCACCAGATCCACCTGGGAGAGCTGCACGCCATTCTGCGGCGGCAGG
AAGATTTTTACCCATTCCTGAAGGACAACCGGGAAAAGATCGAGAAGATCCTGACC
TTCCGCATCCCCTACTACGTGGGCCCTCTGGCCAGGGGAAACAGCAGATTCGCCTGG
ATGACCAGAAAGAGCGAGGAAACCATCACCCCCTGGAACTTCGAGGAAGTGGTGGA
CAAGGGCGCTTCCGCCCAGAGCTTCATCGAGCGGATGACCAACTTCGATAAGAACC
TGCCCAACGAGAAGGTGCTGCCCAAGCACAGCCTGCTGTACGAGTACTTCACCGTGT
ATAACGAGCTGACCAAAGTGAAATACGTGACCGAGGGAATGAGAAAGCCCGCCTTC
 
CTGAGCGGCGAGCAGAAAAAGGCCATCGTGGACCTGCTGTTCAAGACCAACCGGAA
AGTGACCGTGAAGCAGCTGAAAGAGGACTACTTCAAGAAAATCGAGTGCTTCGACT
CCGTGGAAATCTCCGGCGTGGAAGATCGGTTCAACGCCTCCCTGGGCACATACCAC
GATCTGCTGAAAATTATCAAGGACAAGGACTTCCTGGACAATGAGGAAAACGAGGA
CATTCTGGAAGATATCGTGCTGACCCTGACACTGTTTGAGGACAGAGAGATGATCGA
GGAACGGCTGAAAACCTATGCCCACCTGTTCGACGACAAAGTGATGAAGCAGCTGA
AGCGGCGGAGATACACCGGCTGGGGCAGGCTGAGCCGGAAGCTGATCAACGGCATC
CGGGACAAGCAGTCCGGCAAGACAATCCTGGATTTCCTGAAGTCCGACGGCTTCGC
CAACAGAAACTTCATGCAGCTGATCCACGACGACAGCCTGACCTTTAAAGAGGACA
TCCAGAAAGCCCAGGTGTCCGGCCAGGGCGATAGCCTGCACGAGCACATTGCCAAT
CTGGCCGGCAGCCCCGCCATTAAGAAGGGCATCCTGCAGACAGTGAAGGTGGTGGA
CGAGCTCGTGAAAGTGATGGGCCGGCACAAGCCCGAGAACATCGTGATCGAAATGG
CCAGAGAGAACCAGACCACCCAGAAGGGACAGAAGAACAGCCGCGAGAGAATGAA
GCGGATCGAAGAGGGCATCAAAGAGCTGGGCAGCCAGATCCTGAAAGAACACCCC
GTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGATG
TGGACCATATCGTGCCTCAGAGCTTTCTGAAGGACGACTCCATCGACAACAAGGTGC
TGACCAGAAGCGACAAGAACCGGGGCAAGAGCGACAACGTGCCCTCCGAAGAGGT
CGTGAAGAAGATGAAGAACTACTGGCGGCAGCTGCTGAACGCCAAGCTGATTACCC
AGAGAAAGTTCGACAATCTGACCAAGGCCGAGAGAGGCGGCCTGAGCGAACTGGAT
AAGGCCGGCTTCATCAAGAGACAGCTGGTGGAAACCCGGCAGATCACAAAGCACGT
GGCACAGATCCTGGACTCCCGGATGAACACTAAGTACGACGAGAATGACAAGCTGA
TCCGGGAAGTGAAAGTGATCACCCTGAAGTCCAAGCTGGTGTCCGATTTCCGGAAG
GATTTCCAGTTTTACAAAGTGCGCGAGATCAACAACTACCACCACGCCCACGACGCC
TACCTGAACGCCGTCGTGGGAACCGCCCTGATCAAAAAGTACCCTAAGCTGGAAAG
CGAGTTCGTGTACGGCGACTACAAGGTGTACGACGTGCGGAAGATGATCGCCAAGA
GCGAGCAGGAAATCGGCAAGGCTACCGCCAAGTACTTCTTCTACAGCAACATCATG
AACTTTTTCAAGACCGAGATTACCCTGGCCAACGGCGAGATCCGGAAGCGGCCTCT
GATCGAGACAAACGGCGAAACCGGGGAGATCGTGTGGGATAAGGGCCGGGATTTTG
CCACCGTGCGGAAAGTGCTGAGCATGCCCCAAGTGAATATCGTGAAAAAGACCGAG
GTGCAGACAGGCGGCTTCAGCAAAGAGTCTATCCTGCCCAAGAGGAACAGCGATAA
 
GCTGATCGCCAGAAAGAAGGACTGGGACCCTAAGAAGTACGGCGGCTTCGACAGCC
CCACCGTGGCCTATTCTGTGCTGGTGGTGGCCAAAGTGGAAAAGGGCAAGTCCAAG
AAACTGAAGAGTGTGAAAGAGCTGCTGGGGATCACCATCATGGAAAGAAGCAGCTT
CGAGAAGAATCCCATCGACTTTCTGGAAGCCAAGGGCTACAAAGAAGTGAAAAAGG
ACCTGATCATCAAGCTGCCTAAGTACTCCCTGTTCGAGCTGGAAAACGGCCGGAAG
AGAATGCTGGCCTCTGCCGGCGAACTGCAGAAGGGAAACGAACTGGCCCTGCCCTC
CAAATATGTGAACTTCCTGTACCTGGCCAGCCACTATGAGAAGCTGAAGGGCTCCCC
CGAGGATAATGAGCAGAAACAGCTGTTTGTGGAACAGCACAAGCACTACCTGGACG
AGATCATCGAGCAGATCAGCGAGTTCTCCAAGAGAGTGATCCTGGCCGACGCTAAT
CTGGACAAAGTGCTGTCCGCCTACAACAAGCACCGGGATAAGCCCATCAGAGAGCA
GGCCGAGAATATCATCCACCTGTTTACCCTGACCAATCTGGGAGCCCCTGCCGCCTT
CAAGTACTTTGACACCACCATCGACCGGAAGAGGTACACCAGCACCAAAGAGGTGC
TGGACGCCACCCTGATCCACCAGAGCATCACCGGCCTGTACGAGACACGGATCGAC
CTGTCTCAGCTGGGAGGCGACAAGCGTCCTGCTGCTACTAAGAAAGCTGGTCAAGCT
AAGAAAAAGAAA
[> SpRNase3-mCherry-NLS 
ATGAAGCAGCTGGAGGAGTTACTTTCTACCTCTTTCGACATCCAGTTTAAT
GACCTGACCCTGCTGGAAACCGCCTTCACTCACACCTCCTACGCGAATGAGCACCGC
CTACTGAATGTGAGCCACAACGAGCGCCTGGAGTTTCTGGGGGATGCTGTCTTACAG
CTGATCATCTCTGAATATCTGTTTGCCAAATACCCTAAGAAAACCGAAGGGGACATG
TCAAAGCTGCGCTCCATGATAGTCAGGGAAGAGAGCCTGGCGGGCTTTAGTCGTTTT
TGCTCATTCGACGCTTATATCAAGCTGGGAAAAGGCGAAGAGAAGTCCGGCGGCAG
GAGGCGCGATACAATTCTGGGCGATCTCTTTGAAGCGTTTCTGGGCGCACTTCTACT
GGACAAAGGGATCGACGCAGTCCGCCGCTTTCTGAAACAAGTGATGATCCCTCAGG
TCGAAAAGGGAAACTTCGAGAGAGTGAAGGACTATAAAACATGTTTGCAGGAATTT
CTCCAGACCAAGGGAGATGTAGCAATAGATTATCAGGTAATAAGTGAGAAAGGACC
AGCTCACGCCAAACAATTCGAAGTTAGCATCGTTGTTAATGGCGCAGTGTTGTCGAA
GGGCTTGGGTAAATCAAAAAAACTGGCCGAGCAGGACGCTGCTAAAAACGCCCTCG
CTCAGCTCAGCGAGGTAGGATCCGTGAGCAAGGGCGAGGAGGATAACATGGCCATC
ATCAAGGAGTTCATGCGCTTCAAGGTGCACATGGAGGGCTCCGTGAACGGCCACGA
GTTCGAGATCGAGGGCGAGGGCGAGGGCCGCCCCTACGAGGGCACCCAGACCGCCA
 
AGCTGAAGGTGACCAAGGGTGGCCCCCTGCCCTTCGCCTGGGACATCCTGTCCCCTC
AGTTCATGTACGGCTCCAAGGCCTACGTGAAGCACCCCGCCGACATCCCCGACTACT
TGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTA
CAAGGTGAAGCTGCGCGGCACCAACTTCCCCTCCGACGGCCCCGTAATGCAGAAGA
AGACCATGGGCTGGGAGGCCTCCTCCGAGCGGATGTACCCCGAGGACGGCGCCCTG
AAGGGCGAGATCAAGCAGAGGCTGAAGCTGAAGGACGGCGGCCACTACGACGCTG
AGGTCAAGACCACCTACAAGGCCAAGAAGCCCGTGCAGCTGCCCGGCGCCTACAAC
GTCAACATCAAGTTGGACATCACCTCCCACAACGAGGACTACACCATCGTGGAACA
GTACGAACGCGCCGAGGGCCGCCACTCCACCGGCGGCATGGACGAGCTGTACAAGA
AGCGTCCTGCTGCTACTAAGAAAGCTGGTCAAGCTAAGAAAAAGAAA
> 3xFLAG-NLS-SpCas9n-NLS (the D10A nickase mutation is underlined) 
ATGGACTATAAGGACCACGACGGAGACTACAAGGATCATGATATTGATTA
CAAAGACGATGACGATAAGATGGCCCCAAAGAAGAAGCGGAAGGTCGGTATCCAC
GGAGTCCCAGCAGCCGACAAGAAGTACAGCATCGGCCTGGCCATCGGCACCAACTC
TGTGGGCTGGGCCGTGATCACCGACGAGTACAAGGTGCCCAGCAAGAAATTCAAGG
TGCTGGGCAACACCGACCGGCACAGCATCAAGAAGAACCTGATCGGAGCCCTGCTG
TTCGACAGCGGCGAAACAGCCGAGGCCACCCGGCTGAAGAGAACCGCCAGAAGAA
GATACACCAGACGGAAGAACCGGATCTGCTATCTGCAAGAGATCTTCAGCAACGAG
ATGGCCAAGGTGGACGACAGCTTCTTCCACAGACTGGAAGAGTCCTTCCTGGTGGA
AGAGGATAAGAAGCACGAGCGGCACCCCATCTTCGGCAACATCGTGGACGAGGTGG
CCTACCACGAGAAGTACCCCACCATCTACCACCTGAGAAAGAAACTGGTGGACAGC
ACCGACAAGGCCGACCTGCGGCTGATCTATCTGGCCCTGGCCCACATGATCAAGTTC
CGGGGCCACTTCCTGATCGAGGGCGACCTGAACCCCGACAACAGCGACGTGGACAA
GCTGTTCATCCAGCTGGTGCAGACCTACAACCAGCTGTTCGAGGAAAACCCCATCAA
CGCCAGCGGCGTGGACGCCAAGGCCATCCTGTCTGCCAGACTGAGCAAGAGCAGAC
GGCTGGAAAATCTGATCGCCCAGCTGCCCGGCGAGAAGAAGAATGGCCTGTTCGGC
AACCTGATTGCCCTGAGCCTGGGCCTGACCCCCAACTTCAAGAGCAACTTCGACCTG
GCCGAGGATGCCAAACTGCAGCTGAGCAAGGACACCTACGACGACGACCTGGACAA
CCTGCTGGCCCAGATCGGCGACCAGTACGCCGACCTGTTTCTGGCCGCCAAGAACCT
GTCCGACGCCATCCTGCTGAGCGACATCCTGAGAGTGAACACCGAGATCACCAAGG
 
CCCCCCTGAGCGCCTCTATGATCAAGAGATACGACGAGCACCACCAGGACCTGACC
CTGCTGAAAGCTCTCGTGCGGCAGCAGCTGCCTGAGAAGTACAAAGAGATTTTCTTC
GACCAGAGCAAGAACGGCTACGCCGGCTACATTGACGGCGGAGCCAGCCAGGAAG
AGTTCTACAAGTTCATCAAGCCCATCCTGGAAAAGATGGACGGCACCGAGGAACTG
CTCGTGAAGCTGAACAGAGAGGACCTGCTGCGGAAGCAGCGGACCTTCGACAACGG
CAGCATCCCCCACCAGATCCACCTGGGAGAGCTGCACGCCATTCTGCGGCGGCAGG
AAGATTTTTACCCATTCCTGAAGGACAACCGGGAAAAGATCGAGAAGATCCTGACC
TTCCGCATCCCCTACTACGTGGGCCCTCTGGCCAGGGGAAACAGCAGATTCGCCTGG
ATGACCAGAAAGAGCGAGGAAACCATCACCCCCTGGAACTTCGAGGAAGTGGTGGA
CAAGGGCGCTTCCGCCCAGAGCTTCATCGAGCGGATGACCAACTTCGATAAGAACC
TGCCCAACGAGAAGGTGCTGCCCAAGCACAGCCTGCTGTACGAGTACTTCACCGTGT
ATAACGAGCTGACCAAAGTGAAATACGTGACCGAGGGAATGAGAAAGCCCGCCTTC
CTGAGCGGCGAGCAGAAAAAGGCCATCGTGGACCTGCTGTTCAAGACCAACCGGAA
AGTGACCGTGAAGCAGCTGAAAGAGGACTACTTCAAGAAAATCGAGTGCTTCGACT
CCGTGGAAATCTCCGGCGTGGAAGATCGGTTCAACGCCTCCCTGGGCACATACCAC
GATCTGCTGAAAATTATCAAGGACAAGGACTTCCTGGACAATGAGGAAAACGAGGA
CATTCTGGAAGATATCGTGCTGACCCTGACACTGTTTGAGGACAGAGAGATGATCGA
GGAACGGCTGAAAACCTATGCCCACCTGTTCGACGACAAAGTGATGAAGCAGCTGA
AGCGGCGGAGATACACCGGCTGGGGCAGGCTGAGCCGGAAGCTGATCAACGGCATC
CGGGACAAGCAGTCCGGCAAGACAATCCTGGATTTCCTGAAGTCCGACGGCTTCGC
CAACAGAAACTTCATGCAGCTGATCCACGACGACAGCCTGACCTTTAAAGAGGACA
TCCAGAAAGCCCAGGTGTCCGGCCAGGGCGATAGCCTGCACGAGCACATTGCCAAT
CTGGCCGGCAGCCCCGCCATTAAGAAGGGCATCCTGCAGACAGTGAAGGTGGTGGA
CGAGCTCGTGAAAGTGATGGGCCGGCACAAGCCCGAGAACATCGTGATCGAAATGG
CCAGAGAGAACCAGACCACCCAGAAGGGACAGAAGAACAGCCGCGAGAGAATGAA
GCGGATCGAAGAGGGCATCAAAGAGCTGGGCAGCCAGATCCTGAAAGAACACCCC
GTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGATG
TGGACCATATCGTGCCTCAGAGCTTTCTGAAGGACGACTCCATCGACAACAAGGTGC
TGACCAGAAGCGACAAGAACCGGGGCAAGAGCGACAACGTGCCCTCCGAAGAGGT
CGTGAAGAAGATGAAGAACTACTGGCGGCAGCTGCTGAACGCCAAGCTGATTACCC
 
AGAGAAAGTTCGACAATCTGACCAAGGCCGAGAGAGGCGGCCTGAGCGAACTGGAT
AAGGCCGGCTTCATCAAGAGACAGCTGGTGGAAACCCGGCAGATCACAAAGCACGT
GGCACAGATCCTGGACTCCCGGATGAACACTAAGTACGACGAGAATGACAAGCTGA
TCCGGGAAGTGAAAGTGATCACCCTGAAGTCCAAGCTGGTGTCCGATTTCCGGAAG
GATTTCCAGTTTTACAAAGTGCGCGAGATCAACAACTACCACCACGCCCACGACGCC
TACCTGAACGCCGTCGTGGGAACCGCCCTGATCAAAAAGTACCCTAAGCTGGAAAG
CGAGTTCGTGTACGGCGACTACAAGGTGTACGACGTGCGGAAGATGATCGCCAAGA
GCGAGCAGGAAATCGGCAAGGCTACCGCCAAGTACTTCTTCTACAGCAACATCATG
AACTTTTTCAAGACCGAGATTACCCTGGCCAACGGCGAGATCCGGAAGCGGCCTCT
GATCGAGACAAACGGCGAAACCGGGGAGATCGTGTGGGATAAGGGCCGGGATTTTG
CCACCGTGCGGAAAGTGCTGAGCATGCCCCAAGTGAATATCGTGAAAAAGACCGAG
GTGCAGACAGGCGGCTTCAGCAAAGAGTCTATCCTGCCCAAGAGGAACAGCGATAA
GCTGATCGCCAGAAAGAAGGACTGGGACCCTAAGAAGTACGGCGGCTTCGACAGCC
CCACCGTGGCCTATTCTGTGCTGGTGGTGGCCAAAGTGGAAAAGGGCAAGTCCAAG
AAACTGAAGAGTGTGAAAGAGCTGCTGGGGATCACCATCATGGAAAGAAGCAGCTT
CGAGAAGAATCCCATCGACTTTCTGGAAGCCAAGGGCTACAAAGAAGTGAAAAAGG
ACCTGATCATCAAGCTGCCTAAGTACTCCCTGTTCGAGCTGGAAAACGGCCGGAAG
AGAATGCTGGCCTCTGCCGGCGAACTGCAGAAGGGAAACGAACTGGCCCTGCCCTC
CAAATATGTGAACTTCCTGTACCTGGCCAGCCACTATGAGAAGCTGAAGGGCTCCCC
CGAGGATAATGAGCAGAAACAGCTGTTTGTGGAACAGCACAAGCACTACCTGGACG
AGATCATCGAGCAGATCAGCGAGTTCTCCAAGAGAGTGATCCTGGCCGACGCTAAT
CTGGACAAAGTGCTGTCCGCCTACAACAAGCACCGGGATAAGCCCATCAGAGAGCA
GGCCGAGAATATCATCCACCTGTTTACCCTGACCAATCTGGGAGCCCCTGCCGCCTT
CAAGTACTTTGACACCACCATCGACCGGAAGAGGTACACCAGCACCAAAGAGGTGC
TGGACGCCACCCTGATCCACCAGAGCATCACCGGCCTGTACGAGACACGGATCGAC
CTGTCTCAGCTGGGAGGCGACAAGCGTCCTGCTGCTACTAAGAAAGCTGGTCAAGCT
AAGAAAAAGAAA
> hEMX1-HRTemplate-HindIII-NheI 
GAATGCTGCCCTCAGACCCGCTTCCTCCCTGTCCTTGTCTGTCCAAGGAGA
ATGAGGTCTCACTGGTGGATTTCGGACTACCCTGAGGAGCTGGCACCTGAGGGACA
AGGCCCCCCACCTGCCCAGCTCCAGCCTCTGATGAGGGGTGGAGAGAGCTACATG
 
AGGTTGCTAAGAAAGCCTCCCCTGAAGGAGACCACACAGTGTGTGAGGTTGGAGTC
TCTAGCAGCGGGTTCTGTGCCCCCAGGGATAGTCTGGCTGTCCAGGCACTGCTCTTG
ATATAAACACCACCTCCTAGTTATGAAACCATGCCCATTCTGCCTCTCTGTATGGAA
AAGAGCATGGGGCTGGCCCGTGGGGTGGTGTCCACTTTAGGCCCTGTGGGAGATCA
TGGGAACCCACGCAGTGGGTCATAGGCTCTCTCATTTACTACTCACATCCACTCTGT
GAAGAAGCGATTATGATCTCTCCTCTAGAAACTCGTAGAGTCCCATGTCTGCCGGCT
TCCAGAGCCTGCACTCCTCCACCTTGGCTTGGCTTTGCTGGGGCTAGAGGAGCTAGG
ATGCACAGCAGCTCTGTGACCCTTTGTTTGAGAGGAACAGGAAAACCACCCTTCTCT
CTGGCCCACTGTGTCCTCTTCCTGCCCTGCCATCCCCTTCTGTGAATGTTAGACCCAT
GGGAGCAGCTGGTCAGAGGGGACCCCGGCCTGGGGCCCCTAACCCTATGTAGCCTC
AGTCTTCCCATCAGGCTCTCAGCTCAGCCTGAGTGTTGAGGCCCCAGTGGCTGCTCT
GGGGGCCTCCTGAGTTTCTCATCTGTGCCCCTCCCTCCCTGGCCCAGGTGAAGGTGT
GGTTCCAGAACCGGAGGACAAAGTACAAACGGCAGAAGCTGGAGGAGGAAGGGCC
TGAGTCCGAGCAGAAGAAGAAGGGCTCCCATCACATCAACCGGTGGCGCATTGCCA
CGAAGCAGGCCAATGGGGAGGACATCGATGTCACCTCCAATGACaagcttgctagcGGTGG
GCAACCACAAACCCACGAGGGCAGAGTGCTGCTTGCTGCTGGCCAGGCCCCTGCGT
GGGCCCAAGCTGGACTCTGGCCACTCCCTGGCCAGGCTTTGGGGAGGCCTGGAGTC
ATGGCCCCACAGGGCTTGAAGCCCGGGGCCGCCATTGACAGAGGGACAAGCAATGG
GCTGGCTGAGGCCTGGGACCACTTGGCCTTCTCCTCGGAGAGCCTGCCTGCCTGGGC
GGGCCCGCCCGCCACCGCAGCCTCCCAGCTGCTCTCCGTGTCTCCAATCTCCCTTTTG
TTTTGATGCATTTCTGTTTTAATTTATTTTCCAGGCACCACTGTAGTTTAGTGATCCCC
AGTGTCCCCCTTCCCTATGGGAATAATAAAAGTCTCTCTCTTAATGACACGGGCATC
CAGCTCCAGCCCCAGAGCCTGGGGTGGTAGATTCCGGCTCTGAGGGCCAGTGGGGG
CTGGTAGAGCAAACGCGTTCAGGGCCTGGGAGCCTGGGGTGGGGTACTGGTGGAGG
GGGTCAAGGGTAATTCATTAACTCCTCTCTTTTGTTGGGGGACCCTGGTCTCTACCTC
CAGCTCCACAGCAGGAGAAACAGGCTAGACATAGGGAAGGGCCATCCTGTATCTTG
AGGGAGGACAGGCCCAGGTCTTTCTTAACGTATTGAGAGGTGGGAATCAGGCCCAG
GTAGTTCAATGGGAGAGGGAGAGTGCTTCCCTCTGCCTAGAGACTCTGGTGGCTTCT
CCAGTTGAGGAGAAACCAGAGGAAAGGGGAGGATTGGGGTCTGGGGGAGGGAACA
CCATTCACAAAGGCTGACGGTTCCAGTCCGAAGTCGTGGGCCCACCAGGATGCTCA
CCTGTCCTTGGAGAACCGCTGGGCAGGTTGAGACTGCAGAGACAGGGCTTAAGGCT
 
GAGCCTGCAACCAGTCCCCAGTGACTCAGGGCCTCCTCAGCCCAAGAAAGAGCAAC
GTGCCAGGGCCCGCTGAGCTCTTGTGTTCACCTG
> NLS-StCsn1-NLS 
ATGAAAAGGCCGGCGGCCACGAAAAAGGCCGGCCAGGCAAAAAAGAAA
AAGTCCGACCTGGTACTTGGACTGGATATTGGTATCGGTTCGGTGGGAGTCGGAATC
CTCAACAAGGTCACGGGGGAGATCATTCACAAGAACTCGCGGATCTTCCCCGCAGC
TCAGGCTGAGAACAACTTGGTGCGGAGAACG
.
AATAGGCAGGGCAGGCGACTGGCGAGGAGGAAGAAACACAGGAGAGTC
CGATTGAACCGGCTGTTCGAGGAGTCCGGTTTGATCACCGACTTTACGAAAATCTCG
ATTAACCTTAATCCCTATCAGCTTCGGGTGAAAGGCCTGACAGACGAACTTTCGAAT
GAGGAACTTTTCATCGCGCTGAAAAACATGGTCAAGCACAGAGGGATTTCCTACCTC
GATGACGCCTCGGATGACGGAAATTCCTCAGTAGGAGATTATGCACAGATCGTGAA
AGAGAACTCAAAGCAACTGGAAACAAAGACACCGGGGCAGATCCAACTTGAAAGA
TACCAGACATACGGACAGCTCAGAGGAGATTTTACGGTGGAGAAGGACGGTAAAAA
GCACAGACTCATTAACGTATTTCCCACGTCGGCGTACAGATCCGAAGCGCTCCGCAT
CCTTCAGACTCAACAGGAGTTCAACCCGCAAATTACTGATGAGTTCATCAACCGCTA
TTTGGAAATCTTGACCGGAAAGCGCAAGTATTATCATGGGCCGGGTAATGAGAAAT
CCAGAACAGATTACGGCCGATACAGAACTTCGGGGGAAACCTTGGATAACATCTTT
GGTATTTTGATTGGAAAGTGCACCTTTTACCCGGACGAGTTCGAGCGGCCAAGGCG
TCATACACAGCACAAGAGTTTAATCTCTTGAATGATTTGAACAACTTGACGGTCCCC
ACGGAGACAAAGAAGCTCTCCAAAGAGCAAAAGAACCAAATCATCAACTACGTCA
AGAACGAGAAGGCTATGGGGCCAGCGAAGCTGTTCAAGTATATCGCTAAACTTCTC
AGCTGTGATGTGGCGGACATCAAAGGGTACCGAATCGACAAGTCGGGAAAAGCGGA
AATTCACACGTTTGAAGCATATCGAAAGATGAAAACGTTGGAAACACTGGACATTG
AGCAGATGGACCGGGAAACGCTCGACAAACTGGCATACGTGCTCACGTTGAATACT
GAACGAGAGGGAATCCAAGAGGCCCTTGAACATGAGTTCGCCGATGGATCGTTCAG
CCAGAAGCAGGTCGACGAACTTGTGCAATTCCGCAAGGCGAATAGCTCCATCTTCG
GGAAGGGATGGCACAACTTTTCGGTCAAACTCATGATGGAGTTGATCCCAGAACTTT
ATGAGACTTCGGAGGAGCAAATGACGATCTTGACGCGCTTGGGGAAACAGAAAACG
ACAAGCTCATCGAACAAAACTAAGTACATTGATGAGAAATTGCTGACGGAAGAAAT
CTATAATCCGGTAGTAGCGAAATCGGTAAGACAAGCGATCAAAATCGTGAACGCGG
 
CGATCAAGGAATATGGTGACTTTGATAACATCGTAATTGAAATGGCTAGAGAGACG
AACGAAGATGACGAGAAAAAGGCAATCCAGAAGATCCAGAAGGCCAACAAGGATG
AAAAAGATGCAGCGATGCTTAAAGCGGCCAACCAATACAATGGAAAGGCGGAGCT
GCCCCATTCAGTGTTTCACGGTCATAAACAGTTGGCGACCAAGATCCGACTCTGGCA
TCAGCAGGGTGAGCGGTGTCTCTACACCGGAAAGACTATCTCCATCCATGACTTGAT
TAACAATTCGAACCAGTTTGAAGTGGATCATATTCTGCCCCTGTCAATCACCTTTGA
CGACTCGCTTGCGAACAAGGTGCTCGTGTACGCAACGGCAAATCAGGAGAAAGGCC
AGCGGACTCCGTATCAGGCGCTCGACTCAATGGACGATGCGTGGTCATTCCGGGAG
CTGAAGGCGTTCGTACGCGAGAGCAAGACACTGAGCAACAAAAAGAAAGAGTATCT
GCTGACAGAGGAGGACATCTCGAAATTCGATGTCAGGAAGAAGTTCATCGAGCGGA
ATCTTGTCGACACTCGCTACGCTTCCAGAGTAGTACTGAACGCGCTCCAGGAACACT
TTAGAGCGCACAAAATTGACACGAAGGTGTCAGTGGTGAGAGGGCAGTTCACATCC
CAACTCCGCCGACATTGGGGCATCGAAAAGACGCGGACACATATCACCATCATGC
GGTGGACGCGCTGATTATTGCCGCTTCGTCCCAGTTGAATCTCTGGAAAAAGCAGAA
GAACACGCTGGTGTCGTATTCGGAGGATCAGCTTTTGGACATCGAAACCGGGGAGC
TGATTTCCGACGATGAATACAAAGAATCGGTGTTTAAGGCACCATATCAGCATTTCG
TGGACACGCTGAAGAGCAAAGAGTTTGAGGACAGCATCCTCTTTTCGTACCAAGTG
GACTCGAAGTTTAATCGCAAGATTTCAGACGCCACAATCTACGCGACGAGGCAGGC
GAAGGTGGGCAAAGATAAAGCAGATGAAACCTACGTCCTTGGTAAAATCAAGGACA
TCTACACTCAGGACGGGTACGATGCGTTCATGAAAATCTACAAGAAGGATAAGTCG
AAGTTTCTCATGTACCGCCACGATCCACAGACTTTCGAAAAAGTCATTGAGCCTATT
TTGGAGAACTACCCTAACAAGCAAATCAACGAGAAAGGAAAGAAGTCCCGTGCAA
CCCCTTTCTGAAGTACAAGGAAGAGCACGGTTATATCCGCAAATACTCGAAGAAAG
GAAATGGGCCTGAGATTAAGTCGCTTAAGTATTACGACTCAAAGTTGGGTAACCAC
ATCGACATTACCCCGAAAGACTCCAACAACAAAGTCGTGTTGCAGTCCGTCTCGCCC
TGGCGAGCAGATGTGTATTTTAATAAGACGACCGGCAAATATGAGATCCTTGGACTC
AAATACGCAGACCTTCAATTCGAAAAGGGGACGGGCACTTATAAGATTTCACAAGA
GAAGTACAACGACATCAAGAAAAAGGAAGGGGTCGATTCAGATTCGGAGTTCAAAT
TCACCCTCTACAAAAACGACCTCCTGCTTGTGAAGGACACAGAAACGAAGGAGCAG
CAGCTCTTTCGGTTCCTCTCACGCACGATGCCCAAACAAAAACATTACGTCGAACTT
AAACCTTACGATAAGCAAAAGTTTGAAGGGGGAGAGGCACTGATCAAAGTATTGGG
 
TAACGTAGCCAATAGCGGACAGTGTAAGAAAGGGCTGGGAAAGTCCAATATCTCGA
TCTATAAAGTACGAACAGATGTATTGGGAAACCAGCATATCATCAAAAATGAGGGG
GATAAACCCAAACTCGATTTCAAGCGTCCTGCTGCTACTAAGAAAGCTGGTCAAGCT
AAGAAAAAGAAATAA
> U6-St_tracrRNA(7-97) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGTTACTTAAATCTTGCAGAAGCTACAAAGA
TAAGGCTTCATGCCGAAATCAACACCCTGTCATTTTATGGCAGGGTGTTTTCGTTATT
TAA
> EMX1_TALEN_Left 
ATGGACTATAAGGACCACGACGGAGACTACAAGGATCATGATATTGATTA
CAAAGACGATGACGATAAGATGGCCCCAAAGAAGAAGCGGAAGGTCGGTATCCAC
GGAGTCCCAGCAGCCGTAGATTTGAGAACTTTGGGATATTCACAGCAGCAGCAGGA
AAAGATCAAGCCCAAAGTGAGGTCGACAGTCGCGCAGCATCACGAAGCGCTGGTGG
GTCATGGGTTTACACATGCCCACATCGTAGCCTTGTCGCAGCACCCTGCAGCCCTTG
GCACGGTCGCCGTCAAGTACCAGGACATGATTGCGGCGTTGCCGGAAGCCACACAT
GAGGCGATCGTCGGTGTGGGGAAACAGTGGAGCGGAGCCCGAGCGCTTGAGGCCCT
GTTGACGGTCGCGGGAGAGCTGAGAGGGCCTCCCCTTCAGCTGGACACGGGCCAGT
TGCTGAAGATCGCGAAGCGGGGAGGAGTCACGGCGGTCGAGGCGGTGCACGCGTGG
CGCAATGCGCTCACGGGAGCACCCCTCAACCTGACCCCAGAGCAGGTCGTGGCAAT
TGCGAGCAACCACGGGGGAAAGCAGGCACTCGAAACCGTCCAGAGGTTGCTGCCTG
TGCTGTGCCAAGCGCACGGACTTACGCCAGAGCAGGTCGTGGCAATTGCGAGCAAC
CACGGGGGAAAGCAGGCACTCGAAACCGTCCAGAGGTTGCTGCCTGTGCTGTGCCA
AGCGCACGGACTAACCCCAGAGCAGGTCGTGGCAATTGCGAGCAACATCGGGGGAA
AGCAGGCACTCGAAACCGTCCAGAGGTTGCTGCCTGTGCTGTGCCAAGCGCACGGG
TTGACCCCAGAGCAGGTCGTGGCAATTGCGAGCAACCACGGGGGAAAGCAGGCACT
CGAAACCGTCCAGAGGTTGCTGCCTGTGCTGTGCCAAGCGCACGGCCTGACCCCAG
AGCAGGTCGTGGCAATTGCGAGCAACCACGGGGGAAAGCAGGCACTCGAAACCGTC
 
CAGAGGTTGCTGCCTGTGCTGTGCCAAGCGCACGGACTGACACCAGAGCAGGTCGT
GGCAATTGCGAGCAACATCGGGGGAAAGCAGGCACTCGAAACCGTCCAGAGGTTGC
TGCCTGTGCTGTGCCAAGCGCACGGACTTACACCCGAACAAGTCGTGGCAATTGCG
AGCAACCACGGGGGAAAGCAGGCACTCGAAACCGTCCAGAGGTTGCTGCCTGTGCT
GTGCCAAGCGCACGGACTTACGCCAGAGCAGGTCGTGGCAATTGCGAGCAACCACG
GGGGAAAGCAGGCACTCGAAACCGTCCAGAGGTTGCTGCCTGTGCTGTGCCAAGCG
CACGGACTAACCCCAGAGCAGGTCGTGGCAATTGCGAGCAACATCGGGGGAAAGCA
GGCACTCGAAACCGTCCAGAGGTTGCTGCCTGTGCTGTGCCAAGCGCACGGGTTGA
CCCCAGAGCAGGTCGTGGCAATTGCGAGCAACATCGGGGGAAAGCAGGCACTCGAA
ACCGTCCAGAGGTTGCTGCCTGTGCTGTGCCAAGCGCACGGCCTGACCCCAGAGCA
GGTCGTGGCAATTGCGAGCAACCACGGGGGAAAGCAGGCACTCGAAACCGTCCAGA
GGTTGCTGCCTGTGCTGTGCCAAGCGCACGGACTGACACCAGAGCAGGTCGTGGCA
ATTGCGAGCAACCACGGGGGAAAGCAGGCACTCGAAACCGTCCAGAGGTTGCTGCC
TGTGCTGTGCCAAGCGCACGGACTCACGCCTGAGCAGGTAGTGGCTATTGCATCCAA
CAACGGGGGCAGACCCGCACTGGAGTCAATCGTGGCCCAGCTTTCGAGGCCGGACC
CCGCGCTGGCCGCACTCACTAATGATCATCTTGTAGCGCTGGCCTGCCTCGGCGGAC
GACCCGCCTTGGATGCGGTGAAGAAGGGGCTCCCGCACGCGCCTGCATTGATTAAG
CGGACCAACAGAAGGATTCCCGAGAGGACATCACATCGAGTGGCAGGTTCCCAACT
CGTGAAGAGTGAACTTGAGGAGAAAAAGTCGGAGCTGCGGCACAAATTGAAATACG
TACCGCATGAATACATCGAACTTATCGAAATTGCTAGGAACTCGACTCAAGACAGA
ATCCTTGAGATGAAGGTAATGGAGTTCTTTATGAAGGTTTATGGATACCGAGGGAAG
CATCTCGGTGGATCACGAAAACCCGACGGAGCAATCTATACGGTGGGGAGCCCGAT
TGATTACGGAGTGATCGTCGACACGAAAGCCTACAGCGGTGGGTACAATCTTCCCAT
CGGGCAGGCAGATGAGATGCAACGTTATGTCGAAGAAAATCAGACCAGGAACAAA
CACATCAATCCAAATGAGTGGTGGAAAGTGTATCCTTCATCAGTGACCGAGTTTAAG
TTTTTGTTTGTCTCTGGGCATTTCAAAGGCAACTATAAGGCCCAGCTCACACGGTTG
AATCACATTACGAACTGCAATGGTGCGGTTTTGTCCGTAGAGGAACTGCTCATTGGT
GGAGAAATGATCAAAGCGGGAACTCTGACACTGGAAGAAGTCAGACGCAAGTTTAA
CAATGGCGAGATCAATTTCCGCTCA
> EMX1_TALEN_Right

ATGGACTATAAGGACCACGACGGAGACTACAAGGATCATGATATTGATTA
CAAAGACGATGACGATAAGATGGCCCCAAAGAAGAAGCGGAAGGTCGGTATCCAC
GGAGTCCCAGCAGCCGTAGATTTGAGAACTTTGGGATATTCACAGCAGCAGCAGGA
AAAGATCAAGCCCAAAGTGAGGTCGACAGTCGCGCAGCATCACGAAGCGCTGGTGG
GTCATGGGTTTACACATGCCCACATCGTAGCCTTGTCGCAGCACCCTGCAGCCCTTG
GCACGGTCGCCGTCAAGTACCAGGACATGATTGCGGCGTTGCCGGAAGCCACACAT
GAGGCGATCGTCGGTGTGGGGAAACAGTGGAGCGGAGCCCGAGCGCTTGAGGCCCT
GTTGACGGTCGCGGGAGAGCTGAGAGGGCCTCCCCTTCAGCTGGACACGGGCCAGT
TGCTGAAGATCGCGAAGCGGGGAGGAGTCACGGCGGTCGAGGCGGTGCACGCGTGG
CGCAATGCGCTCACGGGAGCACCCCTCAACCTGACCCCAGAGCAGGTCGTGGCAAT
TGCGAGCAACCACGGGGGAAAGCAGGCACTCGAAACCGTCCAGAGGTTGCTGCCTG
TGCTGTGCCAAGCGCACGGACTTACGCCAGAGCAGGTCGTGGCAATTGCGAGCAAC
CACGGGGGAAAGCAGGCACTCGAAACCGTCCAGAGGTTGCTGCCTGTGCTGTGCCA
AGCGCACGGACTAACCCCAGAGCAGGTCGTGGCAATTGCGAGCAACCACGGGGGA
AAGCAGGCACTCGAAACCGTCCAGAGGTTGCTGCCTGTGCTGTGCCAAGCGCACGG
GTTGACCCCAGAGCAGGTCGTGGCAATTGCGAGCAACATCGGGGGAAAGCAGGCAC
TCGAAACCGTCCAGAGGTTGCTGCCTGTGCTGTGCCAAGCGCACGGCCTGACCCCAG
AGCAGGTCGTGGCAATTGCGAGCAACCACGGGGGAAAGCAGGCACTCGAAACCGTC
CAGAGGTTGCTGCCTGTGCTGTGCCAAGCGCACGGACTGACACCAGAGCAGGTCGT
GGCAATTGCGAGCCATGACGGGGGAAAGCAGGCACTCGAAACCGTCCAGAGGTTGC
TGCCTGTGCTGTGCCAAGCGCACGGACTTACACCCGAACAAGTCGTGGCAATTGCG
AGCCATGACGGGGGAAAGCAGGCACTCGAAACCGTCCAGAGGTTGCTGCCTGTGCT
GTGCCAAGCGCACGGACTTACGCCAGAGCAGGTCGTGGCAATTGCGAGCCATGACG
GGGGAAAGCAGGCACTCGAAACCGTCCAGAGGTTGCTGCCTGTGCTGTGCCAAGCG
CACGGACTAACCCCAGAGCAGGTCGTGGCAATTGCGAGCAACGGAGGGGGAAAGC
AGGCACTCGAAACCGTCCAGAGGTTGCTGCCTGTGCTGTGCCAAGCGCACGGGTTG
ACCCCAGAGCAGGTCGTGGCAATTGCGAGCAACGGAGGGGGAAAGCAGGCACTCG
AAACCGTCCAGAGGTTGCTGCCTGTGCTGTGCCAAGCGCACGGCCTGACCCCAGAG
CAGGTCGTGGCAATTGCGAGCCATGACGGGGGAAAGCAGGCACTCGAAACCGTCCA
GAGGTTGCTGCCTGTGCTGTGCCAAGCGCACGGACTGACACCAGAGCAGGTCGTGG
CAATTGCGAGCAACGGAGGGGGAAAGCAGGCACTCGAAACCGTCCAGAGGTTGCTG

CCTGTGCTGTGCCAAGCGCACGGACTCACGCCTGAGCAGGTAGTGGCTATTGCATCC
AACGGAGGGGGCAGACCCGCACTGGAGTCAATCGTGGCCCAGCTTTCGAGGCCGGA
CCCCGCGCTGGCCGCACTCACTAATGATCATCTTGTAGCGCTGGCCTGCCTCGGCGG
ACGACCCGCCTTGGATGCGGTGAAGAAGGGGCTCCCGCACGCGCCTGCATTGATTA
AGCGGACCAACAGAAGGATTCCCGAGAGGAATCACATCGAGTGGCAGGTTCCCAAC
TCGTGAAGAGTGAACTTGAGGAGAAAAAGTCGGAGCTGCGGCACAAATTGAAATAC
GTACCGCATGAATACATCGAACTTATCGAAATTGCTAGGAACTCGACTCAAGACAG
AATCCTTGAGATGAAGGTAATGGAGTTCTTTATGAAGGTTTATGGATACCGAGGGAA
GCATCTCGGTGGATCACGAAAACCCGACGGAGCAATCTATACGGTGGGGAGCCCGA
TTGATTACGGAGTGATCGTCGACACGAAAGCCTACAGCGGTGGGTACAATCTTCCCA
TCGGGCAGGCAGATGAGATGCAACGTTATGTCGAAGAAAATCAGACCAGGAACAAA
CACATCAATCCAAATGAGTGGTGGAAAGTGTATCCTTCATCAGTGACCGAGTTTAAG
TTTTTGTTTGTCTCTGGGCATTTCAAAGGCAACTATAAGGCCCAGCTCACACGGTTG
AATCACATTACGAACTGCAATGGTGCGGTTTTGTCCGTAGAGGAACTGCTCATTGGT
GGAGAAATGATCAAAGCGGGAACTCTGACACTGGAAGAAGTCAGACGCAAGTTTAA
CAATGGCGAGATCAATTTCCGCTCA

**Cas9 transcriptional effectors.** HEK 293FT cells were co-transfected with mutant Cas9 fusion protein and a synthetic guide RNA (sgRNA) using Lipofectamine 2000 (Life Technologies) 24 hours after seeding into a 24 well dish. 72 hours post-transfection, total RNA was purified (RNeasy Plus, Qiagen). 1µg of RNA was reverse transcribed into cDNA (qScript, Quanta BioSciences). Quantitative real-time PCR was done according to the manufacturer's protocol (Life Technologies) and performed in triplicate using TaqMan Assays for hKlf4 (Hs00358836_ml), hSox2 (Hs01053049_s1), and the endogenous control GAPDH (Hs02758991_g1).

The hSpCas9 activator plasmid was cloned into a lentiviral vector under the expression of the hEFla promoter (pLenti-EF1a-Cas9-NLS-VP64). The hSpCas9 repressor plasmid was cloned into the same vector (pLenti-EF1a-SID4x-NLS-Cas9-NLS). Guide sequences (20bp) targeted to the *KLF4* locus are: GCGCGCTCCACACAACTCAC, GCAAAAATAGACAATCAGCA, GAAGGATCTCGGCCAATTTG. Spacer sequences for guide RNAs targeted to the *SOX2* locus are: GCTGCCGGGTTTTGCATGAA, CCGGGCCCGCAGCAAACTTC, GGGGCTGTCAGGGAATAAAT.
>hSpCas9(D10A,H840A)-Linker-NLS-VP64 
MDKKYSIGLAIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKK
HERHPIFGNIVDEVAYHEKYPTIYHLRKKLVDSTDKADLRLIYLALAHMIKFRGHFLIEG
DLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFL
AAKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSEETI
TPWNFEEVVDKGASAQSFIERMTNFDKNLPNEKVLPKHSLLYEYFTVYNELTKVKYVTE
GMRKPAFLSGEQKKAIVDLLFKTNRKVTVKQLKEDYFKKIECFDSVEISGVEDRFNASL
GTYHDLLKIIKDKDFLDNEENEDILEDIVLTLTLFEDREMIEERLKTYAHLFDDKVMKQL
KRRRYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQL
LNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKL
ESEFVYGDYKVYDVRKMIAKSEQEIGKATAKYFFYSNIMNFFKTEITLANGEIRKRPLIET
NGETGEIVWDKGRDFATVRKVLSMPQVNIVKKTEVQTGGFSKESILPKRNSDKLIARKK
DWDPKKYGGFDSPTVAYSVLVVAKVEKGKSKKLKSVKELLGITIMERSSFEKNPIDFLE
AKGYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRDKPI
REQAENIIHLFTLTNLGAPAAFKYFDTTIDRKRYTSTKEVLDATLIHQSITGLYETRIDLSQ
LGGDSAGGGGSGGGGSGGGGSGPKKKRKVAAAGSGRADALDDFDLDMLGSDALDDF
DLDMLGSDALDDFDLDMLGSDALDDFDLDMLIN
>SID4X-NLS-FLAG-Linker-hSpCas9(D10A,H840A)-NLS

MGSGMNIQMLLEAADYLERREREAEHGYASMLPGSGMNIQMLLEAADYLE
RREREAEHGYASMLPGSGMNIQMLLEAADYLERREREAEHGYASMLPGSGMNIQMLL
EAADYLERREREAEHGYASMLPSRSAGPKKKRKVAAADYKDDDDKSRLEPGEKPYKC
PECGKSFSQSGALTRHQRTHTRDKKYSIGLAIGTNSVGWAVITDEYKVPSKKFKVLGNT

DRHSIKKNLIGALLFDSGETAEATRLKRTARRRYTRRKNRICYLQEIFSNEMAKVDDSFF
HRLEESFLVEEDKKHERHPIFGNIVDEVAYHEKYPTIYHLRKKLVDSTDKADLRLIYLAL
AHMIKFRGHFLIEGDLNPDNSDVDKLFIQLVQTYNQLFEENPINASGVDAKAILSARLSK
SRRLENLIAQLPGEKKNGLFGNLIALSLGLTPNFKSNFDLAEDAKLQLSKDTYDDDLDNL
LAQIGDQYADLFLAAKNLSDAILLSDILRVNTEITKAPLSASMIKRYDEHHQDLTLLKAL
VRQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGNS
RFAWMTRKSEETITPWNFEEVVDKGASAQSFIERMTNFDKNLPNEKVLPKHSLLYEYFT
VYNELTKVKYVTEGMRKPAFLSGEQKKAIVDLLFKTNRKVTVKQLKEDYFKKIECFDS
VEISGVEDRFNASLGTYHDLLKIIKDKDFLDNEENEDILEDIVLTLTLFEDREMIEERLKT
YAHLFDDKVMKQLKRRRYTGWGRLSRKLINGIRDKQSGKTILDFLKSDGFANRNFMQL
IHDDSLTFKEDIQKAQVSGQGDSLHEHIANLAGSPAIKKGILQTVKVVDELVKVMGRHK
PENIVIEMARENQTTQKGQKNSRERMKRIEEGIKELGSQILKEHPVENTQLQNEKLYLYY
LQNGRDMYVDQELDINRLSDYDVDAIVPQSFLKDDSIDNKVLTRSDKNRGKSDNVPSEE
VVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESIL
PKRNSDKLIARKKDWDPKKYGGFDSPTVAYSVLVVAKVEKGKSKKLKSVKELLGITIM
ERSSFEKNPIDFLEAKGYKEVKKDLIIKLPKYSLFELENGRKRMLASAGELQKGNELALP
SKYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIHQ
SITGLYETRIDLSQLGGDKRPAATKKAGQAKKKK

### Example 3

### Inducible lentiviral Cas9

Lentivirus preparation. After cloning pCasES10 (which contains a lentiviral transfer plasmid backbone), HEK293FT at low passage (p=5) were seeded in a T-75 flask to 50% confluence the day before transfection in DMEM with 10% fetal bovine serum and without antibiotics. After 20 hours, media was changed to OptiMEM (serum-free) media and transfection was done 4 hours later. Cells were transfected with 10ug of lentiviral transfer plasmid (pCasES10) and the following packaging plasmids: 5ug of pMD2.G (VSV-g pseudotype), and 7.5ug of psPAX2 (gag/pol/rev/tat). Transfection was done in 4mL OptiMEM with a cationic lipid delivery agent (50uL Lipofectamine 2000 and 100ul Plus reagent). After 6 hours, the media was changed to antibiotic-free DMEM with 10% fetal bovine serum.

Lentivirus purification. Viral supernatants were harvested after 48 hours. Supernatants were first cleared of debris and filtered through a 0.45um low protein binding (PVDF) filter. They were then spun in a ultracentrifuge for 2 hours at 24,000 rpm. Viral pellets were resuspended in 50ul of DMEM overnight at 4C. They were then aliquotted and immediately frozen at -80C.

Clonal isolation using FACS. For clonal isolation of HEK293FT and HUES64 human embryonic stem cells, cells were infected in suspension with either 1ul or 5ul of purified virus. Twenty-four hours post infection, 1uM doxycycline was added to the cell culture media. After 24 or 48 hours more, cells underwent fluorescence-assisted cell sorting (FACS) on a BD FACSAria IIu instrument to isolate single cells that robustly expressed EGFP (and hence Cas9) after doxycycline treatment. Cell were plated either in bulk or into individual wells to allow selection of clonal populations with an integrated inducible Cas9 for further use. Sort efficiency was always >95% and cells were visualized immediately after plating to verify EGFP fluorescence.

FIG. 15 depicts Tet Cas9 vector designs

FIG. 16 depicts a vector and EGFP expression in 293FT cells.

Sequence ofpCasES020 inducible Cas9:

caactttgtatagaaaagttggctccgaattcgcccttcaggtccgaggttctagacgagtttactccctatcagtgatagaga
acgatgtcgagtttactccctatcagtgatagagaacgtatgtcgagtttactccctatcagtgatagagaacgtatgtcgagtttactccctatc
agtgatagagaacgtatgtcgagtttatccctatcagtgatagagaacgtatgtcgagtttactccctatcagtgatagagaacgtatgtcgag
gtaggcgtgtacggtgggaggcctatataagcagagctcgtttagtgaaccgtcagatcgcaaagggcgaattcgacccaagtttgtacag
ccaccATGGACTATAAGGACCACGACGGAGACTACAAGGATCATGATATTGATTACAA
AGACGATGACGATAAGATGGCCCCAAAGAAGAAGCGGAAGGTCGGTATCCACGGA
GTCCCAGCAGCCGACAAGAAGTACAGCATCGGCCTGGACATCGGCACCAACTCTGT
GGGCTGGGCCGTGATCACCGACGAGTACAAGGTGCCCAGCAAGAAATTCAAGGTGC
TGGGCAACACCGACCGGCACAGCATCAAGAAGAACCTGATCGGAGCCCTGCTGTTC
GACAGCGGCGAAACAGCCGAGGCCACCCGGCTGAAGAGAACCGCCAGAAGAAGAT
ACACCAGACGGAAGAACCGGATCTGCTATCTGCAAGAGATCTTCAGCAACGAGATG
GCCAAGGTGGACGACAGCTTCTTCCACAGACTGGAAGAGTCCTTCCTGGTGGAAGA

GGATAAGAAGCACGAGCGGCACCCCATCTTCGGCAACATCGTGGACGAGGTGGCCT
ACCACGAGAAGTACCCCACCATCTACCACCTGAGAAAGAAACTGGTGGACAGCACC
GACAAGGCCGACCTGCGGCTGATCTATCTGGCCCTGGCCCACATGATCAAGTTCCGG
GGCCACTTCCTGATCGAGGGCGACCTGAACCCCGACAACAGCGACGTGGACAAGCT
GTTCATCCAGCTGGTGCAGACCTACAACCAGCTGTTCGAGGAAAACCCCATCAACG
CCAGCGGCGTGGACGCCAAGGCCATCCTGTCTGCCAGACTGAGCAAGAGCAGACGG
CTGGAAAATCTGATCGCCCAGCTGCCCGGCGAGAAGAAGAATGGCCTGTTCGGCAA
CCTGATTGCCCTGAGCCTGGGCCTGACCCCCAACTTCAAGAGCAACTTCGACCTGGC
CGAGGATGCCAAACTGCAGCTGAGCAAGGACACCTACGACGACGACCTGGACAACC
TGCTGGCCCAGATCGGCGACCAGTACGCCGACCTGTTTCTGGCCGCCAAGAACCTGT
CCGACGCCATCCTGCTGAGCGACATCCTGAGAGTGAACACCGAGATCACCAAGGCC
CCCCTGAGCGCCTCTATGATCAAGAGATACGACGAGCACCACCAGGACCTGACCCT
GCTGAAAGCTCTCGTGCGGCAGCAGCTGCCTGAGAAGTACAAAGAGATTTTCTTCGA
CCAGAGCAAGAACGGCTACGCCGGCTACATTGACGGCGGAGCCAGCCAGGAAGAG
TTCTACAAGTTCATCAAGCCCATCCTGGAAAAGATGGACGGCACCGAGGAACTGCT
CGTGAAGCTGAACAGAGAGGACCTGCTGCGGAAGCAGCGGACCTTCGACAACGGCA
GCATCCCCCACCAGATCCACCTGGGAGAGCTGCACGCCATTCTGCGGCGGCAGGAA
GATTTTTACCCATTCCTGAAGGACAACCGGGAAAAGATCGAGAAGATCCTGACCTTC
CGCATCCCCTACTACGTGGGCCCTCTGGCCAGGGGAAACAGCAGATTCGCCTGGAT
GACCAGAAAGAGCGAGGAAACCATCACCCCCTGGAACTTCGAGGAAGTGGTGGAC
AAGGGCGCTTCCGCCCAGAGCTTCATCGAGCGGATGACCAACTTCGATAAGAACCT
GCCCAACGAGAAGGTGCTGCCCAAGCACAGCCTGCTGTACGAGTACTTCACCGTGT
ATAACGAGCTGACCAAAGTGAAATACGTGACCGAGGGAATGAGAAAGCCCGCCTTC
CTGAGCGGCGAGCAGAAAAAGGCCATCGTGGACCTGCTGTTCAAGACCAACCGGAA
AGTGACCGTGAAGCAGCTGAAAGAGGACTACTTCAAGAAAATCGAGTGCTTCGACT
CCGTGGAAATCTCCGGCGTGGAAGATCGGTTCAACGCCTCCCTGGGCACATACCAC
GATCTGCTGAAAATTATCAAGGACAAGGACTTCCTGGACAATGAGGAAAACGAGGA
CATTCTGGAAGATATCGTGCTGACCCTGACACTGTTTGAGGACAGAGAGATGATCGA
GGAACGGCTGAAAACCTATGCCCACCTGTTCGACGACAAAGTGATGAAGCAGCTGA
AGCGGCGGAGATACACCGGCTGGGGCAGGCTGAGCCGGAAGCTGATCAACGGCATC
CGGGACAAGCAGTCCGGCAAGACAATCCTGGATTTCCTGAAGTCCGACGGCTTCGC

CAACAGAAACTTCATGCAGCTGATCCACGACGACAGCCTGACCTTTAAAGAGGACA
TCCAGAAAGCCCAGGTGTCCGGCCAGGGCGATAGCCTGCACGAGCACATTGCCAAT
CTGGCCGGCAGCCCCGCCATTAAGAAGGGCATCCTGCAGACAGTGAAGGTGGTGGA
CGAGCTCGTGAAAGTGATGGGCCGGCACAAGCCCGAGAACATCGTGATCGAAATGG
CCAGAGAGAACCAGACCACCCAGAAGGGACAGAAGAACAGCCGCGAGAGAATGAA
GCGGATCGAAGAGGGCATCAAAGAGCTGGGCAGCCAGATCCTGAAAGAACACCCC
GTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGATG
TGGACCATATCGTGCCTCAGAGCTTTCTGAAGGACGACTCCATCGACAACAAGGTGC
TGACCAGAAGCGACAAGAACCGGGGCAAGAGCGACAACGTGCCCTCCGAAGAGGT
CGTGAAGAAGATGAAGAACTACTGGCGGCAGCTGCTGAACGCCAAGCTGATTACCC
AGAGAAAGTTCGACAATCTGACCAAGGCCGAGAGAGGCGGCCTGAGCGAACTGGAT
AAGGCCGGCTTCATCAAGAGACAGCTGGTGGAAACCCGGCAGATCACAAAGCACGT
GGCACAGATCCTGGACTCCCGGATGAACACTAAGTACGACGAGAATGACAAGCTGA
TCCGGGAAGTGAAAGTGATCACCCTGAAGTCCAAGCTGGTGTCCGATTTCCGGAAG
GATTTCCAGTTTTACAAAGTGCGCGAGATCAACAACTACCACCACGCCCACGACGCC
TACCTGAACGCCGTCGTGGGAACCGCCCTGATCAAAAAGTACCCTAAGCTGGAAAG
CGAGTTCGTGTACGGCGACTACAAGGTGTACGACGTGCGGAAGATGATCGCCAAGA
GCGAGCAGGAAATCGGCAAGGCTACCGCCAAGTACTTCTTCTACAGCAACATCATG
AACTTTTTCAAGACCGAGATTACCCTGGCCAACGGCGAGATCCGGAAGCGGCCTCT
GATCGAGACAAACGGCGAAACCGGGGAGATCGTGTGGGATAAGGGCCGGGATTTTG
CCACCGTGCGGAAAGTGCTGAGCATGCCCCAAGTGAATATCGTGAAAAAGACCGAG
GTGCAGACAGGCGGCTTCAGCAAAGAGTCTATCCTGCCCAAGAGGAACAGCGATAA
GCTGATCGCCAGAAAGAAGGACTGGGACCCTAAGAAGTACGGCGGCTTCGACAGCC
CCACCGTGGCCTATTCTGTGCTGGTGGTGGCCAAAGTGGAAAAGGGCAAGTCCAAG
AAACTGAAGAGTGTGAAAGAGCTGCTGGGGATCACCATCATGGAAAGAAGCAGCTT
CGAGAAGAATCCCATCGACTTTCTGGAAGCCAAGGGCTACAAAGAAGTGAAAAAGG
ACCTGATCATCAAGCTGCCTAAGTACTCCCTGTTCGAGCTGGAAAACGGCCGGAAG
AGAATGCTGGCCTCTGCCGGCGAACTGCAGAAGGGAAACGAACTGGCCCTGCCCTC
CAAATATGTGAACTTCCTGTACCTGGCCAGCCACTATGAGAAGCTGAAGGGCTCCCC
CGAGGATAATGAGCAGAAACAGCTGTTTGTGGAACAGCACAAGCACTACCTGGACG

AGATCATCGAGCAGATCAGCGAGTTCTCCAAGAGAGTGATCCTGGCCGACGCTAAT
CTGGACAAAGTGCTGTCCGCCTACAACAAGCACCGGGATAAGCCCATCAGAGAGCA
GGCCGAGAATATCATCCACCTGTTTACCCTGACCAATCTGGGAGCCCCTGCCGCCTT
CAAGTACTTTGACACCACCATCGACCGGAAGAGGTACACCAGCACCAAAGAGGTGC
TGGACGCCACCCTGATCCACCAGAGCATCACCGGCCTGTACGAGACACGGATCGAC
CTGTCTCAGCTGGGAGGCGACAAAAGGCCGGCGGCCACGAAAAAGGCCGGCCAGG
CAAAAAAGAAAAAGgaattctctagaGGCAGTGGAGAGGGCAGAGGAAGTCTGCTAACAT
GCGGTGACGTCGAGGAGAATCCTGGCCCAGTGAGCAAGGGCGAGGAGCTGTTCACC
GGGGTGGTGCCCATCCTGGTCGAGCTGGACGGCGACGTAAACGGCCACAAGTTCAG
CGTGTCCGGCGAGGGCGAGGGCGATGCCACCTACGGCAAGCTGACCCTGAAGTTCA
TCTGCACCACCGGCAAGCTGCCCGTGCCCTGGCCCACCCTCGTGACCACCCTGACCT
ACGGCGTGCAGTGCTTCAGCCGCTACCCCGACCACATGAAGCAGCACGACTTCTTCA
AGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGC
TGGAGTACAACTACAACAGCCACAACGTCTATATCATGGCCGACAAGCAGAAGAAC
GGCATCAAGGTGAACTTCAAGATCCGCCACAACATCGAGGACGGCAGCGTGCAGCT
CGCCGACCACTACCAGCAGAACACCCCCATCGGCGACGGCCCCGTGCTGCTGCCCG
ACAACCACTACCTGAGCACCCAGTCCGCCCTGAGCAAAGACCCCAACGAGAAGCGC
GATCACATGGTCCTGCTGGAGTTCGTGACCGCCGCCGGGATCACTCTCGGCATGGAC
GAGCTGTACAAGCTCGAGGGAAGCGGAGCTACTAACTTCAGCCTGCTGAAGCAGGC
TGGCGACGTGGAGGAGAACCCTGGACCTatgtctaggctggacaagagcaaagtcataaacggagctctggaa
ttactcaatggtgtcggtatcgaaggcctgacgacaaggaaactcgctcaaaagctgggagttgagcagcctaccctgtactggcacgtga
agaacaagcgggccctgctcgatgccctgccaatcgagatgctggacaggcatcatacccacttctgccccctggaaggcgagtcatggc
aagactttctgcggaacaacgccaagtcataccgctgtgctctcctctcacatcgcgacggggctaaagtgcatctcggcacccgcccaac
agagaaacagtacgaaaccctggaaaatcagctcgcgttcctgtgtcagcaaggcttctccctggagaacgcactgtacgctctgtccgcc
gtgggccactttacactgggctgcgtattggaggaacaggagcatcaagtagcaaaagaggaaagagagacacctaccaccgattctatg
cccccacttctgagacaagcaattgagctgttcgaccggcagggagccgaacctgccttccttttcggcctggaactaatcatatgtggcctg
gagaaacagctaaagtgcgaaagcggcgggccgaccgacgcccttgacgattttgacttagacatgctcccagccgatgcccttgacgatt
ttgaccttgacatgctccccgggtaatgtacaaagtggtgaattccggcaattcgatatcaagcttatcgataatcaacctctggattacaaaatt
tgtgaaagattgactggtattcttaactatgttgctccttttacgctatgtggatacgctgctttaatgcctttgtatcatgctattgcttcccgtatgg

ctttcattttctcctccttgtataaatcctggttgctgtctctttatgaggagttgtggcccgttgtcaggcaacgtggcgtggtgtgcactgtgtttg
ctgacgcaacccccactggttggggcattgccaccacctgtcagctcctttccgggactttcgctttccccctccctattgccacggcggaac
tcatcgccgcctgccttgcccgctgctggacaggggctcggctgttgggcactgacaattccgtggtgttgtcggggaaatcatcgtcctttc
cttggctgctcgcctgtgttgccacctggattctgcgcgggacgtccttctgctacgtcccttcggccctcaatccagcggaccttccttcccg
cggcctgctgccggctctgcggcctcttccgcgtcttcgccttcgccctcagacgagtcggatctccctttgggccgcctccccgcatcgata
ccgtcgacctcgagacctagaaaaacatggagcaatcacaagtagcaatacagcagctaccaatgctgattgtgcctggctagaagcaca
agaggaggaggaggtgggttttccagtcacacctcaggtacctttaagaccaatgacttacaaggcagctgtagatcttagccactttttaaa
agaaaaggggggactggaagggctaattcactcccaacgaagacaagatatccttgatctgtggatctaccacacacaaggctacttccct
gattggcagaactacacaccagggccagggatcagatatccactgacctttggatggtgctacaagctagtaccagttgagcaagagaag
gtagaagaagccaatgaaggagagaacacccgcttgttacaccctgtgagcctgcatgggatggatgacccggagagagaagtattaga
gtggaggtttgacagccgcctagcatttcatcacatggcccgagagctgcatccggactgtactgggtctctctggttagaccagatctgagc
ctgggagctctctggctaactagggaacccactgcttaagcctcaataaagcttgccttgagtgcttcaagtagtgtgtgcccgtctgttgtgtg
actctggtaactagagatccctcagacccttttagtcagtgtggaaaatctctagcagggcccgtttaaacccgctgatcagcctcgactgtgc
cttctagttgccagccatctgttgtttgcccctcccccgtgccttccttgaccctggaaggtgccactcccactgtcctttcctaataaaatgagg
aaattgcatcgcattgtctgagtaggtgtcattctattctggggggtggggtggggcaggacagcaagggggaggattgggaagacaatag
caggcatgctggggatgcggtgggctctatggcttctgaggcggaaagaaccagctggggctctagggggtatccccacgcgccctgta
gcggcgcattaagcgcggcgggtgtggtggttacgcgcagcgtgaccgctacacttgccagcgccctagcgcccgctcctttcgctttcttc
ccttcctttctcgccacgttcgccggctttccccgtcaagctctaaatcgggggctccctttagggttccgatttagtgctttacggcacctcgac
cccaaaaaacttgattagggtgatggttcacgtagtgggccatcgccctgatagacggtttttcgccctttgacgttggagtccacgttctttaat
agtggactcttgttccaaactggaacaacactcaaccctatctcggtctattcttttgatttataagggattttgccgatttcggcctattggttaaa
aaatgagctgatttaacaaaaatttaacgcgaattaattctgtggaatgtgtgtcagttagggtgtggaaagtccccaggctccccagcaggc
agaagtatgcaaagcatgcatctcaattagtcagcaaccaggtgtggaaagtccccaggctccccagcaggcagaagtatgcaaagcatg
catctcaattagtcagcaaccatagtcccgcccctaactccgcccatcccgcccctaactccgcccagttccgcccattctccgccccatggc
tgactaattttttttatttatgcagaggccgaggccgcctctgcctctgagctattccagaagtagtgaggaggcttttttggaggcctaggctttt
gcaaaaagctcccgggagcttgtatatccattttcggatctgatcagcacgtgttgacaattaatcatcggcatagtatatcggcatagtataat
acgacaaggtgaggaactaaaccatggccaagttgaccagtgccgttccggtgctcaccgcgcgcgacgtcgccggagcggtcgagttc
tggaccgaccggctcgggttctcccgggacttcgtggaggacgacttcgccggtgtggtccgggacgacgtgaccctgttcatcagcgcg
gtccaggaccaggtggtgccggacaacaccctggcctgggtgtgggtgcgcggcctggacgagctgtacgccgagtggtcggaggtcg
tgtccacgaacttccgggacgcctccgggccggccatgaccgagatcggcgagcagccgtgggggcgggagttcgccctgcgcgacc
cggccggcaactgcgtgcacttcgtggccgaggagcaggactgacacgtgctacgagatttcgattccaccgccgccttctatgaaaggtt
gggcttcggaatcgttttccgggacgccggctggatgatcctccagcgcggggatctcatgctggagttcttcgcccaccccaacttgtttatt

gcagcttataatggttacaaataaagcaatagcatcacaaatttcacaaataaagcatttttttcactgcattctagttgtggtttgtccaaactcat
caatgtatcttatcatgtctgtataccgtcgacctctagctagagcttggcgtaatcatggtcatagctgtttcctgtgtgaaattgttatccgctca
caattccacacaacatacgagccggaagcataaagtgtaaagcctggggtgcctaatgagtgagctaactcacattaattgcgttgcgctca
ctgcccgctttccagtcgggaaacctgtcgtgccagctgcattaatgaatcggccaacgcgcggggagaggcggtttgcgtattgggcgct
cttccgcttcctcgctcactgactcgctgcgctcggtcgttcggctgcggcgagcggtatcagctcactcaaaggcggtaatacggttatcca
cagaatcaggggataacgcaggaaagaacatgtgagcaaaaggccagcaaaaggccaggaaccgtaaaaaggccgcgttgctggcgtt
tttccataggctccgcccccctgacgagcatcacaaaaatcgacgctcaagtcagaggtggcgaaacccgacaggactataaagatacca
ggcgtttccccctggaagctccctcgtgcgctctcctgttccgaccctgccgcttaccggatacctgtccgcctttctcccttcgggaagcgtg
gcgctttctcatagctcacgctgtaggtatctcagttcggtgtaggtcgttcgctccaagctgggctgtgtgcacgaaccccccgttcagccc
gaccgctgcgccttatccggtaactatcgtcttgagtccaacccggtaagacacgacttatcgccactggcagcagccactggtaacaggat
tagcagagcgaggtatgtaggcggtgctacagagttcttgaagtggtggcctaactacggctacactagaagaacagtatttggtatctgcg
ctctgctgaagccagttaccttcggaaaaagagttggtagctcttgatccggcaaacaaaccaccgctggtagcggtggtttttttgtttgcaa
gcagcagattacgcgcagaaaaaaaggatctcaagaagatcctttgatcttttctacggggtctgacgctcagtggaacgaaaactcacgtt
aagggattttggtcatgagattatcaaaaaggatcttcacctagatccttttaaattaaaaatgaagttttaaatcaatctaaagtatatatgagtaa
acttggtctgacagttaccaatgcttaatcagtgaggcacctatctcagcgatctgtctatttcgttcatccatagttgcctgactccccgtcgtgt
agataactacgatacgggagggcttaccatctggccccagtgctgcaatgataccgcgagacccacgctcaccggctccagatttatcagc
aataaaccagccagccggaagggccgagcgcagaagtggtcctgcaactttatccgcctccatccagtctattaattgttgccgggaagcta
gagtaagtagttcgccagttaatagtttgcgcaacgttgttgccattgctacaggcatcgtggtgtcacgctcgtcgtttggtatggcttcattca
gctccggttcccaacgatcaaggcgagttacatgatcccccatgttgtgcaaaaaagcggttagctccttcggtcctccgatcgttgtcagaa
gtaagttggccgcagtgttatcactcatggttatggcagcactgcataattctcttactgtcatgccatccgtaagatgcttttctgtgactggtga
gtactcaaccaagtcattctgagaatagtgtatgcggcgaccgagttgctcttgcccggcgtcaatacgggataataccgcgccacatagca
gaactttaaaagtgctcatcattggaaaacgttcttcggggcgaaaactctcaaggatcttaccgctgttgagatccagttcgatgtaacccact
cgtgcacccaactgatcttcagcatcttttactttcaccagcgtttctgggtgagcaaaaacaggaaggcaaaatgccgcaaaaaagggaat
aagggcgacacggaaatgttgaatactcatactcttcctttttcaatattattgaagcatttatcagggttattgtctcatgagcggatacatatttg
aatgtatttagaaaaataaacaaataggggttccgcgcacatttccccgaaaagtgccacctgacgtcgacggatcgggagatctcccgatc
ccctatggtgcactctcagtacaatctgctctgatgccgcatagttaagccagtatctgctccctgcttgtgtgttggaggtcgctgagtagtgc
gcgagcaaaatttaagctacaacaaggcaaggcttgaccgacaattgcatgaagaatctgcttagggttaggcgttttgcgctgcttcgcgat
gtacgggccagatatacgcgttgacattgattattgactagttattaatagtaatcaattacggggtcattagttcatagcccatatatggagttcc
gcgttacataacttacggtaaatggcccgcctggctgaccgcccaacgacccccgcccattgacgtcaataatgacgtatgttcccatagta
acgccaatagggactttccattgacgtcaatgggtggagtatttacggtaaactgcccacttggcagtacatcaagtgtatcatatgccaagta
cgccccctattgacgtcaatgacggtaaatggcccgcctggcattatgcccagtacatgaccttatgggactttcctacttggcagtacatcta

cgtattagtcatcgctattaccatggtgatgcggttttggcagtacatcaatgggcgtggatagcggtttgactcacggggatttccaagtctcc
accccattgacgtcaatgggagtttgttttggcaccaaaatcaacgggactttccaaaatgtcgtaacaactccgccccattgacgcaaatgg
gcggtaggcgtgtacggtgggaggtctatataagcagcgcgttttgcctgtactgggtctctctggttagaccagatctgagcctgggagctc
tctggctaactagggaacccactgcttaagcctcaataaagcttgccttgagtgcttcaagtagtgtgtgcccgtctgttgtgtgactctggtaa
ctagagatccctcagacccttttagtcagtgtggaaaatctctagcagtggcgcccgaacagggacttgaaagcgaaagggaaaccagag
gagctctctcgacgcaggactcggcttgctgaagcgcgcacggcaagaggcgaggggcggcgactggtgagtacgccaaaaattttgac
tagcggaggctagaaggagagagatgggtgcgagagcgtcagtattaagcgggggagaattagatcgcgatgggaaaaaattcggttaa
ggccagggggaaagaaaaaatataaattaaaacatatagtatgggcaagcagggagctagaacgattcgcagttaatcctggcctgttaga
aacatcagaaggctgtagacaaatactgggacagctacaaccatcccttcagacaggatcagaagaacttagatcattatataatacagtag
caaccctctattgtgtgcatcaaaggatagagataaaagacaccaaggaagctttagacaagatagaggaagagcaaaacaaaagtaaga
ccaccgcacagcaagcggccgctgatcttcagacctggaggaggagatatgagggacaattggagaagtgaattatataaatataaagta
gtaaaaattgaaccattaggagtagcacccaccaaggcaaagagaagagtggtgcagagagaaaaaagagcagtgggaataggagcttt
gttccttgggttcttgggagcagcaggaagcactatgggcgcagcgtcaatgacgctgacggtacaggccagacaattattgtctggtatag
tgcagcagcagaacaatttgctgagggctattgaggcgcaacagcatctgttgcaactcacagtctggggcatcaagcagctccaggcaa
gaatcctggctgtggaaagatacctaaaggatcaacagctcctggggatttggggttgctctggaaaactcatttgcaccactgctgtgcctt
ggaatgctagttggagtaataaatctctggaacagatttggaatcacacgacctggatggagtgggacagagaaattaacaattacacaagc
ttaatacactccttaattgaagaatcgcaaaaccagcaagaaaagaatgaacaagaattattggaattagataaatgggcaagtttgtggaatt
ggtttaacataacaaattggctgtggtatataaaattattcataatgatagtaggaggcttggtaggtttaagaatagtttttgctgtactttctatag
tgaatagagttaggcagggatattcaccattatcgtttcagacccacctcccaaccccgaggggacccgacaggcccgaaggaatagaag
aagaaggtggagagagagacagagacagatccattcgattagtgaacggatcggcactgcgtgcgccaattctgcagacaaatggcagt
attcatccacaattttaaaagaaaaggggggattggggggtacagtgcaggggaaagaatagtagacataatagcaacagacatacaaact
aaagaattacaaaaacaaattacaaaaattcaaaattttcgggtttattacagggacagcagagatccagtttggttaattaa

## Claims

1. A Clustered Regularly Interspersed Short Palindromic Repeats (CRISPR)-Cas complex, comprising:
- a Cas9, wherein the Cas9 has two or more nuclear localization signals;
- a guide sequence linked to a tracr mate sequence, and
- a tracr sequence hybridizable with all or a portion of the tracr mate sequence;
wherein the guide sequence is designed to have complementarity with a target sequence in a eukaryotic cell and is capable of directing sequence-specific binding of the CRISPR complex to the target sequence in the eukaryotic cell.

2. A Clustered Regularly Interspersed Short Palindromic Repeats (CRISPR)-Cas vector system, comprising:
a) a first regulatory element operably linked to one or more sequences encoding (1) a guide sequence linked to a tracr mate sequence, and (2) a tracr sequence hybridizable with all or a portion of the tracr mate sequence;
b) a second regulatory element operably linked to a sequence encoding a Cas9, wherein the Cas9 has two or more nuclear localization signals;
wherein components (a) and (b) are located on same or different vectors of the system and wherein the guide sequence is designed to have complementarity with a target sequence in a eukaryotic cell.

3. The CRISPR-Cas vector system according to claim 2, wherein the sequence encoding the Cas9 is codon optimized for expression in eukaryotic cells.

4. The CRISPR-Cas vector system according to any one of claims 2 or 3, comprising two or more guide sequences.

5. The CRISPR-Cas vector system according to any one of claims 2-4, wherein said same or different vectors are one or more viral vectors.

6. The CRISPR-Cas vector system according to claim 5, wherein said one or more viral vectors are one or more retrovirus, adenovirus, adeno-associated alphavirus or herpes simplex virus vectors.

7. The CRISPR-Cas vector system according to claim 6, wherein the retrovirus is a lentivirus.

8. The CRISPR-Cas vector system according to claim 6, wherein the alphavirus is a Sindbis virus, Semliki Forest virus or Venezuelan equine encephalitis virus.

9. The CRISPR-Cas vector system according to according to any one of claims 2-8, wherein components (a) and (b) are located on the same vector.

10. The CRISPR-Cas complex or CRISPR-Cas vector system according to any one of claims 1-9, wherein the tracr mate sequence is linked with the tracr sequence to form a chimeric RNA (chiRNA).

11. The CRISPR-Cas complex or CRISPR-Cas vector system according to any one of claims 1-10, wherein the Cas9 directs cleavage of both strands of a target polynucleotide containing the target sequence.

12. The CRISPR-Cas complex or CRISPR-Cas vector system according to any one of claims 1-10, wherein the Cas9 is mutated with respect to a corresponding wild-type Cas9 such that the mutated Cas9 lacks the ability to cleave one or both strands of a target polynucleotide containing the target sequence.

13. The CRISPR-Cas complex or CRISPR-Cas vector system according to any one of claims 1-10 and 12, wherein the Cas9 comprises a mutation selected from the group consisting ofD10A, H840A, N854A and N863A with reference to the position numbering of a *S. pyogenes* Cas9.

14. The CRISPR-Cas complex or CRISPR-Cas vector system according to any one of claims 1-13, wherein the Cas9 comprises one or more nuclear localization signal(s) at the amino-terminus, and one or more nuclear localization signal(s) at the carboxy-terminus.

15. The CRISPR-Cas complex or CRISPR-Cas vector system according to any one of claims 1-14, wherein the Cas9 is a *S. pyogenes* Cas9.

16. The CRISPR-Cas complex or CRISPR-Cas vector system according to any one of claims 1-14, wherein the Cas9 is a *S*. *thermophilus* Cas9.

17. The CRISPR-Cas complex or CRISPR-Cas vector system according to any one of claims 1-16 for use in therapy.

18. Use of the CRISPR complex or CRISPR-Cas vector system according to any one of claims 1-16 for genome engineering, wherein said use is not a method of treatment of the animal or human body by therapy, and wherein said use is not a process for modifying the germ-line genetic identity of human beings.

## Patentansprüche

1. Clustered-Regularly-Interspersed-Short-Palindromic Repeats-(CRISPR)-Cas-Komplex, umfassend:
- ein Cas9, wobei das Cas9 zwei oder mehr Kernlokalisierungssignale aufweist;
- eine mit einer tracr-Paarsequenz (tracr mate sequence) verknüpfte Guide-Sequenz, und
- eine tracr-Sequenz, welche mit der vollständigen tracr-Paarsequenz oder eines Teils der tracr-Paarsequenz hybridisierbar ist;
wobei die Guide-Sequenz gestaltet ist, Komplementarität mit einer Zielsequenz in einer eukaryotischen Zelle aufzuweisen, und die Fähigkeit hat, sequenzspezifische Bindung des CRISPR-Komplexes an die Zielsequenz in der eukaryotischen Zelle zu steuern.

2. Clustered-Regularly-Interspersed-Short-Palindromic Repeats-(CRISPR)-Cas-Vektorsystem, umfassend:
a) ein erstes regulatorisches Element, welches funktionell mit einer oder mehreren Sequenz(en) verknüpft ist, welche (1) eine mit einer tracr-Paarsequenz verknüpfte Guide-Sequenz und (2) eine tracr-Sequenz, welche mit der vollständigen tracr-Paarsequenz oder eines Teils der tracr-Paarsequenz hybridisierbar ist, codiert/codieren;
b) ein zweites regulatorisches Element, welches funktionell mit einer Sequenz, welche ein Cas9 codiert, verknüpft ist, wobei das Cas9 zwei oder mehr Kernlokalisationssignale aufweist;
wobei Komponenten (a) und (b) auf demselben oder unterschiedlichen Vektor(en) des Systems lokalisiert sind und wobei die Guide-Sequenz gestaltet ist, Komplementarität mit einer Zielsequenz in einer eukaryotischen Zelle aufzuweisen.

3. CRISPR-Cas-Vektorsystem gemäß Anspruch 2, wobei die Sequenz, welche das Cas9 codiert, codon-optimiert für Expression in eukaryotischen Zellen ist.

4. CRISPR-Cas-Vektorsystem gemäß irgendeinem der Ansprüche 2 oder 3, umfassend zwei oder mehr Guide-Sequenzen.

5. CRISPR-Cas-Vektorsystem gemäß irgendeinem der Ansprüche 2 bis 4, wobei der derselbe oder unterschiedliche Vektor ein oder mehrere virale(r) Vektor/Vektoren ist/sind.

6. CRISPR-Cas-Vektorsystem gemäß Anspruch 5, wobei der derselbe oder unterschiedliche Vektor ein oder mehrere Retrovirus-, Adenovirus-, Adeno-assoziierter-Alphavirus- oder Herpex-simplex-Virus-Vektor(en) ist/sind.

7. CRISPR-Cas-Vektorsystem gemäß Anspruch 6, wobei das Retrovirus ein Lentivirus ist.

8. CRISPR-Cas-Vektorsystem gemäß Anspruch 6, wobei das Alphavirus ein Sindbis-Virus, Semliki-Forest-Virus oder venezolanisches Pferdeenzephalomyelitis-Virus ist.

9. CRISPR-Cas-Vektorsystem gemäß irgendeinem der Ansprüche 2-8, wobei Komponenten (a) und (b) auf demselben Vektor lokalisiert sind.

10. CRISPR-Cas-Komplex oder CRISPR-Cas-Vektorsystem gemäß irgendeinem der Ansprüche 1-9, wobei die tracr-Paarsequenz mit der tracr-Sequenz verknüpft ist, um eine chimäre RNA (chiRNA) zu bilden.

11. CRISPR-Cas-Komplex oder CRISPR-Cas-Vektorsystem gemäß irgendeinem der Ansprüche 1-10, wobei das Cas9 Schneiden beider Stränge eines Zielpolynukleotids, welches die Zielsequenz enthält, steuert.

12. CRISPR-Cas-Komplex oder CRISPR-Cas-Vektorsystem gemäß irgendeinem der Ansprüche 1-10, wobei das Cas9 gegenüber einem entsprechenden Wildtyp-Cas9 mutiert ist, so dass dem mutierten Cas9 die Fähigkeit fehlt, einen oder beide Stränge eines Zielpolynukleotids, welches die Zielsequenz enthält, zu schneiden.

13. CRISPR-Cas-Komplex oder CRISPR-Cas-Vektorsystem gemäß irgendeinem der Ansprüche 1-10 und 12, wobei das Cas9 eine Mutation, ausgewählt aus der Gruppe bestehend aus D10A, H840A, N854A und N863A, hinsichtlich der Positionsnummerierung eines *S. pyogenes*-Cas9, umfasst.

14. CRISPR-Cas-Komplex oder CRISPR-Cas-Vektorsystem gemäß irgendeinem der Ansprüche 1-13, wobei das Cas9 ein oder mehrere Kernlokalisationssignal(e) am Aminoterminus und ein oder mehrere Kernlokalisationssignal(e) am Carboxyterminus umfasst.

15. CRISPR-Cas-Komplex oder CRISPR-Cas-Vektorsystem gemäß irgendeinem der Ansprüche 1-14, wobei das Cas9 ein *S. pyogenes-*Cas9 ist.

16. CRISPR-Cas-Komplex oder CRISPR-Cas-Vektorsystem gemäß irgendeinem der Ansprüche 1-14, wobei das Cas9 ein *S. thermophilus-*Cas9 ist.

17. CRISPR-Cas-Komplex oder CRISPR-Cas-Vektorsystem gemäß irgendeinem der Ansprüche 1-16, zur therapeutischen Verwendung.

18. Verwendung des CRISPR-Komplexes oder des CRISPR-Cas-Vektorsystems gemäß irgendeinem der Ansprüche 1-16 zur Genom-Editierung (genome engineering), wobei die Verwendung kein Verfahren zur therapeutischen Behandlung des menschlichen oder tierischen Körpers ist, und wobei die Verwendung kein Verfahren zur Veränderung der genetischen Identität der Keimbahn menschlicher Lebewesen ist.

## Revendications

1. Complexe de Courtes Répétitions Palindromiques Groupées et Régulièrement Espacées (*Clustered Regularly Interspaced Short Palindromic Repeats*) (CRISPR)-Cas, comprenant :
- une Cas9, où la Cas9 possède deux, ou plus, signaux de localisation nucléaire ;
- une séquence guide liée à une séquence tracr mate, et
- une séquence tracr pouvant s'hybrider avec l'ensemble ou une portion de la séquence tracr mate ;
où la séquence guide est conçue pour avoir une complémentarité avec une séquence cible dans une cellule eucaryote et est capable de diriger une liaison séquence-spécifique du complexe CRISPR à la séquence cible dans la cellule eucaryote.

2. Système de vecteur de Courtes Répétitions Palindromiques Groupées et Régulièrement Espacées (*Clustered Regularly Interspaced Short Palindromic Repeats*) (CRISPR)-Cas, comprenant :
a) un premier élément régulateur lié de manière opérable à une ou plusieurs séquences codant pour (1) une séquence guide liée à une séquence tracr mate, et (2) une séquence tracr pouvant s'hybrider avec l'ensemble ou une portion de la séquence tracr mate ;
b) un deuxième élément régulateur lié de manière opérable à une séquence codant pour une Cas9, où la Cas9 possède deux, ou plus, signaux de localisation nucléaire ;
où les composants (a) et (b) sont situés sur des vecteurs identiques ou différents du système et où la séquence guide est conçue pour avoir une complémentarité avec une séquence cible dans une cellule eucaryote.

3. Système de vecteur CRISPR-Cas selon la revendication 2, dans lequel la séquence codant pour la Cas9 est à codons optimisés pour une expression dans des cellules eucaryotes.

4. Système de vecteur CRISPR-Cas selon l'une quelconque des revendications 2 ou 3, comprenant deux, ou plus, séquences guides.

5. Système de vecteur CRISPR-Cas selon l'une quelconque des revendications 2-4, dans lequel lesdits vecteurs identiques ou différents sont un ou plusieurs vecteurs viraux.

6. Système de vecteur CRISPR-Cas selon la revendication 5, dans lequel lesdits un ou plusieurs vecteurs viraux sont un ou plusieurs vecteurs de rétrovirus, d'adénovirus, d'alphavirus adéno-associé ou de virus herpès simplex.

7. Système de vecteur CRISPR-Cas selon la revendication 6, dans lequel le rétrovirus est un lentivirus.

8. Système de vecteur CRISPR-Cas selon la revendication 6, dans lequel l'alphavirus est un virus Sindbis, un virus de la Forêt de Semliki, ou un virus d'encéphalite équine du Venezuela.

9. Système de vecteur CRISPR-Cas selon l'une quelconque des revendications 2-8, dans lequel les composants (a) et (b) sont situés sur le même vecteur.

10. Complexe CRISPR-Cas ou système de vecteur CRISPR-Cas selon l'une quelconque des revendications 1-9, dans lequel la séquence tracr mate est liée à la séquence tracr afin de former un ARN chimérique (chiARN).

11. Complexe CRISPR-Cas ou système de vecteur CRISPR-Cas selon l'une quelconque des revendications 1-10, dans lequel la Cas9 dirige le clivage des deux brins d'un polynucléotide cible contenant la séquence cible.

12. Complexe CRISPR-Cas ou système de vecteur CRISPR-Cas selon l'une quelconque des revendications 1-10, dans lequel la Cas9 est mutée par rapport à une Cas9 de type sauvage correspondante, de sorte que la Cas9 mutée soit dépourvue de l'aptitude à cliver un brin ou les deux brins d'un polynucléotide cible contenant la séquence cible.

13. Complexe CRISPR-Cas ou système de vecteur CRISPR-Cas selon l'une quelconque des revendications 1-10 et 12, dans lequel la Cas9 comprend une mutation choisie dans le groupe constitué par D10A, H840A, N854A et N863A, en faisant référence à la numérotation de position d'une Cas9 de *S. pyogenes.*

14. Complexe CRISPR-Cas ou système de vecteur CRISPR-Cas selon l'une quelconque des revendications 1-13, dans lequel la Cas9 comprend un ou plusieurs signaux de localisation nucléaire en N-terminus, et un ou plusieurs signaux de localisation nucléaire en C-terminus.

15. Complexe CRISPR-Cas ou système de vecteur CRISPR-Cas selon l'une quelconque des revendications 1-14, dans lequel la Cas9 est une Cas9 de *S. pyogenes.*

16. Complexe CRISPR-Cas ou système de vecteur CRISPR-Cas selon l'une quelconque des revendications 1-14, dans lequel la Cas9 est une Cas9 de *S. thermophilus.*

17. Complexe CRISPR-Cas ou système de vecteur CRISPR-Cas selon l'une quelconque des revendications 1-16, pour une utilisation en thérapie.

18. Utilisation du complexe CRISPR-Cas ou du système de vecteur CRISPR-Cas selon l'une quelconque des revendications 1-16, pour l'ingénierie du génome (*genome engineering*), où ladite utilisation n'est pas une méthode de traitement thérapeutique du corps humain ou animal, et où ladite utilisation n'est pas un procédé de modification de l'identité génétique germinale de l'être humain.
